# EUROPÄISCHE PATENTSCHRIFT

(11) **EP 3 661 920 B1**
(45) Veröffentlichungstag und Bekanntmachung des Hinweises auf die Patenterteilung: **30.06.2021**
(21) Anmeldenummer: 18746952.3
(22) Anmeldetag: 02.08.2018
(51) Int. Cl.: C07D 257/06, C07D 307/46, C07D 333/22, C07D 405/12, C07D 409/12, C07C 65/32, C07C 65/40, C07C 69/76, C07C 69/92, C07C 323/62, A01N 43/713

(54) **3-ACYL-BENZAMIDE UND IHRE VERWENDUNG ALS HERBIZIDE**
3-ACYL-BENZAMIDES AND THEIR USE AS HERBICIDES
3-ACYL-BENZAMIDES ET LEUR UTILISATION COMME HERBICIDE

(30) Priorität: 04.08.2017 EP 17185026
(43) Veröffentlichungstag der Anmeldung: 10.06.2020
(73) Patentinhaber: Bayer Aktiengesellschaft, 51373 Leverkusen (DE)
(72) Erfinder: WALDRAFF, Christian, 61118 Bad Vilbel (DE); KÖHN, Arnim, 55270 Klein-Winternheim (DE); AHRENS, Hartmut, 63225 Langen (DE); BRAUN, Ralf, 76857 Ramberg (DE); DIETRICH, Hansjörg, 65835 Liederbach am Taunus (DE); MACHETTIRA, Anu, Bheemaiah, 60326 Frankfurt am Main (DE); ROSINGER, Christopher, Hugh, 65719 Hofheim (DE); GATZWEILER, Elmar, 61231 Bad Nauheim (DE); ASMUS, Elisabeth, 63768 Hösbach (DE)
(74) Vertreter: BIP Patents
(86) Internationale Anmeldenummer: PCT/EP2018/070991
(87) Internationale Veröffentlichungsnummer: WO 2019/025540

(56) Entgegenhaltungen:
- WO-A1-2012/028579
- WO-A1-2013/017559
- WO-A1-2017/005567
- WO-A1-2017/055146

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

WO 2012/028579 A1 offenbart herbizid wirksame Benzamide, die in 3-Position des Phenylrings eine Vielzahl von Substituenten tragen können. WO 2017/005567 A1, EP 3 118 199 A1 und WO 2017/055146 Albeschreiben ebenfalls herbizid wirksame Phenylamide, die in 3-Position des Phenylrings eine Vielzahl von Substituenten tragen können. Darüberhinaus offenbaren diese Schriften jeweils unter den Beispiel-Nr. 1-364 bis 1-367 und 1-426 bis 1-429 einzelne Phenylamide, die in 3-Position des Phenylrings einen Acetyl- oder Cyclopropylcarbonyl-Rest tragen. Jedoch weisen die aus den oben genannten Schriften bekannten Benzoylamide nicht immer eine ausreichende herbizide Wirkung und/oder Verträglichkeit gegenüber Kulturpflanzen auf.

Aufgabe der vorliegenden Erfindung ist es, alternative herbizid wirksame Wirkstoffe bereitzustellen. Diese Aufgabe wird durch die nachfolgend beschriebenen erfindungsgemäßen Benzamide gelöst, die in 3-Position des Phenylrings einen Acyl-Rest tragen.
Ein Gegenstand der vorliegenden Erfindung sind somit 3-Acyl-benzamide der Formel (I) 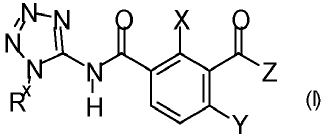 worin die Symbole und Indizes folgende Bedeutungen haben:
R^{x} bedeutet (C₁-C₆)-Alkyl,
X bedeutet Halogen, (C₁-C₆)-Alkyl, Halogen-(C1-C₆)-alkyl, (C₃-C₆)-Cycloalkyl, R¹O, R²S(O)ₙ oder R¹O-(C₁-C₆)-Alkyl,
Y bedeutet Halogen, (C₁-C₆)-Alkyl, Halogen-(C₁-C₆)-alkyl oder R¹O, R²S(O)ₙ,
Z bedeutet (C₁-C₆)-Alkyl, (C₃-C₆)-Cycloalkyl, (C₂-C₆)-Alkenyl, (C₃-C₆)-Alkinyl, Halogen-(C₁-C₆)-alkyl, (C₁-C₆)-Alkyl-O-(C₁-C₆)-alkyl, (C₁-C₆)-Alkyl-C(O), (C1-C₆)-Alkyl-C(O)-(C₁-C₆)-alkyl, Phenyl oder Heterocyclyl, wobei die Reste Phenyl, Heterocyclyl, (C₂-C₆)-Alkenyl, (C₃-C₆)-Alkinyl und (C₃-C₆)-Cycloalkyl jeweils m Substituenten R³ tragen,
R¹ bedeutet (C₁-C₆)-Alkyl oder Halogen-(C₁-C₆)-alkyl,
R² bedeutet (C₁-C₆)-Alkyl,
R³ bedeutet Halogen, (C₁-C₆)-Alkyl, (C₁-C₃)-Alkyl-O-C(O), Cyano oder Halogen-(C1-C₆)-alkyl,
m bedeutet 0, 1, 2, 3 oder 4,
n bedeutet 0, 1 oder 2.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, l-Methylprop-2-en-l-yl, 2-Methyl-prop-2-en-l-yl, But-2-en-l-yl, But-3-en-1-yl, 1-Methyl-but-3-en-1-yl und 1-Methyl-but-2-en-1-yl. Alkinyl bedeutet z.B. Propargyl, But-2-in-l-yl, But-3-in-l-yl, l-Methyl-but-3-in-l-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen wie Cyclopropyl, Cyclobutyl, Cyclopentyl oder Cyclohexyl.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen. Beispielsweise steht Heterocyclyl für Piperidinyl, Pyrrolidinyl, Tetrahydrofuranyl, Dihydrofuranyl, Oxetanyl, Thienyl und Furyl.

Die Verbindungen der Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der Formel (I) umfasst, jedoch nicht spezifisch definiert sind.

Bevorzugt sind Verbindungen der Formel (I), worin die Symbole und Indices folgende Bedeutungen haben:
R^{x} bedeutet (C₁-C₆)-Alkyl,
X bedeutet Halogen, (C₁-C₆)-Alkyl, Halogen-(C1-C₆)-alkyl, (C₃-C₆)-Cycloalkyl, R¹O, R²S(O)ₙ oder R¹O-(C₁-C₆)-Alkyl,
Y bedeutet Halogen, (C₁-C₆)-Alkyl, Halogen-(C₁-C₆)-alkyl oder R¹O, R²S(O)ₙ,
Z bedeutet (C₁-C₆)-Alkyl, (C₃-C₆)-Cycloalkyl, (C₂-C₆)-Alkenyl, (C₃-C₆)-Alkinyl, Halogen-(C₁-C₆)-alkyl, (C₁-C₆)-Alkyl-O-(C₁-C₆)-alkyl, (C₁-C₆)-Alkyl-C(O), (C₁-C₆)-Alkyl-C(O)-(C₁-C₆)-alkyl oder Phenyl, wobei die Reste Phenyl, (C₂-C₆)-Alkenyl, (C₃-C₆)-Alkinyl und (C₃-C₆)-Cycloalkyl jeweils m Substituenten R³ tragen,
R¹ bedeutet (C₁-C₆)-Alkyl oder Halogen-(C1-C₆)-alkyl,
R² bedeutet (C₁-C₆)-Alkyl,
R³ bedeutet Halogen, (C₁-C₆)-Alkyl, (C₁-C₃)-Alkyl-O-C(O), Cyano oder Halogen-(C1-C₆)-alkyl,
m bedeutet 0, 1, 2, 3 oder 4,
n bedeutet 0, 1 oder 2.

Besonders bevorzugt sind Verbindungen der Formel (I), worin die Symbole und Indices folgende Bedeutungen haben:
R^{x} bedeutet (C₁-C₆)-Alkyl,
X bedeutet Fluor, Chlor, Brom, Iod, Methyl, Ethyl, Cyclopropyl, Trifluormethyl, Difluormethyl, Methoxymethyl, Methoxy, Methylsulfanyl, Methylsulfinyl, Methylsulfonyl, Ethylsulfanyl oder Ethylsulfonyl,
Y bedeutet Chlor, Brom, Iod, Methyl, Ethyl, Trifluormethyl, Difluormethyl, Methylsulfanyl, Methylsulfinyl, Methylsulfonyl oder Ethylsulfonyl,
Z bedeutet Methyl, Ethyl, n-Propyl, iso-Propyl, Cyclopropyl, n-Butyl, tert-Butyl, Methoxymethyl, Chlormethyl, Acetyl, Vinyl, 1-Methylvinyl, 2-Methylvinyl, (1,2-Dimethyl)-vinyl, (2,2-Dimethyl)-vinyl, 1-Methylcyclopropyl, 2-Methylcyclopropyl, (2,2-Dimethyl)-cyclopropyl, (1,2-Dimethyl)-cyclopropyl, 2-Fluorcyclopropyl, (2,2-Difluor)-cyclopropyl, Cyclobutyl, Cyclopentyl, Cyclohexyl, 2-Thienyl, 2-Furyl, Phenyl, 4-Methoxyphenyl, 4-Chlorphenyl, (3-Trifluormethyl)-phenyl, 3,5-Difluorphenyl, Trifluormethyl oder Difluormethyl.

In allen nachfolgend genannten Formeln haben die Substituenten und Symbole, sofern nicht anders definiert, dieselbe Bedeutung wie unter Formel (I) beschrieben.

Verbindungen der Formel (II) eignen sich sehr gut als Intermediate zur Herstellung der erfindungsgemäßen Verbindungen der Formel (I). 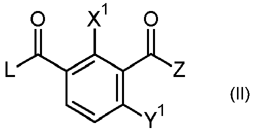 Darin haben die Symbole und Indizes folgende Bedeutungen:
L bedeutet Chlor, Methoxy oder Hydroxy,
X¹ bedeutet Methyl, Ethyl, Cyclopropyl, Methoxy, Methylsulfanyl, Ethylsulfanyl, Fluor, Chlor, Brom oder Iod,
Y¹ bedeutet Trifluormethyl oder Difluormethyl,
Z bedeutet Methyl, Ethyl, n-Propyl, iso-Propyl, Cyclopropyl, n-Butyl, tert-Butyl, Methoxymethyl, Chlormethyl, Acetyl, Vinyl, 1-Methylvinyl, 2-Methylvinyl, (1,2-Dimethyl)-vinyl, (2,2-Dimethyl)-vinyl, 1-Methylcyclopropyl, 2-Methylcyclopropyl, (2,2-Dimethyl)-cyclopropyl, (1,2-Dimethyl)-cyclopropyl, 2-Fluorcyclopropyl, (2,2-Difluor)-cyclopropyl, Cyclobutyl, Cyclopentyl, Cyclohexyl, 2-Thienyl, 2-Furyl, Phenyl, 4-Methoxyphenyl, 4-Chlorphenyl, (3-Trifluormethyl)-phenyl, (3,5-Difluor)-phenyl, Trifluormethyl oder Difluormethyl.

Erfindungsgemäße Verbindungen der Formel (I) können beispielsweise nach den in WO2012/028579 A1 angegebenen Methoden hergestellt werden. Die dazu benötigten Verbindungen der Formel (II) lassen sich durch Anwendung dem Fachmann bekannter Reaktionen synthetisieren, wobei die Verwendung der Syntheserouten u.a. vom Substitutionsmuster der Verbindungen der Formel (I) bzw. der Formel (II) abhängt. In den nachfolgenden Schemata 1 und 2 dargestellten Formeln haben die Substituenten L, X¹, Y¹ und Z jeweils die oben für Verbindungen der Formel (II) genannten Bedeutungen.

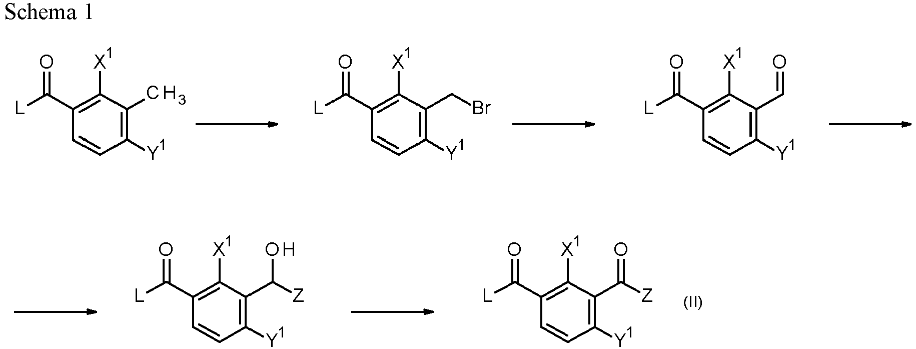

Verbindungen der Formel (II) können beispielsweise gemäß der in Schema 1 angegebenen Reaktionsfolge - ausgehend von substituierten Methylaromaten - durch Seitenkettenbromierung, Oxidation, nucleophiler Einführung der Gruppe Z und anschließender Oxidation hergestellt werden. Die substituierten Methylaromaten sind grundsätzlich bekannt bzw. können nach den in WO2012/028579 A1 angegebenen Methoden hergestellt werden.

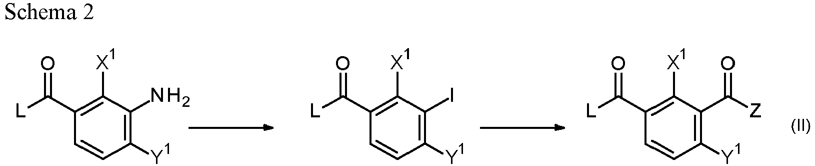

Verbindungen der Formel (II) können beispielsweise auch gemäß der in Schema 2 angegebenen Reaktionsfolge - ausgehend von substituierten Aminoaromaten - durch Diazotierung, Sandmeyer-Reaktion und anschließender Grignard-Reaktion hergestellt werden.
Verbindungen der Formel (II), worin Z für Cyclopropyl steht, können auch aus Verbindungen der Formel (II), worin Z für Vinyl steht, durch Cyclopropanierung mit z.B. Diazomethan oder Trimethylsulfoxoniumhalogeniden hergestellt werden

Kollektionen aus Verbindungen der Formel (I), die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der Formel (I) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl in fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) in Form von Substanzkollektionen, die Bibliotheken genannt werden. Gegenstand der vorliegenden Erfindung sind auch Bibliotheken, die mindestens zwei Verbindungen der Formel (I) enthalten.

Die erfindungsgemäßen Verbindungen weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria und Sorghum.
Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola und Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen, abhängig von ihrer jeweiligen chemischen Struktur und der ausgebrachten Aufwandmenge, hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Maniok, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren (,,molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen (_{,,}gene stacking").
Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykol-ethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether,
Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten
1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### Chemische Beispiele

Herstellung von Verbindungen der Formel (I)

### Beispiel 1: Herstellung von 3-Acetyl-2-chlor-N-(l-methyl-1H-tetrazol-5-yl)-4-(trifluormethyl)benzamid (Beispiel-Nr. 1-137)

150 mg (0.56 mmol) 3-Acetyl-2-chlor-4-(trifluormethyl)benzoesäure und 74 mg (0.73 mmol) 1-Methyl-1*H*-tetrazol-5-amin werden in 5 ml CH₂Cl₂ gelöst und bei RT mit 0.5 ml (0.84 mmol) einer 50 %igen Lösung von Propanphosphonsäureanhydrid in THF versetzt. Die Mischung wird 1 Stunde gerührt, danach werden 0.4 ml Triethylamin und katalytische Mengen DMAP zugegeben. Danach wird die Mischung weitere 3 Stunden bei RT gerührt und danach mit 5 ml 2N Salzsäure, 5 ml Wasser und 5 ml CH₂Cl₂ versetzt und 10 Minuten gerührt. Die organische Phase wird abgetrennt und eingedampft. Chromatographische Reinigung (Acetonitril/Wasser + 0.5% Trifluoressigsäure) liefert 110 mg 3-Acetyl-2-chlor-N-(l-methyl-1*H*-tetrazol-5-yl)-4-(tnfluormethyl)benzamid.

### Beispiel 2: Herstellung von 3-Acetyl-2-chlor-N-(1-ethyl-1H-tetrazol-5-yl)-4-(trifluormethyl)benzamid (Beispiel-Nr. 2-137).

200 mg (0.75 mmol) 3-Acetyl-2-chlor-4-(trifluormethyl)benzoesäure werden in 3 ml Pyridin vorgelegt und mit 107 mg (0.9 mmol) 1-Ethyl-1*H*-tetrazol-5-amin versetzt. Danach werden 0.1 ml (1.14 mmol) Oxalylchlorid zugegeben und 12 h bei RT gerührt. Die Mischung wird dann mit 5 ml Wasser versetzt, 10 Minuten nachgerührt und mit CH₂Cl₂ extrahiert. Die organische Phase wird abgetrennt, getrocknet und eingedampft. Chromatographische Reinigung (Acetonitril/Wasser + 0.5% Trifluoressigsäure) ergibt 66 mg 3-Acetyl-2-chlor-N-(1-ethyl-1*H*-tetrazol-5-yl)-4-(trifluormethyl)benzamid.

### Beispiel 3: Herstellung von 3-(Cyclopropylcarbonyl)-2-methyl-N-(1-methyl-1H-tetrazol-5-yl)-4-(trifluormethyl)benzamid (Beispiel-Nr. 1-18)

In analoger Weise zu Beispiel 2 werden aus 240 mg (0.88 mmol) 3-(Cyclopropylcarbonyl)-2-methyl-4-(trifluormethyl)benzoesäure und 107 mg (1.05 mmol) 1-Methyl-1H-tetrazol-5-amin 196 mg 3-(Cyclopropylcarbonyl)-2-methyl-N-(1-methyl-1*H*-tetrazol-5-yl)-4-(trifluormethyl)benzamid erhalten.

### Beispiel 4: Herstellung von 3-(Cyclopropylcarbonyl)-N-(1-ethyl-1H-tetrazol-5-yl)-2-methyl-4-(trifluormethyl)benzamid (Beispiel-Nr. 2-18)

Ebenso erhält man aus 155 mg (0.56 mmol) 3-(Cyclopropylcarbonyl)-2-methyl-4-(trifluormethyl)-benzoesäure und 81 mg (0.68 mmol) 1-Ethyl-1H-tetrazol-5-amin 95 mg 3-(Cyclopropylcarbonyl)-N-(1-ethyl-1*H*-tetrazol-5 -yl)- 2-methyl-4-(trifluormethyl )benzamid.

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:

| | | | |
|---|---|---|---|
| Ph = Phenyl | Me = Methyl | Et = Ethyl | c-Pr = cyclo-Propyl |
| Bu = Butyl | i-Pr = iso-Propyl | | |

**Tabelle 1: Erfindungsgemäße Verbindungen der Formel (I), worin R^{x} für eine Methylgruppe steht und die anderen Substituenten die unten genannten Bedeutungen haben.**

| | | | |
|---|---|---|---|
| 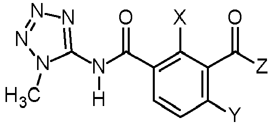 | | | |

| Nr. | X | Y | Z |
|---|---|---|---|
| 1-1 | Me | Me | Me |
| 1-2 | Me | Me | Et |
| 1-3 | Me | Me | c-Pr |
| 1-4 | Me | SMe | Me |
| 1-5 | Me | SMe | Et |
| 1-6 | Me | SMe | c-Pr |
| 1-7 | Me | SO₂Me | Me |
| 1-8 | Me | SO₂Me | Et |
| 1-9 | Me | SO₂Me | c-Pr |
| 1-10 | Me | SO₂Me | CH₂OMe |
| 1-11 | Me | SO₂Me | CH₂Cl |
| 1-12 | Me | SO₂Me | (1-Me)-c-Pr |
| 1-13 | Me | SO₂Me | (2-Me)-c-Pr |
| 1-14 | Me | CF₃ | Me |
| 1-15 | Me | CF₃ | Et |
| 1-16 | Me | CF₃ | n-Pr |
| 1-17 | Me | CF₃ | i-Pr |
| 1-18 | Me | CF₃ | c-Pr |
| 1-19 | Me | CF₃ | n-Bu |
| 1-20 | Me | CF₃ | t-Bu |
| 1-21 | Me | CF₃ | CH₂OMe |
| 1-22 | Me | CF₃ | CH₂Cl |
| 1-23 | Me | CF₃ | Ac |
| 1-24 | Me | CF₃ | (1-Me)-c-Pr |
| 1-25 | Me | CF₃ | (2-Me)-c-Pr |
| 1-26 | Me | CF₃ | (2,2-Me₂)-c-Pr |
| 1-27 | Me | CF₃ | (1,2-Me₂)-c-Pr |
| 1-28 | Me | CF₃ | (2-F)-c-Pr |
| 1-29 | Me | CF₃ | (2,2-F₂)-c-Pr |
| 1-30 | Me | CF₃ | c-Bu |
| 1-31 | Me | CF₃ | c-Pentyl |
| 1-32 | Me | CF₃ | c-Hexyl |
| 1-33 | Me | CF₃ | 2-Thienyl |
| 1-34 | Me | CF₃ | 2-Furyl |
| 1-35 | Me | CF₃ | Ph |
| 1-36 | Me | CF₃ | (4-MeO)-Ph |
| 1-37 | Me | CF₃ | (4-Cl)-Ph |
| 1-38 | Me | CF₃ | (3-CF₃)-Ph |
| 1-39 | Me | CF₃ | CF₃ |
| 1-40 | Me | CF₃ | CHF₂ |
| 1-41 | Me | CHF₂ | Me |
| 1-42 | Me | CHF₂ | Et |
| 1-43 | Me | CHF₂ | c-Pr |
| 1-44 | Me | CHF₂ | CH₂OMe |
| 1-45 | Me | CHF₂ | CH₂Cl |
| 1-46 | Me | CHF₂ | (1-Me)-c-Pr |
| 1-47 | Me | CHF₂ | (2-Me)-c-Pr |
| 1-48 | OMe | CF₃ | Me |
| 1-49 | OMe | CF₃ | Et |
| 1-50 | OMe | CF₃ | c-Pr |
| 1-51 | OMe | CF₃ | CH₂OMe |
| 1-52 | OMe | CF₃ | CH₂Cl |
| 1-53 | OMe | CF₃ | (1-Me)-c-Pr |
| 1-54 | OMe | CF₃ | (2-Me)-c-Pr |
| 1-55 | OMe | CHF₂ | Me |
| 1-56 | OMe | CHF₂ | Et |
| 1-57 | OMe | CHF₂ | c-Pr |
| 1-58 | SMe | CF₃ | Me |
| 1-59 | SMe | CF₃ | Et |
| 1-60 | SMe | CF₃ | c-Pr |
| 1-61 | SMe | CF₃ | CH₂OMe |
| 1-62 | SMe | CF₃ | CH₂Cl |
| 1-63 | SMe | CF₃ | (1-Me)-c-Pr |
| 1-64 | SMe | CF₃ | (2-Me)-c-Pr |
| 1-65 | SMe | CHF₂ | Me |
| 1-66 | SMe | CHF₂ | Et |
| 1-67 | SMe | CHF₂ | c-Pr |
| 1-68 | SMe | CHF₂ | CH₂OMe |
| 1-69 | SMe | CHF₂ | CH₂Cl |
| 1-70 | SMe | CHF₂ | (1-Me)-c-Pr |
| 1-71 | SMe | CHF₂ | (2-Me)-c-Pr |
| 1-72 | SMe | SO₂Me | Me |
| 1-73 | SMe | SO₂Me | Et |
| 1-74 | SMe | SO₂Me | c-Pr |
| 1-75 | SMe | SO₂Me | CH₂OMe |
| 1-76 | SMe | SO₂Me | CH₂Cl |
| 1-77 | SMe | SO₂Me | (1-Me)-c-Pr |
| 1-78 | SMe | SO₂Me | (2-Me)-c-Pr |
| 1-79 | SEt | CF₃ | Me |
| 1-80 | SEt | CF₃ | Et |
| 1-81 | SEt | CF₃ | c-Pr |
| 1-82 | SEt | CF₃ | CH₂OMe |
| 1-83 | SEt | CF₃ | CH₂Cl |
| 1-84 | SEt | CF₃ | (1-Me)-c-Pr |
| 1-85 | SEt | CF₃ | (2-Me)-c-Pr |
| 1-86 | SEt | CHF₂ | Me |
| 1-87 | SEt | CHF₂ | Et |
| 1-88 | SEt | CHF₂ | c-Pr |
| 1-89 | SEt | CHF₂ | CH₂OMe |
| 1-90 | SEt | CHF₂ | CH₂Cl |
| 1-91 | SEt | CHF₂ | (1-Me)-c-Pr |
| 1-92 | SEt | CHF₂ | (2-Me)-c-Pr |
| 1-93 | SOMe | CF₃ | Me |
| 1-94 | SOMe | CF₃ | Et |
| 1-95 | SOMe | CF₃ | c-Pr |
| 1-96 | SOMe | CHF₂ | Me |
| 1-97 | SOMe | CHF₂ | Et |
| 1-98 | SOMe | CHF₂ | c-Pr |
| 1-99 | SO₂Me | CF₃ | Me |
| 1-100 | SO₂Me | CF₃ | Et |
| 1-101 | SO₂Me | CF₃ | c-Pr |
| 1-102 | SO₂Me | CHF₂ | Me |
| 1-103 | SO₂Me | CHF₂ | Et |
| 1-104 | SO₂Me | CHF₂ | c-Pr |
| 1-105 | SO₂Et | CF₃ | Me |
| 1-106 | SO₂Et | CF₃ | Et |
| 1-107 | SO₂Et | CF₃ | c-Pr |
| 1-108 | F | CF₃ | Me |
| 1-109 | F | CF₃ | Et |
| 1-110 | F | CF₃ | c-Pr |
| 1-111 | F | CHF₂ | Me |
| 1-112 | F | CHF₂ | Et |
| 1-113 | F | CHF₂ | c-Pr |
| 1-114 | Cl | Cl | Me |
| 1-115 | Cl | Cl | Et |
| 1-116 | Cl | Cl | c-Pr |
| 1-117 | Cl | Cl | CH₂OMe |
| 1-118 | Cl | Cl | CH₂Cl |
| 1-119 | Cl | Cl | (1-Me)-c-Pr |
| 1-120 | Cl | Cl | (2-Me)-c-Pr |
| 1-121 | Cl | SMe | Me |
| 1-122 | Cl | SMe | Et |
| 1-123 | Cl | SMe | c-Pr |
| 1-124 | Cl | SOMe | Me |
| 1-125 | Cl | SOMe | Et |
| 1-126 | Cl | SOMe | c-Pr |
| 1-127 | Cl | SO₂Me | Me |
| 1-128 | Cl | SO₂Me | Et |
| 1-129 | Cl | SO₂Me | c-Pr |
| 1-130 | Cl | SO₂Me | CH₂OMe |
| 1-131 | Cl | SO₂Me | CH₂Cl |
| 1-132 | Cl | SO₂Me | (1-Me)-c-Pr |
| 1-133 | Cl | SO₂Me | (2-Me)-c-Pr |
| 1-134 | Cl | Me | Me |
| 1-135 | Cl | Me | Et |
| 1-136 | Cl | Me | c-Pr |
| 1-137 | Cl | CF₃ | Me |
| 1-138 | Cl | CF₃ | Et |
| 1-139 | Cl | CF₃ | n-Pr |
| 1-140 | Cl | CF₃ | i-Pr |
| 1-141 | Cl | CF₃ | c-Pr |
| 1-142 | Cl | CF₃ | n-Bu |
| 1-143 | Cl | CF₃ | t-Bu |
| 1-144 | Cl | CF₃ | CH₂OMe |
| 1-145 | Cl | CF₃ | CH₂Cl |
| 1-146 | Cl | CF₃ | Ac |
| 1-147 | Cl | CF₃ | (1-Me)-c-Pr |
| 1-148 | Cl | CF₃ | (2-Me)-c-Pr |
| 1-149 | Cl | CF₃ | (2,2-Me₂)-c-Pr |
| 1-150 | Cl | CF₃ | (1,2-Me₂)-c-Pr |
| 1-151 | Cl | CF₃ | (2-F)-c-Pr |
| 1-152 | Cl | CF₃ | (2,2-F₂)-c-Pr |
| 1-153 | Cl | CF₃ | c-Bu |
| 1-154 | Cl | CF₃ | c-Pentyl |
| 1-155 | Cl | CF₃ | c-Hexyl |
| 1-156 | Cl | CF₃ | 2-Thienyl |
| 1-157 | Cl | CF₃ | 2-Furyl |
| 1-158 | Cl | CF₃ | Ph |
| 1-159 | Cl | CF₃ | (4-MeO)-Ph |
| 1-160 | Cl | CF₃ | (4-Cl)-Ph |
| 1-161 | Cl | CF₃ | (3-CF₃)-Ph |
| 1-162 | Cl | CF₃ | CF₃ |
| 1-163 | Cl | CF₃ | CHF₂ |
| 1-164 | Cl | CHF₂ | Me |
| 1-165 | Cl | CHF₂ | Et |
| 1-166 | Cl | CHF₂ | n-Pr |
| 1-167 | Cl | CHF₂ | i-Pr |
| 1-168 | Cl | CHF₂ | c-Pr |
| 1-169 | Cl | CHF₂ | n-Bu |
| 1-171 | Cl | CHF₂ | t-Bu |
| 1-172 | Cl | CHF₂ | CH₂OMe |
| 1-173 | Cl | CHF₂ | CH₂Cl |
| 1-174 | Cl | CHF₂ | Ac |
| 1-175 | Cl | CHF₂ | (1-Me)-c-Pr |
| 1-176 | Cl | CHF₂ | (2-Me)-c-Pr |
| 1-177 | Cl | CHF₂ | (2,2-Me₂)-c-Pr |
| 1-178 | Cl | CHF₂ | (1,2-Me₂)-c-Pr |
| 1-179 | Cl | CHF₂ | (2-F)-c-Pr |
| 1-180 | Cl | CHF₂ | (2,2-F₂)-c-Pr |
| 1-181 | Cl | CHF₂ | c-Bu |
| 1-182 | Cl | CHF₂ | c-Pentyl |
| 1-183 | Cl | CHF₂ | c-Hexyl |
| 1-184 | Cl | CHF₂ | 2-Thienyl |
| 1-185 | Cl | CHF₂ | 2-Furyl |
| 1-186 | Cl | CHF₂ | Ph |
| 1-187 | Cl | CHF₂ | (4-MeO)-Ph |
| 1-188 | Cl | CHF₂ | (4-Cl)-Ph |
| 1-189 | Cl | CHF₂ | (3-CF₃)-Ph |
| 1-190 | Cl | CHF₂ | CF₃ |
| 1-191 | Cl | CHF₂ | CHF₂ |
| 1-192 | Cl | I | Me |
| 1-193 | Cl | I | Et |
| 1-194 | Cl | I | c-Pr |
| 1-195 | Br | CF₃ | Me |
| 1-196 | Br | CF₃ | Et |
| 1-197 | Br | CF₃ | c-Pr |
| 1-198 | Br | CF₃ | CH₂OMe |
| 1-199 | Br | CF₃ | CH₂Cl |
| 1-200 | Br | CF₃ | (1-Me)-c-Pr |
| 1-201 | Br | CF₃ | (2-Me)-c-Pr |
| 1-202 | Br | CHF₂ | Me |
| 1-203 | Br | CHF₂ | Et |
| 1-204 | Br | CHF₂ | c-Pr |
| 1-205 | Br | CHF₂ | CH₂OMe |
| 1-206 | Br | CHF₂ | CH₂Cl |
| 1-207 | Br | CHF₂ | (1-Me)-c-Pr |
| 1-208 | Br | CHF₂ | (2-Me)-c-Pr |
| 1-209 | Br | SO₂Me | Me |
| 1-210 | Br | SO₂Me | Et |
| 1-211 | Br | SO₂Me | c-Pr |
| 1-212 | Br | SO₂Me | CH₂OMe |
| 1-213 | Br | SO₂Me | CH₂Cl |
| 1-214 | Br | SO₂Me | (1-Me )-c-Pr |
| 1-215 | Br | SO₂Me | (2-Me)-c-Pr |
| 1-216 | CH₂OMe | CF₃ | Me |
| 1-217 | CH₂OMe | CF₃ | Et |
| 1-218 | CH₂OMe | CF₃ | c-Pr |
| 1-219 | CH₂OMe | SO₂Me | Me |
| 1-220 | CH₂OMe | SO₂Me | Et |
| 1-221 | CH₂OMe | SO₂Me | c-Pr |
| 1-222 | Et | CF₃ | Me |
| 1-223 | Et | CF₃ | Et |
| 1-224 | Et | CF₃ | c-Pr |
| 1-225 | Et | CHF₂ | Me |
| 1-226 | Et | CHF₂ | Et |
| 1-227 | Et | CHF₂ | c-Pr |
| 1-228 | Et | SO₂Me | Me |
| 1-229 | Et | SO₂Me | Et |
| 1-230 | Et | SO₂Me | c-Pr |
| 1-231 | c-Pr | CF₃ | Me |
| 1-232 | c-Pr | CF₃ | Et |
| 1-233 | c-Pr | CF₃ | c-Pr |
| 1-234 | c-Pr | CF₃ | CH₂OMe |
| 1-235 | c-Pr | CF₃ | CH₂Cl |
| 1-236 | c-Pr | CF₃ | (1-Me)-c-Pr |
| 1-237 | c-Pr | CF₃ | (2-Me)-c-Pr |
| 1-238 | c-Pr | CHF₂ | Me |
| 1-239 | c-Pr | CHF₂ | Et |
| 1-240 | c-Pr | CHF₂ | c-Pr |
| 1-241 | c-Pr | CHF₂ | CH₂OMe |
| 1-242 | c-Pr | CHF₂ | CH₂Cl |
| 1-243 | c-Pr | CHF₂ | (1-Me)-c-Pr |
| 1-244 | c-Pr | CHF₂ | (2-Me)-c-Pr |
| 1-245 | c-Pr | SO₂Me | Me |
| 1-246 | c-Pr | SO₂Me | Et |
| 1-247 | c-Pr | SO₂Me | c-Pr |
| 1-248 | c-Pr | SO₂Me | CH₂OMe |
| 1-249 | c-Pr | SO₂Me | CH₂Cl |
| 1-250 | c-Pr | SO₂Me | (1-Me)-c-Pr |
| 1-251 | c-Pr | SO₂Me | (2-Me)-c-Pr |
| 1-252 | I | CF₃ | Me |
| 1-253 | I | CF₃ | Et |
| 1-254 | I | CF₃ | c-Pr |
| 1-255 | I | CF₃ | CH₂OMe |
| 1-256 | I | CF₃ | CH₂Cl |
| 1-257 | I | CF₃ | (1-Me)-c-Pr |
| 1-258 | I | CF₃ | (2-Me)-c-Pr |
| 1-259 | I | CHF₂ | Me |
| 1-260 | I | CHF₂ | Et |
| 1-261 | I | CHF₂ | c-Pr |
| 1-262 | I | CHF₂ | CH₂OMe |
| 1-263 | I | CHF₂ | CH₂Cl |
| 1-264 | I | CHF₂ | (1-Me)-c-Pr |
| 1-265 | I | CHF₂ | (2-Me)-c-Pr |
| 1-266 | I | SO₂Me | Me |
| 1-267 | I | SO₂Me | Et |
| 1-268 | I | SO₂Me | c-Pr |
| 1-269 | I | SO₂Me | CH₂OMe |
| 1-270 | I | SO₂Me | CH₂Cl |
| 1-271 | I | SO₂Me | (1-Me)-c-Pr |
| 1-272 | I | SO₂Me | (2-Me)-c-Pr |
| 1-273 | CF₃ | CF₃ | Me |
| 1-274 | CF₃ | CF₃ | Et |
| 1-275 | CF₃ | CF₃ | c-Pr |
| 1-276 | CF₃ | CF₃ | CH₂OMe |
| 1-277 | CF₃ | CF₃ | CH₂Cl |
| 1-278 | CF₃ | CF₃ | (1-Me)-c-Pr |
| 1-279 | CF₃ | CF₃ | (2-Me)-c-Pr |
| 1-280 | Cl | Cl | i-Pr |
| 1-281 | Cl | Cl | c-Pentyl |
| 1-282 | Cl | Cl | 2-Thienyl |
| 1-283 | Cl | Cl | (4-MeO)-Ph |
| 1-284 | SMe | CF₃ | c-Bu |
| 1-285 | SMe | CF₃ | c-Pentyl |
| 1-286 | SMe | CF₃ | c-Hexyl |
| 1-287 | Cl | CF₃ | (3,5-F₂)-Ph |
| 1-288 | SMe | CF₃ | (3,5-F₂)-Ph |
| 1-289 | Cl | Br | Me |
| 1-290 | Cl | Br | Et |
| 1-291 | Cl | Br | c-Pr |
| 1-292 | Me | Cl | Me |
| 1-293 | Me | Cl | Et |
| 1-294 | Me | Cl | c-Pr |
| 1-295 | Cl | CF₃ | Vinyl |

**Tabelle 2: Erfindungsgemäße Verbindungen der Formel (I), worin R^{x} für eine Ethylgruppe steht und die anderen Substituenten die unten genannten Bedeutungen haben.**

| | | | |
|---|---|---|---|
| 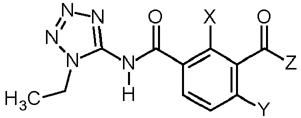 | | | |

| Nr. | X | Y | Z |
|---|---|---|---|
| 2-1 | Me | Me | Me |
| 2-2 | Me | Me | Et |
| 2-3 | Me | Me | c-Pr |
| 2-4 | Me | SMe | Me |
| 2-5 | Me | SMe | Et |
| 2-6 | Me | SMe | c-Pr |
| 2-7 | Me | SO₂Me | Me |
| 2-8 | Me | SO₂Me | Et |
| 2-9 | Me | SO₂Me | c-Pr |
| 2-10 | Me | SO₂Me | CH₂OMe |
| 2-11 | Me | SO₂Me | CH₂Cl |
| 2-12 | Me | SO₂Me | (1-Me)-c-Pr |
| 2-13 | Me | SO₂Me | (2-Me)-c-Pr |
| 2-14 | Me | CF₃ | Me |
| 2-15 | Me | CF₃ | Et |
| 2-16 | Me | CF₃ | n-Pr |
| 2-17 | Me | CF₃ | i-Pr |
| 2-18 | Me | CF₃ | c-Pr |
| 2-19 | Me | CF₃ | n-Bu |
| 2-20 | Me | CF₃ | t-Bu |
| 2-21 | Me | CF₃ | CH₂OMe |
| 2-22 | Me | CF₃ | CH₂Cl |
| 2-23 | Me | CF₃ | Ac |
| 2-24 | Me | CF₃ | (1-Me)-c-Pr |
| 2-25 | Me | CF₃ | (2-Me)-c-Pr |
| 2-26 | Me | CF₃ | (2,2-Me₂)-c-Pr |
| 2-27 | Me | CF₃ | (1,2-Me₂)-c-Pr |
| 2-28 | Me | CF₃ | (2-F)-c-Pr |
| 2-29 | Me | CF₃ | (2,2-F₂)-c-Pr |
| 2-30 | Me | CF₃ | c-Bu |
| 2-31 | Me | CF₃ | c-Pentyl |
| 2-32 | Me | CF₃ | c-Hexyl |
| 2-33 | Me | CF₃ | 2-Thienyl |
| 2-34 | Me | CF₃ | 2-Furyl |
| 2-35 | Me | CF₃ | Ph |
| 2-36 | Me | CF₃ | (4-MeO)-Ph |
| 2-37 | Me | CF₃ | (4-Cl)-Ph |
| 2-38 | Me | CF₃ | (3-CF₃)-Ph |
| 2-39 | Me | CF₃ | CF₃ |
| 2-40 | Me | CF₃ | CHF₂ |
| 2-41 | Me | CHF₂ | Me |
| 2-42 | Me | CHF₂ | Et |
| 2-43 | Me | CHF₂ | c-Pr |
| 2-44 | Me | CHF₂ | CH₂OMe |
| 2-45 | Me | CHF₂ | CH₂Cl |
| 2-46 | Me | CHF₂ | (1-Me)-c-Pr |
| 2-47 | Me | CHF₂ | (2-Me)-c-Pr |
| 2-48 | OMe | CF₃ | Me |
| 2-49 | OMe | CF₃ | Et |
| 2-50 | OMe | CF₃ | c-Pr |
| 2-51 | OMe | CF₃ | CH₂OMe |
| 2-52 | OMe | CF₃ | CH₂Cl |
| 2-53 | OMe | CF₃ | (1-Me)-c-Pr |
| 2-54 | OMe | CF₃ | (2-Me)-c-Pr |
| 2-55 | OMe | CHF₂ | Me |
| 2-56 | OMe | CHF₂ | Et |
| 2-57 | OMe | CHF₂ | c-Pr |
| 2-58 | SMe | CF₃ | Me |
| 2-59 | SMe | CF₃ | Et |
| 2-60 | SMe | CF₃ | c-Pr |
| 2-61 | SMe | CF₃ | CH₂OMe |
| 2-62 | SMe | CF₃ | CH₂Cl |
| 2-63 | SMe | CF₃ | (1-Me)-c-Pr |
| 2-64 | SMe | CF₃ | (2-Me)-c-Pr |
| 2-65 | SMe | CHF₂ | Me |
| 2-66 | SMe | CHF₂ | Et |
| 2-67 | SMe | CHF₂ | c-Pr |
| 2-68 | SMe | CHF₂ | CH₂OMe |
| 2-69 | SMe | CHF₂ | CH₂Cl |
| 2-70 | SMe | CHF₂ | (1-Me)-c-Pr |
| 2-71 | SMe | CHF₂ | (2-Me)-c-Pr |
| 2-72 | SMe | SO₂Me | Me |
| 2-73 | SMe | SO₂Me | Et |
| 2-74 | SMe | SO₂Me | c-Pr |
| 2-75 | SMe | SO₂Me | CH₂OMe |
| 2-76 | SMe | SO₂Me | CH₂Cl |
| 2-77 | SMe | SO₂Me | (1-Me)-c-Pr |
| 2-78 | SMe | SO₂Me | (2-Me)-c-Pr |
| 2-79 | SEt | CF₃ | Me |
| 2-80 | SEt | CF₃ | Et |
| 2-81 | SEt | CF₃ | c-Pr |
| 2-82 | SEt | CF₃ | CH₂OMe |
| 2-83 | SEt | CF₃ | CH₂Cl |
| 2-84 | SEt | CF₃ | (1-Me)-c-Pr |
| 2-85 | SEt | CF₃ | (2-Me)-c-Pr |
| 2-86 | SEt | CHF₂ | Me |
| 2-87 | SEt | CHF₂ | Et |
| 2-88 | SEt | CHF₂ | c-Pr |
| 2-89 | SEt | CHF₂ | CH₂OMe |
| 2-90 | SEt | CHF₂ | CH₂Cl |
| 2-91 | SEt | CHF₂ | (1-Me)-c-Pr |
| 2-92 | SEt | CHF₂ | (2-Me)-c-Pr |
| 2-93 | SOMe | CF₃ | Me |
| 2-94 | SOMe | CF₃ | Et |
| 2-95 | SOMe | CF₃ | c-Pr |
| 2-96 | SOMe | CHF₂ | Me |
| 2-97 | SOMe | CHF₂ | Et |
| 2-98 | SOMe | CHF₂ | c-Pr |
| 2-99 | SO₂Me | CF₃ | Me |
| 2-100 | SO₂Me | CF₃ | Et |
| 2-101 | SO₂Me | CF₃ | c-Pr |
| 2-102 | SO₂Me | CHF₂ | Me |
| 2-103 | SO₂Me | CHF₂ | Et |
| 2-104 | SO₂Me | CHF₂ | c-Pr |
| 2-105 | SO₂Et | CF₃ | Me |
| 2-106 | SO₂Et | CF₃ | Et |
| 2-107 | SO₂Et | CF₃ | c-Pr |
| 2-108 | F | CF₃ | Me |
| 2-109 | F | CF₃ | Et |
| 2-110 | F | CF₃ | c-Pr |
| 2-111 | F | CHF₂ | Me |
| 2-112 | F | CHF₂ | Et |
| 2-113 | F | CHF₂ | c-Pr |
| 2-114 | Cl | Cl | Me |
| 2-115 | Cl | Cl | Et |
| 2-116 | Cl | Cl | c-Pr |
| 2-117 | Cl | Cl | CH₂OMe |
| 2-118 | Cl | Cl | CH₂Cl |
| 2-119 | Cl | Cl | (1-Me)-c-Pr |
| 2-120 | Cl | Cl | (2-Me)-c-Pr |
| 2-121 | Cl | SMe | Me |
| 2-122 | Cl | SMe | Et |
| 2-123 | Cl | SMe | c-Pr |
| 2-124 | Cl | SOMe | Me |
| 2-125 | Cl | SOMe | Et |
| 2-126 | Cl | SOMe | c-Pr |
| 2-127 | Cl | SO₂Me | Me |
| 2-128 | Cl | SO₂Me | Et |
| 2-129 | Cl | SO₂Me | c-Pr |
| 2-130 | Cl | SO₂Me | CH₂OMe |
| 2-131 | Cl | SO₂Me | CH₂Cl |
| 2-132 | Cl | SO₂Me | (1-Me)-c-Pr |
| 2-133 | Cl | SO₂Me | (2-Me)-c-Pr |
| 2-134 | Cl | Me | Me |
| 2-135 | Cl | Me | Et |
| 2-136 | Cl | Me | c-Pr |
| 2-137 | Cl | CF₃ | Me |
| 2-138 | Cl | CF₃ | Et |
| 2-139 | Cl | CF₃ | n-Pr |
| 2-140 | Cl | CF₃ | i-Pr |
| 2-141 | Cl | CF₃ | c-Pr |
| 2-142 | Cl | CF₃ | n-Bu |
| 2-143 | Cl | CF₃ | t-Bu |
| 2-144 | Cl | CF₃ | CH₂OMe |
| 2-145 | Cl | CF₃ | CH₂Cl |
| 2-146 | Cl | CF₃ | Ac |
| 2-147 | Cl | CF₃ | (1-Me )-c-Pr |
| 2-148 | Cl | CF₃ | (2-Me)-c-Pr |
| 2-149 | Cl | CF₃ | (2,2-Me₂)-c-Pr |
| 2-150 | Cl | CF₃ | (1,2-Me₂)-c-Pr |
| 2-151 | Cl | CF₃ | (2-F)-c-Pr |
| 2-152 | Cl | CF₃ | (2,2-F₂)-c-Pr |
| 2-153 | Cl | CF₃ | c-Bu |
| 2-154 | Cl | CF₃ | c-Pentyl |
| 2-155 | Cl | CF₃ | c-Hexyl |
| 2-156 | Cl | CF₃ | 2-Thienyl |
| 2-157 | Cl | CF₃ | 2-Furyl |
| 2-158 | Cl | CF₃ | Ph |
| 2-159 | Cl | CF₃ | (4-MeO)-Ph |
| 2-160 | Cl | CF₃ | (4-Cl)-Ph |
| 2-161 | Cl | CF₃ | (3-CF₃)-Ph |
| 2-162 | Cl | CF₃ | CF₃ |
| 2-163 | Cl | CF₃ | CHF₂ |
| 2-164 | Cl | CHF₂ | Me |
| 2-165 | Cl | CHF₂ | Et |
| 2-166 | Cl | CHF₂ | n-Pr |
| 2-167 | Cl | CHF₂ | i-Pr |
| 2-168 | Cl | CHF₂ | c-Pr |
| 2-169 | Cl | CHF₂ | n-Bu |
| 2-171 | Cl | CHF₂ | t-Bu |
| 2-172 | Cl | CHF₂ | CH₂OMe |
| 2-173 | Cl | CHF₂ | CH₂Cl |
| 2-174 | Cl | CHF₂ | Ac |
| 2-175 | Cl | CHF₂ | (1-Me)-c-Pr |
| 2-176 | Cl | CHF₂ | (2-Me)-c-Pr |
| 2-177 | Cl | CHF₂ | (2,2-Me₂)-c-Pr |
| 2-178 | Cl | CHF₂ | (1,2-Me₂)-c-Pr |
| 2-179 | Cl | CHF₂ | (2-F)-c-Pr |
| 2-180 | Cl | CHF₂ | (2,2-F₂)-c-Pr |
| 2-181 | Cl | CHF₂ | c-Bu |
| 2-182 | Cl | CHF₂ | c-Pentyl |
| 2-183 | Cl | CHF₂ | c-Hexyl |
| 2-184 | Cl | CHF₂ | 2-Thienyl |
| 2-185 | Cl | CHF₂ | 2-Furyl |
| 2-186 | Cl | CHF₂ | Ph |
| 2-187 | Cl | CHF₂ | (4-MeO)-Ph |
| 2-188 | Cl | CHF₂ | (4-Cl)-Ph |
| 2-189 | Cl | CHF₂ | (3-CF₃)-Ph |
| 2-190 | Cl | CHF₂ | CF₃ |
| 2-191 | Cl | CHF₂ | CHF₂ |
| 2-192 | Cl | I | Me |
| 2-193 | Cl | I | Et |
| 2-194 | Cl | I | c-Pr |
| 2-195 | Br | CF₃ | Me |
| 2-196 | Br | CF₃ | Et |
| 2-197 | Br | CF₃ | c-Pr |
| 2-198 | Br | CF₃ | CH₂OMe |
| 2-199 | Br | CF₃ | CH₂Cl |
| 2-200 | Br | CF₃ | (1-Me)-c-Pr |
| 2-201 | Br | CF₃ | (2-Me)-c-Pr |
| 2-202 | Br | CHF₂ | Me |
| 2-203 | Br | CHF₂ | Et |
| 2-204 | Br | CHF₂ | c-Pr |
| 2-205 | Br | CHF₂ | CH₂OMe |
| 2-206 | Br | CHF₂ | CH₂Cl |
| 2-207 | Br | CHF₂ | (1-Me)-c-Pr |
| 2-208 | Br | CHF₂ | (2-Me)-c-Pr |
| 2-209 | Br | SO₂Me | Me |
| 2-210 | Br | SO₂Me | Et |
| 2-211 | Br | SO₂Me | c-Pr |
| 2-212 | Br | SO₂Me | CH₂OMe |
| 2-213 | Br | SO₂Me | CH₂Cl |
| 2-214 | Br | SO₂Me | (1-Me)-c-Pr |
| 2-215 | Br | SO₂Me | (2-Me)-c-Pr |
| 2-216 | CH₂OMe | CF₃ | Me |
| 2-217 | CH₂OMe | CF₃ | Et |
| 2-218 | CH₂OMe | CF₃ | c-Pr |
| 2-219 | CH₂OMe | SO₂Me | Me |
| 2-220 | CH₂OMe | SO₂Me | Et |
| 2-221 | CH₂OMe | SO₂Me | c-Pr |
| 2-222 | Et | CF₃ | Me |
| 2-223 | Et | CF₃ | Et |
| 2-224 | Et | CF₃ | c-Pr |
| 2-225 | Et | CHF₂ | Me |
| 2-226 | Et | CHF₂ | Et |
| 2-227 | Et | CHF₂ | c-Pr |
| 2-228 | Et | SO₂Me | Me |
| 2-229 | Et | SO₂Me | Et |
| 2-230 | Et | SO₂Me | c-Pr |
| 2-231 | c-Pr | CF₃ | Me |
| 2-232 | c-Pr | CF₃ | Et |
| 2-233 | c-Pr | CF₃ | c-Pr |
| 2-234 | c-Pr | CF₃ | CH₂OMe |
| 2-235 | c-Pr | CF₃ | CH₂Cl |
| 2-236 | c-Pr | CF₃ | (1-Me)-c-Pr |
| 2-237 | c-Pr | CF₃ | (2-Me)-c-Pr |
| 2-238 | c-Pr | CHF₂ | Me |
| 2-239 | c-Pr | CHF₂ | Et |
| 2-240 | c-Pr | CHF₂ | c-Pr |
| 2-241 | c-Pr | CHF₂ | CH₂OMe |
| 2-242 | c-Pr | CHF₂ | CH₂Cl |
| 2-243 | c-Pr | CHF₂ | (1-Me)-c-Pr |
| 2-244 | c-Pr | CHF₂ | (2-Me)-c-Pr |
| 2-245 | c-Pr | SO₂Me | Me |
| 2-246 | c-Pr | SO₂Me | Et |
| 2-247 | c-Pr | SO₂Me | c-Pr |
| 2-248 | c-Pr | SO₂Me | CH₂OMe |
| 2-249 | c-Pr | SO₂Me | CH₂Cl |
| 2-250 | c-Pr | SO₂Me | (1-Me)-c-Pr |
| 2-251 | c-Pr | SO₂Me | (2-Me)-c-Pr |
| 2-252 | I | CF₃ | Me |
| 2-253 | I | CF₃ | Et |
| 2-254 | I | CF₃ | c-Pr |
| 2-255 | I | CF₃ | CH₂OMe |
| 2-256 | I | CF₃ | CH₂Cl |
| 2-257 | I | CF₃ | (1-Me)-c-Pr |
| 2-258 | I | CF₃ | (2-Me)-c-Pr |
| 2-259 | I | CHF₂ | Me |
| 2-260 | I | CHF₂ | Et |
| 2-261 | I | CHF₂ | c-Pr |
| 2-262 | I | CHF₂ | CH₂OMe |
| 2-263 | I | CHF₂ | CH₂Cl |
| 2-264 | I | CHF₂ | (1-Me)-c-Pr |
| 2-265 | I | CHF₂ | (2-Me)-c-Pr |
| 2-266 | I | SO₂Me | Me |
| 2-267 | I | SO₂Me | Et |
| 2-268 | I | SO₂Me | c-Pr |
| 2-269 | I | SO₂Me | CH₂OMe |
| 2-270 | I | SO₂Me | CH₂Cl |
| 2-271 | I | SO₂Me | (1-Me)-c-Pr |
| 2-272 | I | SO₂Me | (2-Me)-c-Pr |
| 2-273 | CF₃ | CF₃ | Me |
| 2-274 | CF₃ | CF₃ | Et |
| 2-275 | CF₃ | CF₃ | c-Pr |
| 2-276 | CF₃ | CF₃ | CH₂OMe |
| 2-277 | CF₃ | CF₃ | CH₂Cl |
| 2-278 | CF₃ | CF₃ | (1-Me)-c-Pr |
| 2-279 | CF₃ | CF₃ | (2-Me)-c-Pr |
| 2-280 | Cl | Cl | i-Pr |
| 2-281 | Cl | Cl | c-Pentyl |
| 2-282 | Cl | Cl | 2-Thienyl |
| 2-283 | Cl | Cl | (4-MeO)-Ph |
| 2-284 | SMe | CF₃ | c-Bu |
| 2-285 | SMe | CF₃ | c-Hexyl |
| 2-286 | Cl | CF₃ | (3,5-F₂)-Ph |
| 2-287 | SMe | CF₃ | (3,5-F₂)-Ph |
| 2-288 | Cl | Br | Me |
| 2-289 | Cl | Br | Et |
| 2-290 | Cl | Br | c-Pr |
| 2-291 | Me | Cl | Me |
| 2-292 | Me | Cl | Et |
| 2-293 | Me | Cl | c-Pr |
| 2-294 | Cl | CF₃ | Vinyl |

**Tabelle 3: Erfindungsgemäße Verbindungen der Formel (I), worin R^{x} für eine Propylgruppe steht und die anderen Substituenten die unten genannten Bedeutungen haben.**

| | | | |
|---|---|---|---|
| 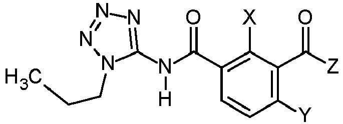 | | | |

| Nr. | X | Y | Z |
|---|---|---|---|
| 3-1 | Me | Me | Me |
| 3-2 | Me | Me | Et |
| 3-3 | Me | Me | c-Pr |
| 3-4 | Me | SMe | Me |
| 3-5 | Me | SMe | Et |
| 3-6 | Me | SMe | c-Pr |
| 3-7 | Me | SO₂Me | Me |
| 3-8 | Me | SO₂Me | Et |
| 3-9 | Me | SO₂Me | c-Pr |
| 3-10 | Me | SO₂Me | CH₂OMe |
| 3-11 | Me | SO₂Me | CH₂Cl |
| 3-12 | Me | SO₂Me | (1-Me)-c-Pr |
| 3-13 | Me | SO₂Me | (2-Me)-c-Pr |
| 3-14 | Me | CF₃ | Me |
| 3-15 | Me | CF₃ | Et |
| 3-16 | Me | CF₃ | n-Pr |
| 3-17 | Me | CF₃ | i-Pr |
| 3-18 | Me | CF₃ | c-Pr |
| 3-19 | Me | CF₃ | n-Bu |
| 3-20 | Me | CF₃ | t-Bu |
| 3-21 | Me | CF₃ | CH₂OMe |
| 3-22 | Me | CF₃ | CH₂Cl |
| 3-23 | Me | CF₃ | Ac |
| 3-24 | Me | CF₃ | (1-Me)-c-Pr |
| 3-25 | Me | CF₃ | (2-Me)-c-Pr |
| 3-26 | Me | CF₃ | (2,2-Me₂)-c-Pr |
| 3-27 | Me | CF₃ | (1,2-Me₂)-c-Pr |
| 3-28 | Me | CF₃ | (2-F)-c-Pr |
| 3-29 | Me | CF₃ | (2,2-F₂)-c-Pr |
| 3-30 | Me | CF₃ | c-Bu |
| 3-31 | Me | CF₃ | c-Pentyl |
| 3-32 | Me | CF₃ | c-Hexyl |
| 3-33 | Me | CF₃ | 2-Thienyl |
| 3-34 | Me | CF₃ | 2-Furyl |
| 3-35 | Me | CF₃ | Ph |
| 3-36 | Me | CF₃ | (4-MeO)-Ph |
| 3-37 | Me | CF₃ | (4-Cl)-Ph |
| 3-38 | Me | CF₃ | (3-CF₃)-Ph |
| 3-39 | Me | CF₃ | CF₃ |
| 3-40 | Me | CF₃ | CHF₂ |
| 3-41 | Me | CHF₂ | Me |
| 3-42 | Me | CHF₂ | Et |
| 3-43 | Me | CHF₂ | c-Pr |
| 3-44 | Me | CHF₂ | CH₂OMe |
| 3-45 | Me | CHF₂ | CH₂Cl |
| 3-46 | Me | CHF₂ | (1-Me)-c-Pr |
| 3-47 | Me | CHF₂ | (2-Me)-c-Pr |
| 3-48 | OMe | CF₃ | Me |
| 3-49 | OMe | CF₃ | Et |
| 3-50 | OMe | CF₃ | c-Pr |
| 3-51 | OMe | CF₃ | CH₂OMe |
| 3-52 | OMe | CF₃ | CH₂Cl |
| 3-53 | OMe | CF₃ | (1-Me)-c-Pr |
| 3-54 | OMe | CF₃ | (2-Me)-c-Pr |
| 3-55 | OMe | CHF₂ | Me |
| 3-56 | OMe | CHF₂ | Et |
| 3-57 | OMe | CHF₂ | c-Pr |
| 3-58 | SMe | CF₃ | Me |
| 3-59 | SMe | CF₃ | Et |
| 3-60 | SMe | CF₃ | c-Pr |
| 3-61 | SMe | CF₃ | CH₂OMe |
| 3-62 | SMe | CF₃ | CH₂Cl |
| 3-63 | SMe | CF₃ | (1-Me)-c-Pr |
| 3-64 | SMe | CF₃ | (2-Me)-c-Pr |
| 3-65 | SMe | CHF₂ | Me |
| 3-66 | SMe | CHF₂ | Et |
| 3-67 | SMe | CHF₂ | c-Pr |
| 3-68 | SMe | CHF₂ | CH₂OMe |
| 3-69 | SMe | CHF₂ | CH₂Cl |
| 3-70 | SMe | CHF₂ | (1-Me)-c-Pr |
| 3-71 | SMe | CHF₂ | (2-Me)-c-Pr |
| 3-72 | SMe | SO₂Me | Me |
| 3-73 | SMe | SO₂Me | Et |
| 3-74 | SMe | SO₂Me | c-Pr |
| 3-75 | SMe | SO₂Me | CH₂OMe |
| 3-76 | SMe | SO₂Me | CH₂Cl |
| 3-77 | SMe | SO₂Me | (1-Me)-c-Pr |
| 3-78 | SMe | SO₂Me | (2-Me)-c-Pr |
| 3-79 | SEt | CF₃ | Me |
| 3-80 | SEt | CF₃ | Et |
| 3-81 | SEt | CF₃ | c-Pr |
| 3-82 | SEt | CF₃ | CH₂OMe |
| 3-83 | SEt | CF₃ | CH₂Cl |
| 3-84 | SEt | CF₃ | (1-Me)-c-Pr |
| 3-85 | SEt | CF₃ | (2-Me)-c-Pr |
| 3-86 | SEt | CHF₂ | Me |
| 3-87 | SEt | CHF₂ | Et |
| 3-88 | SEt | CHF₂ | c-Pr |
| 3-89 | SEt | CHF₂ | CH₂OMe |
| 3-90 | SEt | CHF₂ | CH₂Cl |
| 3-91 | SEt | CHF₂ | (1-Me)-c-Pr |
| 3-92 | SEt | CHF₂ | (2-Me)-c-Pr |
| 3-93 | SOMe | CF₃ | Me |
| 3-94 | SOMe | CF₃ | Et |
| 3-95 | SOMe | CF₃ | c-Pr |
| 3-96 | SOMe | CHF₂ | Me |
| 3-97 | SOMe | CHF₂ | Et |
| 3-98 | SOMe | CHF₂ | c-Pr |
| 3-99 | SO₂Me | CF₃ | Me |
| 3-100 | SO₂Me | CF₃ | Et |
| 3-101 | SO₂Me | CF₃ | c-Pr |
| 3-102 | SO₂Me | CHF₂ | Me |
| 3-103 | SO₂Me | CHF₂ | Et |
| 3-104 | SO₂Me | CHF₂ | c-Pr |
| 3-105 | SO₂Et | CF₃ | Me |
| 3-106 | SO₂Et | CF₃ | Et |
| 3-107 | SO₂Et | CF₃ | c-Pr |
| 3-108 | F | CF₃ | Me |
| 3-109 | F | CF₃ | Et |
| 3-110 | F | CF₃ | c-Pr |
| 3-111 | F | CHF₂ | Me |
| 3-112 | F | CHF₂ | Et |
| 3-113 | F | CHF₂ | c-Pr |
| 3-114 | Cl | Cl | Me |
| 3-115 | Cl | Cl | Et |
| 3-116 | Cl | Cl | c-Pr |
| 3-117 | Cl | Cl | CH₂OMe |
| 3-118 | Cl | Cl | CH₂Cl |
| 3-119 | Cl | Cl | (1-Me)-c-Pr |
| 3-120 | Cl | Cl | (2-Me)-c-Pr |
| 3-121 | Cl | SMe | Me |
| 3-122 | Cl | SMe | Et |
| 3-123 | Cl | SMe | c-Pr |
| 3-124 | Cl | SOMe | Me |
| 3-125 | Cl | SOMe | Et |
| 3-126 | Cl | SOMe | c-Pr |
| 3-127 | Cl | SO₂Me | Me |
| 3-128 | Cl | SO₂Me | Et |
| 3-129 | Cl | SO₂Me | c-Pr |
| 3-130 | Cl | SO₂Me | CH₂OMe |
| 3-131 | Cl | SO₂Me | CH₂Cl |
| 3-132 | Cl | SO₂Me | (1-Me)-c-Pr |
| 3-133 | Cl | SO₂Me | (2-Me)-c-Pr |
| 3-134 | Cl | Me | Me |
| 3-135 | Cl | Me | Et |
| 3-136 | Cl | Me | c-Pr |
| 3-137 | Cl | CF₃ | Me |
| 3-138 | Cl | CF₃ | Et |
| 3-139 | Cl | CF₃ | n-Pr |
| 3-140 | Cl | CF₃ | i-Pr |
| 3-141 | Cl | CF₃ | c-Pr |
| 3-142 | Cl | CF₃ | n-Bu |
| 3-143 | Cl | CF₃ | t-Bu |
| 3-144 | Cl | CF₃ | CH₂OMe |
| 3-145 | Cl | CF₃ | CH₂Cl |
| 3-146 | Cl | CF₃ | Ac |
| 3-147 | Cl | CF₃ | (1-Me)-c-Pr |
| 3-148 | Cl | CF₃ | (2-Me)-c-Pr |
| 3-149 | Cl | CF₃ | (2,2-Me₂)-c-Pr |
| 3-150 | Cl | CF₃ | (1,2-Me₂)-c-Pr |
| 3-151 | Cl | CF₃ | (2-F)-c-Pr |
| 3-152 | Cl | CF₃ | (2,2-F₂)-c-Pr |
| 3-153 | Cl | CF₃ | c-Bu |
| 3-154 | Cl | CF₃ | c-Pentyl |
| 3-155 | Cl | CF₃ | c-Hexyl |
| 3-156 | Cl | CF₃ | 2-Thienyl |
| 3-157 | Cl | CF₃ | 2-Furyl |
| 3-158 | Cl | CF₃ | Ph |
| 3-159 | Cl | CF₃ | (4-MeO)-Ph |
| 3-160 | Cl | CF₃ | (4-Cl)-Ph |
| 3-161 | Cl | CF₃ | (3-CF₃)-Ph |
| 3-162 | Cl | CF₃ | CF₃ |
| 3-163 | Cl | CF₃ | CHF₂ |
| 3-164 | Cl | CHF₂ | Me |
| 3-165 | Cl | CHF₂ | Et |
| 3-166 | Cl | CHF₂ | n-Pr |
| 3-167 | Cl | CHF₂ | i-Pr |
| 3-168 | Cl | CHF₂ | c-Pr |
| 3-169 | Cl | CHF₂ | n-Bu |
| 3-171 | Cl | CHF₂ | t-Bu |
| 3-172 | Cl | CHF₂ | CH₂OMe |
| 3-173 | Cl | CHF₂ | CH₂Cl |
| 3-174 | Cl | CHF₂ | Ac |
| 3-175 | Cl | CHF₂ | (1-Me)-c-Pr |
| 3-176 | Cl | CHF₂ | (2-Me)-c-Pr |
| 3-177 | Cl | CHF₂ | (2,2-Me₂)-c-Pr |
| 3-178 | Cl | CHF₂ | (1,2-Me₂)-c-Pr |
| 3-179 | Cl | CHF₂ | (2-F)-c-Pr |
| 3-180 | Cl | CHF₂ | (2,2-F₂)-c-Pr |
| 3-181 | Cl | CHF₂ | c-Bu |
| 3-182 | Cl | CHF₂ | c-Pentyl |
| 3-183 | Cl | CHF₂ | c-Hexyl |
| 3-184 | Cl | CHF₂ | 2-Thienyl |
| 3-185 | Cl | CHF₂ | 2-Furyl |
| 3-186 | Cl | CHF₂ | Ph |
| 3-187 | Cl | CHF₂ | (4-MeO)-Ph |
| 3-188 | Cl | CHF₂ | (4-Cl)-Ph |
| 3-189 | Cl | CHF₂ | (3-CF₃)-Ph |
| 3-190 | Cl | CHF₂ | CF₃ |
| 3-191 | Cl | CHF₂ | CHF₂ |
| 3-192 | Cl | I | Me |
| 3-193 | Cl | I | Et |
| 3-194 | Cl | I | c-Pr |
| 3-195 | Br | CF₃ | Me |
| 3-196 | Br | CF₃ | Et |
| 3-197 | Br | CF₃ | c-Pr |
| 3-198 | Br | CF₃ | CH₂OMe |
| 3-199 | Br | CF₃ | CH₂Cl |
| 3-200 | Br | CF₃ | (1-Me)-c-Pr |
| 3-201 | Br | CF₃ | (2-Me)-c-Pr |
| 3-202 | Br | CHF₂ | Me |
| 3-203 | Br | CHF₂ | Et |
| 3-204 | Br | CHF₂ | c-Pr |
| 3-205 | Br | CHF₂ | CH₂OMe |
| 3-206 | Br | CHF₂ | CH₂Cl |
| 3-207 | Br | CHF₂ | (1-Me)-c-Pr |
| 3-208 | Br | CHF₂ | (2-Me)-c-Pr |
| 3-209 | Br | SO₂Me | Me |
| 3-210 | Br | SO₂Me | Et |
| 3-211 | Br | SO₂Me | c-Pr |
| 3-212 | Br | SO₂Me | CH₂OMe |
| 3-213 | Br | SO₂Me | CH₂Cl |
| 3-214 | Br | SO₂Me | (1-Me)-c-Pr |
| 3-215 | Br | SO₂Me | (2-Me)-c-Pr |
| 3-216 | CH₂OMe | CF₃ | Me |
| 3-217 | CH₂OMe | CF₃ | Et |
| 3-218 | CH₂OMe | CF₃ | c-Pr |
| 3-219 | CH₂OMe | SO₂Me | Me |
| 3-220 | CH₂OMe | SO₂Me | Et |
| 3-221 | CH₂OMe | SO₂Me | c-Pr |
| 3-222 | Et | CF₃ | Me |
| 3-223 | Et | CF₃ | Et |
| 3-224 | Et | CF₃ | c-Pr |
| 3-225 | Et | CHF₂ | Me |
| 3-226 | Et | CHF₂ | Et |
| 3-227 | Et | CHF₂ | c-Pr |
| 3-228 | Et | SO₂Me | Me |
| 3-229 | Et | SO₂Me | Et |
| 3-230 | Et | SO₂Me | c-Pr |
| 3-231 | c-Pr | CF₃ | Me |
| 3-232 | c-Pr | CF₃ | Et |
| 3-233 | c-Pr | CF₃ | c-Pr |
| 3-234 | c-Pr | CF₃ | CH₂OMe |
| 3-235 | c-Pr | CF₃ | CH₂Cl |
| 3-236 | c-Pr | CF₃ | (1-Me)-c-Pr |
| 3-237 | c-Pr | CF₃ | (2-Me)-c-Pr |
| 3-238 | c-Pr | CHF₂ | Me |
| 3-239 | c-Pr | CHF₂ | Et |
| 3-240 | c-Pr | CHF₂ | c-Pr |
| 3-241 | c-Pr | CHF₂ | CH₂OMe |
| 3-242 | c-Pr | CHF₂ | CH₂Cl |
| 3-243 | c-Pr | CHF₂ | (1-Me)-c-Pr |
| 3-244 | c-Pr | CHF₂ | (2-Me)-c-Pr |
| 3-245 | c-Pr | SO₂Me | Me |
| 3-246 | c-Pr | SO₂Me | Et |
| 3-247 | c-Pr | SO₂Me | c-Pr |
| 3-248 | c-Pr | SO₂Me | CH₂OMe |
| 3-249 | c-Pr | SO₂Me | CH₂Cl |
| 3-250 | c-Pr | SO₂Me | (1-Me)-c-Pr |
| 3-251 | c-Pr | SO₂Me | (2-Me)-c-Pr |
| 3-252 | I | CF₃ | Me |
| 3-253 | I | CF₃ | Et |
| 3-254 | I | CF₃ | c-Pr |
| 3-255 | I | CF₃ | CH₂OMe |
| 3-256 | I | CF₃ | CH₂Cl |
| 3-257 | I | CF₃ | (1-Me)-c-Pr |
| 3-258 | I | CF₃ | (2-Me)-c-Pr |
| 3-259 | I | CHF₂ | Me |
| 3-260 | I | CHF₂ | Et |
| 3-261 | I | CHF₂ | c-Pr |
| 3-262 | I | CHF₂ | CH₂OMe |
| 3-263 | I | CHF₂ | CH₂Cl |
| 3-264 | I | CHF₂ | (1-Me)-c-Pr |
| 3-265 | I | CHF₂ | (2-Me)-c-Pr |
| 3-266 | I | SO₂Me | Me |
| 3-267 | I | SO₂Me | Et |
| 3-268 | I | SO₂Me | c-Pr |
| 3-269 | I | SO₂Me | CH₂OMe |
| 3-270 | I | SO₂Me | CH₂Cl |
| 3-271 | I | SO₂Me | (1-Me)-c-Pr |
| 3-272 | I | SO₂Me | (2-Me)-c-Pr |
| 3-273 | CF₃ | CF₃ | Me |
| 3-274 | CF₃ | CF₃ | Et |
| 3-275 | CF₃ | CF₃ | c-Pr |
| 3-276 | CF₃ | CF₃ | CH₂OMe |
| 3-277 | CF₃ | CF₃ | CH₂Cl |
| 3-278 | CF₃ | CF₃ | (1-Me)-c-Pr |
| 3-279 | CF₃ | CF₃ | (2-Me)-c-Pr |
| 3-280 | SMe | CF₃ | c-Bu |
| 3-281 | SMe | CF₃ | c-Hexyl |
| 3-282 | Cl | CF₃ | (3,5-F₂)-Ph |
| 3-283 | SMe | CF₃ | (3,5-F₂)-Ph |
| 3-284 | Cl | Br | Me |
| 3-285 | Cl | Br | Et |
| 3-286 | Cl | Br | c-Pr |
| 3-287 | Me | Cl | Me |
| 3-288 | Me | Cl | Et |
| 3-289 | Me | Cl | c-Pr |
| 3-290 | Cl | CF₃ | Vinyl |

Zu zahlreichen in obigen Tabellen genannten erfindungsgemäßen Verbindungen der Formel (I) werden nachfolgend NMR-Daten im sogenannten NMR-Peak-Listenverfahren offenbart. Dabei werden die ¹H-NMR-Daten ausgewählter Beispiele in Form von ¹H-NMR-Peaklisten notiert. Zu jedem Signalpeak wird erst der δ-Wert in ppm und dann die Signalintensität in runden Klammern aufgeführt. Die δ-Wert-Signalintensitäts- Zahlenpaare von verschiedenen Signalpeaks werden durch Semikolons voneinander getrennt aufgelistet. Die Peakliste eines Beispieles hat daher die Form:
δ₁ (Intensität₁⁾; δ₂ (Intensität₂); ........; δᵢ (Intensitätᵢ⁾ ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden. Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der erfindungsgemäßen Verbindungen und/oder Peaks von Verunreinigungen haben gewöhnlich im Durchschnitt eine geringere Intensität als die Peaks der erfindungsgemäßen Verbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der erfindungsgemäßen Verbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.
Beispiel-Nr. 1-1: ¹H-NMR (400 MHz, DMSO-d₆): δ = 11.48 (br s, 1H); 7.58 (d, 1H); 7.25 (d, 1H); 3.97 (s, 3H); 2.50 (s, 3H); 2.27 (s, 3H), 2.24 (s, 3H);
Beispiel-Nr. 1-3: ¹H-NMR (400 MHz, DMSO-d₆): δ = 11.49 (br s, 1H); 7.59 (d, 1H); 7.27 (d, 1H); 3.97 (s, 3H); 2.30 (s, 3H); 2.26 (s, 3H); 2.25 (m, 1H); 1.15 (m, 4H);
Beispiel-Nr. 1-7: ¹H-NMR (400 MHz, DMSO-d₆): δ = 11.81 (br s, 1H); 7.97 (d, 1H); 7.93 (d, 1H); 4.01 (s, 3H); 3.22 (s, 3H); 2.59 (s, 3H); 2.35 (s, 3H);
Beispiel-Nr. 1-9: ¹H-NMR (400 MHz, DMSO-d₆): δ = 11.84 (br s, 1H); 7.96 (d, 1H); 7.91 (d, 1H); 3.99 (s, 3H); 3.21 (s, 3H); 2.41 (s, 3H); 2.36 (m, 1H); 1.22 (m, 4H);
Beispiel-Nr. 1-14: ¹H-NMR (400 MHz, DMSO-d₆): δ = 11.79 (br s, 1H); 7.88 (d, 1H); 7.83 (d, 1H); 4.01 s, 3H); 2.57 (s, 3H); 2.34 (s, 3H);
Beispiel-Nr. 1-15: ¹H-NMR (400 MHz, DMSO-d₆): δ = 11.77 (br s, 1H); 7.89 (d, 1H); 7.83 (d, 1H); 4.01 (s, 3H); 2.85 (q, 2H); 2.29 (s, 3H); 1.12 (t, 3H);
Beispiel-Nr. 1-16: ¹H-NMR (400 MHz, DMSO-d₆): δ = 11.77 (br s, 1H); 7.89 (d, 1H); 7.83 (d, 1H); 4.01 (s, 3H); 2.82 (t, 2H); 2.30 (s, 3H); 1.66 (m, 2H); 0.96 (t, 3H);
Beispiel-Nr. 1-18: ¹H-NMR (400 MHz, DMSO-d₆): δ = 11.67 (br s, 1H); 7.88 (d, 1H); 7.83 (d, 1H); 4.36 (q, 2H); 2.38 (s, 3H); 2.35 (m, 1H); 1.47 (t, 3H); 1.23 (m, 4H);
Beispiel-Nr. 1-21: ¹H-NMR (400 MHz, DMSO-d₆): δ = 11.80 (br s, 1H); 7.92 (d, 1H); 7.84 (d, 1H); 4.45 (s, 2H); 4.01 (s, 3H); 3.36 (s, 3H); 2.33 (s, 3H);
Beispiel-Nr. 1-22: ¹H-NMR (400 MHz, DMSO-d₆): δ = 11.71 (br s, 1H); 7.96 (d, 1H); 7.89 (d, 1H); 4.97 (s, 2H); 4.36 (q, 2H); 2.33 (s, 3H); 1.47 (s, 3H);
Beispiel-Nr. 1-48: ¹H-NMR (400 MHz, DMSO-d₆): δ = 11.76 (br s, 1H); 7.94 (d, 1H); 7.72 (d, 1H); 4.01 (s, 3H); 3.84 (s, 1H); 2.54 (s, 3H);
Beispiel-Nr. 1-50: ¹H-NMR (400 MHz, DMSO-d₆): δ = 11.78 (br s, 1H); 7.94 (d, 1H); 7.74 (d, 1H); 4.01 (s, 3H); 3.84 (s, 3H); 2.35 (m, 1H); 1.16 (m, 4H);
Beispiel-Nr. 1-58: ¹H-NMR (400 MHz, CDCl₃): δ = 11.06 (br s, 1H); 7.84 (m, 2H); 4.16 (s, 3H); 2.67 (s, 3H); 2.40 (s, 3H);
Beispiel-Nr. 1-60: ¹H-NMR (400 MHz, DMSO-d₆): δ = 11.83 (br s, 1H); 8.03 (d, 1H); 7.95 (d, 1H); 4.05 (s, 3H); 2.37 (m, 1H); 2.37 (s, 3H); 1.24 (m, 4H);
Beispiel-Nr. 1-65: ¹H-NMR (400 MHz, DMSO-d₆): δ = 11.78 (br s, 1H); 7.85 (m, 2H); 7.09 (t, 1H); 4.05 (s, 3H); 2.61 (s, 3H); 2.34 (s, 3H);
Beispiel-Nr. 1-67: ¹H-NMR (400 MHz, DMSO-d₆): δ = 11.78 (br s, 1H); 7.86 (m, 2H); 6.97 (t, 1H); 4.05 (3H); 2.36 (s, 3H); 2.35 (m, 1H); 1.25 (m, 2H); 1.20 (m, 2H);
Beispiel-Nr. 1-114: ¹H-NMR (400 MHz, DMSO-d₆): δ = 11.90 (br s, 1H); 7.86 (d, 1H); 7.76 (d, 1H); 3.99 (s, 3H); 2.60 (s, 3H);
Beispiel-Nr. 1-115: ¹H-NMR (400 MHz, DMSO-d₆): δ = 11.90 (br s, 1H); 7.86 (d, 1H); 7.76 (d, 1H); 3.99 (s, 3H); 2.89 (q, 2H); 1.14 (t, 3H);
Beispiel-Nr. 1-116: ¹H-NMR (400 MHz, DMSO-d₆): δ = 11.91 (br s, 1H); 7.85 (d, 1H); 7.76 (d, 1H); 4.00 (s, 3H); 2.34 (m, 1H); 1.23 (m, 4H);
Beispiel-Nr. 1-121: ¹H-NMR (400 MHz, DMSO-d₆): δ = 11.79 (br s, 1H); 7.78 (d, 1H); 7.55 (d, 1H); 3.98 (s, 3H); 2.58 (s, 3H); 2.55 (s, 3H);
Beispiel-Nr. 1-123: ¹H-NMR (400 MHz, DMSO-d₆): δ = 11.77 (br s, 1H); 7.76 (d, 1H); 7.52 (d, 1H); 3.99 (s, 3H); 2.57 (s, 3H); 2.28 (m, 1H); 1.19 (m, 4H);
Beispiel-Nr. 1-127: ¹H-NMR (400 MHz, DMSO-d₆): δ = 12.05 (br s, 1H); 8.11 (m, 2H); 4.02 (s, 3H); 3.29 (s, 3H); 2.63 (s, 3H);
Beispiel-Nr. 1-129: ¹H-NMR (400 MHz, DMSO-d₆): δ = 12.04 (br s, 1H); 8.11 (m, 2H); 4.02 (s, 3H); 3.27 (s, 3H); 2.40 (m, 1H); 1.26 (m, 4H);
Beispiel-Nr. 1-134: ¹H-NMR (400 MHz, DMSO-d₆): δ = 11.76 (br s, 1H); 7.68 (d, 1H); 7.43 (d, 1H); 3.98 (s, 3H); 2.56 (s, 3H); 2.28 (s, 3H);
Beispiel-Nr. 1-135: ¹H-NMR (400 MHz, DMSO-d₆): δ = 11.78 (br s, 1H); 7.68 (d, 1H); 7.42 (d, 1H); 3.98 (s, 3H); 2.86 (q, 2H); 2.25 (s, 3H); 1.12 (t, 3H);
Beispiel-Nr. 1-136: ¹H-NMR (400 MHz, CDCl₃): δ = 10.11 (br s, 1H); 7.72 (d, 1H); 7.31 (d, 1H); 4.10 (s, 3H); 2.36 (s, 3H); 2.28 (m, 1H); 1.38 (m, 2H); 1.18 (m, 2H);
Beispiel-Nr. 1-137: ¹H-NMR (400 MHz, DMSO-d₆): δ = 12.00 (br s, 1H); 8.04 (m, 2H); 4.02 (s, 3H); 2.62 (s, 3H);
Beispiel-Nr. 1-138: ¹H-NMR (400 MHz, CDCl₃): δ = 11.20 (br s, 1H); 7.87 (d, 1H); 7.77 (d, 1H); 4.13 (s, 3H); 2.91 (q, 2H); 1.25 (t, 3H);
Beispiel-Nr. 1-139: ¹H-NMR (400 MHz, DMSO-d₆): δ = 12.03 (br s, 1H); 8.05 (m, 2H); 4.02 (s, 3H); 2.98 (t, 2H); 1.69 (m, 2H); 0.97 (t, 3H);
Beispiel-Nr. 1-140: ¹H-NMR (400 MHz, DMSO-d₆): δ = 12.02 (br s, 1H); 8.05 (m, 2H); 4.02 (s, 3H); 3.08 (m, 1H); 1.17 (d, 6H);
Beispiel-Nr. 1-141: ¹H-NMR (400 MHz, DMSO-d₆): δ = 12.02 (br s, 1H); 8.05 (m, 2H); 4.02 (s, 3H); 2.51 (s, 3H); 2.39 (m, 1H); 1.25 (m, 4H);
Beispiel-Nr. 1-143: ¹H-NMR (400 MHz, DMSO-d₆): δ = 12.07 (br s, 1H); 8.05 (s, 1H); 4.02 (s, 3H); 1.26 (s, 9H);
Beispiel-Nr. 1-144: ¹H-NMR (400 MHz, DMSO-d₆): δ = 12.06 (br s, 1H); 8.10 (d, 1H); 8.05 (d, 1H); 4.50 (s, 2H); 3.37 (s, 3H);
Beispiel-Nr. 1-145: ¹H-NMR (400 MHz, DMSO-d₆): δ = 12.04 (br s, 1H); 8.14 (d, 1H); 8.09 (d, 1H); 4.98 (s, 2H); 4.02 (s, 3H);
Beispiel-Nr. 1-146: ¹H-NMR (400 MHz, DMSO-d₆): δ = 12.09 (br s, 1H); 8.19 (d, 1H); 8.12 (d, 1H); 4.01 (s, 3H); 2.59 (s, 3H);
Beispiel-Nr. 1-147: ¹H-NMR (400 MHz, DMSO-d₆): δ = 12.05 (br s, 1H); 8.04 (s, 2H); 4.02 (s, 3H); 1.40 (d, 2H); 1.22 (s, 3H); 1.13 (d, 2H);
Beispiel-Nr. 1-148: ¹H-NMR (400 MHz, DMSO-d₆): δ = 12.00 (br s, 1H); 8.03 (m, 2H); 4.02 (m, 3H); 2.07 (m, 1H); 1.62 (m, 1H); 1.50 (m, 1H); 1.18 (m, 1H); 1.16 d, 3H);
Beispiel-Nr. 1-149: ¹H-NMR (400 MHz, DMSO-d₆): δ = 12.00 (br s, 1H); 8.01 (m, 2H); 4.02 (s, 3H); 2.22 (m, 1H); 1.38 (m, 2H); 1.29 (s, 3H); 1.22 (s, 3H);
Beispiel-Nr. 1-150: ¹H-NMR (400 MHz, DMSO-d₆): δ = 12.07 (br s, 1H); 8.04 (br s, 2H); 4.02 (s, 3H); 1.64 (m, 2H); 1.17 (s, 3H); 1.15 (s, 3H); 0.88 (m, 1H);
Beispiel-Nr. 1-153: ¹H-NMR (400 MHz, DMSO-d₆): δ = 12.00 (br s, 1H); 8.02 (m, 2H); 4.01 (s, 3H); 3.76 (m, 1H); 2.32 (m, 2H); 2.19 (m, 2H); 1.97 (m, 1H); 1.83 (m, 1H);
Beispiel-Nr. 1-154: ¹H-NMR (400 MHz, DMSO-d₆): δ = 12.01 (br s, 1H); 8.04 (m, 2H); 4.02 (s, 3H); 3.34 (m, 1H); 1.94 (m, 2H); 1.79 (m, 2H); 1.64 (m, 4H);
Beispiel-Nr. 1-155: ¹H-NMR (400 MHz, DMSO-d₆): δ = 12.01 (br s, 1H); 8.04 (m, 2H); 4.02 (s, 3H); 2.78 (m, 1H); 1.95 (m, 2H); 1.78 (m, 2H); 1.66 (m, 1H); 1.30 (m, 4H); 1.17 (m, 1H);
Beispiel-Nr. 1-162: ¹H-NMR (400 MHz, DMSO-d₆): δ = 12.13 (br s, 1H); 8.35 (d, 1H); 8.27 (d, 1H); 4.02 (s, 3H);
Beispiel-Nr. 1-163: ¹H-NMR (400 MHz, DMSO-d₆): δ = 12.11 (br s, 1H); 8.25 (d, 1H); 8.18 (d, 1H); 7.00 (t, 1H); 4.02 (s, 3H);
Beispiel-Nr. 1-164: ¹H-NMR (400 MHz, DMSO-d₆): δ = 11.95 (br s, 1H); 7.96 (d, 1H); 7.81 (d, 1H); 7.13 (t, 1H); 4.01 (s, 3H); 2.60 (s. 3H);
Beispiel-Nr. 1-165: ¹H-NMR (400 MHz, DMSO-d₆): δ = 11.97 (br s, 1H); 7.96 (d, 1H); 7.81 (d, 1H); 7.11 (t, 1H); 4.01 (s, 3H); 2.89 (q, 2H); 1.13 (t, 3H);
Beispiel-Nr. 1-168: ¹H-NMR (400 MHz, DMSO-d₆): δ = 11.95 (br s, 1H); 7.97 (d, 1H); 7.82 (d, 1H); 7.05 (t, 1H); 4.01 (s, 3H); 2.38 (m, 1H); 1.23 (m, 4H);
Beispiel-Nr. 1-192: ¹H-NMR (400 MHz, DMSO-d₆): δ = 11.86 (br s, 1H); 8.06 (d, 1H); 7.55 (d, 1H); 3.99 (s, 3H); 2.58 (s, 3H);
Beispiel-Nr. 1-194: ¹H-NMR (400 MHz, DMSO-d₆): δ = 11.87 (br s, 1H); 8.06 (d, 1H); 7.54 (d, 1H); 3.99 (s, 3H); 2.29 (m, 1H); 1.25 (m, 4H);
Beispiel-Nr. 1-195: ¹H-NMR (400 MHz, DMSO-d₆): δ = 12.00 (br s, 1H); 8.06 (d, 1H); 8.02 (d, 1H); 4.03 (s, 3H); 2.62 (s, 3H);
Beispiel-Nr. 1-197: ¹H-NMR (400 MHz, DMSO-d₆): δ = 12.00 (br s, 1H); 8.05 (d, 1H); 8.00 (d, 1H); 8.04 (s, 3H); 2.36 (m, 1H); 1.27 (m, 4H);
Beispiel-Nr. 1-202: ¹H-NMR (400 MHz, DMSO-d₆): δ = 11.94 (br s, 1H); 7.91 (d, 1H); 7.84 (d, 1H); 7.12 (t, 1H); 4.03 (s, 3H); 2.6 (s, 3H);
Beispiel-Nr. 1-203: ¹H-NMR (400 MHz, CDCl₃): δ = 10.77 (br s, 1H); 7.78 (d, 1H); 7.75 (d, 1H); 6.68 (t, 1H); 4.15 (s, 3H); 2.95 (q, 2H); 2.26 (t, 3H);
Beispiel-Nr. 1-204: ¹H-NMR (400 MHz, DMSO-d₆): δ = 11.96 (br s, 1H); 7.92 (d, 1H); 7.86 (d, 1H); 7.03 (t, 1H); 4.03 (s, 3H); 2.34 (m, 1H); 1.26 (m, 4H);
Beispiel-Nr. 1-231: ¹H-NMR (400 MHz, DMSO-d₆): δ = 11.72 (br s, 1H); 7.84 (m, 2H); 4.04 (s, 3H); 2.64 (s, 3H); 2.28 (m, 1H); 0.95 (m, 2H); 5.54 (m, 2H);
Beispiel-Nr. 1-233: ¹H-NMR (400 MHz, DMSO-d₆): δ = 11.73 (br s, 1H); 7.83 (m, 2H); 4.04 (s, 3H); 2.46 (m, 1H); 2.25 (m, 1H); 1.23 (m, 4H); 0.94 (m, 2H); 0.57 (m, 2H);
Beispiel-Nr. 1-280: ¹H-NMR (400 MHz, DMSO-d₆): δ = 11.91 (br s, 1H); 7.86 (d, 1H); 7.76 (d, 1H); 4.00 (s, 3H); 3.13 (m, 1H); 1.19 (d, 6H);
Beispiel-Nr. 1-281: ¹H-NMR (400 MHz, DMSO-d₆): δ = 11.90 (br s, 1H); 7.85 (d, 1H); 7.75 (d, 1H); 3.99 (s, 3H); 3.39 (q, 2H); 1.89 (m, 4H); 1.65 (m, 4H);
Beispiel-Nr. 1-282: ¹H-NMR (400 MHz, DMSO-d₆): δ = 11.94 (br s, 1H); 8.27 (d, 1H); 7.95 (d, 1H); 7.83 (d, 1H); 7.56 (m, 1H); 7.31 (m, 1H); 4.00 (s, 3H);
Beispiel-Nr. 1-283: ¹H-NMR (400 MHz, DMSO-d₆): δ = 11.95 (br s, 1H); 7.92 (d, 1H); 7.81 (d, 2H); 7.76 (d, 2H); 7.13 (d, 2H); 4.00 (s, 3H); 3.88 (s, 3H);
Beispiel-Nr. 1-284: ¹H-NMR (400 MHz, DMSO-d₆): δ = 11.84 (br s, 1H); 8.02 (d, 1H); 7.95 (d, 1H); 4.05 (s, 3H); 3.73 (m, 1H); 2.32 (s, 3H); 2.32 (m, 2H); 2.17 (m, 2H); 1.97 (m, 1H); 1.82 (m, 1H);
Beispiel-Nr. 1-285: ¹H-NMR (400 MHz, DMSO-d₆): δ = 11.85 (br s, 1H); 8.04 (d, 1H); 7.96 (d, 1H); 4.05 (s, 3H); 3.34 (m, 1H); 2.32 (s, 3H); 1.92 (m, 2H); 1.79 (m, 2H); 1.68 (m, 2H); 1.58 (m, 2H);
Beispiel-Nr. 1-286: ¹H-NMR (400 MHz, DMSO-d₆): δ = 11.85 (br s, 1H); 8.04 (d, 1H); 7.96 (d, 1H); 4.05 (s, 3H); 2.77 (m, 1H); 2.30 (s, 3H); 1.94 (m, 2H); 1.77 (m, 2H); 1.65 (m, 1H); 1.29 (m, 4H); 1.14 (m, 1H);
Beispiel-Nr. 1-287: ¹H-NMR (400 MHz, DMSO-d₆): δ = 12.01 (br s, 1H); 8.15 (m, 2H); 7.76 (m, 1H); 7.54 (m, 2H); 4.01 (s, 3H);
Beispiel-Nr. 1-289: ¹H-NMR (400 MHz, DMSO-d₆): δ = 11.89 (br s, 1H); 7.89 (d, 1H); 7.77 (d, 1H); 3.99 (s, 3H); 2.59 (s, 3H);
Beispiel-Nr. 1-291: ¹H-NMR (400 MHz, DMSO-d₆): δ = 11.89 (br s, 1H); 7.89 (d, 1H); 7.76 (d, 1H); 4.00 (s, 3H); 2.32 (m, 1H); 1.24 (m, 4H);
Beispiel-Nr. 1-292: ¹H-NMR (400 MHz, DMSO-d₆): δ = 11.67 (br s, 1H); 7.72 (d, 1H); 7.57 (d, 1H); 3.89 (s, 3H); 2.57 (s, 3H); 2.29 (s, 3H);
Beispiel-Nr. 1-293: ¹H-NMR (400 MHz, DMSO-d₆): δ = 11.64 (br s, 1H); 7.71 (d, 1H); 7.56 (d, 1H); 3.98 (s, 3H); 2.86 (q, 2H); 2.26 (s, 3H); 1.12 (t, 3H);
Beispiel-Nr. 1-294: ¹H-NMR (400 MHz, DMSO-d₆): δ = 11.65 (br s, 1H); 7.70 (d, 1H); 7.56 (d, 1H); 3.97 (s, 3H); 2.30 (s, 3H); 2.29 (m, 1H); 1.17 (m, 4H);
Beispiel-Nr. 2-1: ¹H-NMR (400 MHz, DMSO-d₆): δ = 11.38 (br s, 1H); 7.56 (d, 1H); 7.25 (d, 1H); 4.32 (q, 2H); 2.50 (s, 3H); 2.27 (s, 3H); 2.24 (s, 3H); 1.46 (t, 3H);
Beispiel-Nr. 2-7: ¹H-NMR (400 MHz, DMSO-d₆): δ = 11.70 (br s, 1H); 7.97 (d, 1H); 7.92 (d, 1H); 4.36 (q, 2H); 3.22 (s, 3H); 2.59 (s, 3H); 2.35 (s, 3H); 1.47 (t, 3H);
Beispiel-Nr. 2-9: ¹H-NMR (400 MHz, DMSO-d₆): δ = 11.72 (br s, 1H); 7.98 (d, 1H); 7.92 (d, 1H); 4.36 (q, 2H); 3.21 (s, 3H); 2.41 (s, 3H); 2.38 (m, 1H); 1.48 (t, 3H); 1.23 (m, 4H);
Beispiel-Nr. 2-14: ¹H-NMR (400 MHz, DMSO-d₆): δ = 11.67 (br s, 1H); 7.88 (d, 1H); 7.83 (d, 1H); 4.36 (q, 2H); 2.57 (s, 3H); 2.34 (s, 3H); 1.47 (t, 3H);
Beispiel-Nr. 2-15: ¹H-NMR (400 MHz, DMSO-d₆): δ = 11.67 (br s, 1H); 7.88 (d, 1H); 7.83 (d, 1H); 4.34 (q, 2H); 2.85 (q, 2H); 2.29 (s, 3H); 1.47 (t, 3H); 1.12 (t, 3H);
Beispiel-Nr. 2-16: ¹H-NMR (400 MHz, DMSO-d₆): δ = 11.67 (br s, 1H); 7.88 (d, 1H); 7.83 (d, 1H); 4.35 (q, 2H); 2.83 (t, 2H); 2.30 (s, 3H); 1.67 (m, 2H); 1.47 (t, 3H); 0.96 (t, 3H);
Beispiel-Nr. 2-18: ¹H-NMR (400 MHz, DMSO-d₆): δ = 11.68 (br s, 1H); 7.88 (d, 1H); 7.84 (d, 1H); 4.36 (q, 2H); 2.38 (s, 3H); 2.35 (m, 1H); 1.48 (t, 3H); 1.23 (m, 4H);
Beispiel-Nr. 2-22: ¹H-NMR (400 MHz, DMSO-d₆): δ = 11.83 (br s, 1H); 7.97 (d, 1H); 7.89 (d, 1H); 4.97 (s, 3H); 2.33 (s, 3H);
Beispiel-Nr. 2-48: ¹H-NMR (400 MHz, DMSO-d₆): δ = 11.65 (br s, 1H); 7.93 (d, 1H); 7.72 (d, 1H); 4.35 (q, 2H); 3.84 (s, 3H); 2.55 (s, 3H); 1.47 (t, 3H);
Beispiel-Nr. 2-50: ¹H-NMR (400 MHz, DMSO-d₆): δ = 11.63 (br s, 1H); 7.94 (d, 1H); 7.74 (d, 1H); 4.35 (q, 2H); 3.84 (s, 3H); 2.37 (m, 1H); 1.48 (t, 3H); 1.18 (m, 4H);
Beispiel-Nr. 2-58: ¹H-NMR (400 MHz, CDCl₃): δ = 10.85 (br s, 1H); 7.88 (d, 1H); 7.83 (d, 1H); 4.52 (q, 2H); 2.67 (s, 3H); 2.41 (s, 3H); 1.64 (t, 3H);
Beispiel-Nr. 2-60: ¹H-NMR (400 MHz, DMSO-d₆): δ = 11.74 (br s, 1H); 8.03 (d, 1H); 7.94 (d, 1H); 4.43 (q, 2H); 2.37 (s, 3H); 2.36 (m, 1H); 1.49 (t, 3H); 1.24 (m, 4H);
Beispiel-Nr. 2-65: ¹H-NMR (400 MHz, DMSO-d₆): δ = 11.68 (br s, 1H); 7.84 (m, 2H); 7.09 (t, 1H); 4.42 (q, 2H); 2.62 (s, 3H); 2.35 (s, 3H); 1.49 (t, 3H);
Beispiel-Nr. 2-67: ¹H-NMR (400 MHz, DMSO-d₆): δ = 11.68 (br s, 1H); 7.86 (s, 2H); 6.97 (t, 1H); 4.43 (q, 2H); 2.36 (s, 3H); 2.36 (m, 1H); 1.49 (t, 3H); 1.26 (m, 2H); 1.20 (m, 2H);
Beispiel-Nr. 2-114: ¹H-NMR (400 MHz, DMSO-d₆): δ = 11.80 (br s, 1H); 7.85 (d, 1H); 7.76 (d, 1H); 4.35 (q, 2H); 3.99; 2.60 (s, 3H); 1.46 (t, 3H);
Beispiel-Nr. 2-115: ¹H-NMR (400 MHz, DMSO-d₆): δ = 11.80 (br s, 1H); 7.86 (d, 1H); 7.76 (d, 1H); 4.35 (q, 2H); 2.89 (q, 2H); 1.46 (t, 3H); 1.14 (t, 3H);
Beispiel-Nr. 2-116: ¹H-NMR (400 MHz, DMSO-d₆): δ = 11.81 (br s, 1H); 7.85 (d, 1H); 7.76 (d, 1H); 4.36 (q, 2H); 2.34 (m, 1H); 1.47 (t, 3H); 1.23 (m, 4H);
Beispiel-Nr. 2-121: ¹H-NMR (400 MHz, DMSO-d₆): δ = 11.69 (br s, 1H); 7.76 (d, 1H); 7.55 (d, 1H); 4.34 (q, 2H); 2.58 (s, 3H); 2.55 (s, 3H); 1.46 (t, 3H);
Beispiel-Nr. 2-123: ¹H-NMR (400 MHz, DMSO-d₆): δ = 11.68 (br s, 1H); 7.75 (d, 1H); 7.52 (d, 1H); 4.35 (q, 2H); 2.57 (s, 3H); 2.28 (m, 1H); 1.46 (t, 3H); 1.20 (m, 4H);
Beispiel-Nr. 2-127: ¹H-NMR (400 MHz, DMSO-d₆): δ = 11.95 (br s, 1H); 8.12 (m, 2H); 4.35 (q, 2H); 3.29 (s, 3H); 2.64 (s, 3H); 1.47 (t, 3H);
Beispiel-Nr. 2-129: ¹H-NMR (400 MHz, DMSO-d₆): δ = 11.95 (br s, 1H); 8.11 (m, 2H); 4.38 (q, 2H); 3.27 (s, 3H); 2.40 (m, 1H); 1.48 (t, 3H); 1.26 (m, 4H);
Beispiel-Nr. 2-134: ¹H-NMR (400 MHz, DMSO-d₆): δ = 11.66 (br s, 1H); 7.67 (d, 1H); 7.43 (d, 1H); 4.35 (q, 2H); 2.56 (s, 3H); 2.28 (s, 3H); 1.46 (t, 3H);
Beispiel-Nr. 2-135: ¹H-NMR (400 MHz, DMSO-d₆): δ = 11.66 (br s, 1H); 7.67 (d, 1H); 7.43 (d, 1H); 4.34 (q, 2H); 2.86 (q, 2H); 2.25 (s, 3H); 1.46 (t, 3H); 1.12 (t, 3H);
Beispiel-Nr. 2-136: ¹H-NMR (400 MHz, DMSO-d₆): δ = 11.67 (br s, 1H); 7.68 (d, 1H); 7.44 (d, 1H); 4.35 (q, 2H); 2.32 (m, 1H); 2.31 (s, 3H); 1.46 (t, 3H); 1.18 (m, 4H);
Beispiel-Nr. 2-137: ¹H-NMR (400 MHz, DMSO-d₆): δ = 11.91 (br s, 1H); 8.05 (d, 1H); 8.02 (d, 1H); 4.37 (q, 2H); 2.62 (s, 3H); 1.47 (t, 3H);
Beispiel-Nr. 2-139: ¹H-NMR (400 MHz, DMSO-d₆): δ = 11.93 (br s, 1H); 8.03 (m, 2H); 4.36 (q, 2H); 2.89 (m, 2H); 1.68 (m, 2H); 1.47 (t, 3H); 0.97 (t, 3H);
Beispiel-Nr. 2-140: ¹H-NMR (400 MHz, DMSO-d₆): δ = 11.93 (br s, 1H); 8.03 (m, 2H); 4.37 (q, 2H); 3.07 (m, 1H); 1.47 (t, 3H); 1.17 (d, 6H);
Beispiel-Nr. 2-141: ¹H-NMR (400 MHz, DMSO-d₆): δ = 11.91 (br s, 1H); 8.03 (m, 2H); 4.38 (q, 2H); 2.39 (m, 1H); 1.47 (t, 3H); 1.25 (m, 4H);
Beispiel-Nr. 2-143: ¹H-NMR (400 MHz, DMSO-d₆): δ = 11.97 (br s, 1H); 8.05 (s, 2H); 4.37 (q, 2H); 1.47 (t, 3H); 1.25 (s, 9H);
Beispiel-Nr. 2-144: ¹H-NMR (400 MHz, DMSO-d₆): δ = 11.96 (br s, 1H); 8.09 (d, 1H); 8.04 (d, 1H); 4.50 (s, 2H); 4.37 (q, 2H); 3.37 (s, 3H); 1.47 (t, 3H);
Beispiel-Nr. 2-145: ¹H-NMR (400 MHz, CDCl₃): δ = 11.15 (br s, 1H); 7.95 (d, 1H); 7.81 (d, 1H); 4.64 (s, 2H); 4.51 (q, 2H); 1.63 (t, 3H);
Beispiel-Nr. 2-147: ¹H-NMR (400 MHz, DMSO-d₆): δ = 11.95 (br s, 1H); 8.04 (s, 2H); 4.38 (q, 2H); 1.48 (t, 3H); 1.40 (d, 2H); 1.22 (s, 3H); 1.13 (d, 2H);
Beispiel-Nr. 2-148: ¹H-NMR (400 MHz, DMSO-d₆): δ = 11.90 (br s, 1H); 8.02 (m, 2H); 4.38 (q, 2H); 2.14 (m, 1H); 1.64 (m, 1H); 1.49 (m, 1H); 1.48 (t, 3H); 1.16 (m, 1H); 1.16 (d, 3H);
Beispiel-Nr. 2-149: ¹H-NMR (400 MHz, DMSO-d₆): δ = 11.91 (br s, 1H); 8.01 (m, 2H); 4.37 (q, 2H); 2.22 (m, 1H); 1.48 (t, 3H); 1.35 (m, 2H); 1.29 (s, 3H); 1.22 (s, 3H);
Beispiel-Nr. 2-150: ¹H-NMR (400 MHz, DMSO-d₆): δ = 11.99 (br s, 1H); 8.03 (br s, 2H); 4.38 (q, 2H); 1.63 (m, 2H); 1.48 (t, 3H); 1.17 (s, 3H); 1.15 (s, 3H); 0.88 (m, 1H);
Beispiel-Nr. 2-153: ¹H-NMR (400 MHz, DMSO-d₆): δ = 11.91 (br s, 1H); 8.02 (m, 2H); 4.37 (q, 2H); 3.75 (m, 1H); 2.31 (m, 2H); 2.20 (m, 2H); 1.99 (m, 1H); 1.83 (m, 1H); 1.47 (t, 3H);
Beispiel-Nr. 2-154: ¹H-NMR (400 MHz, DMSO-d₆): δ = 11.92 (br s, 1H); 8.04 (m, 2H); 4.37 (q, 2H); 3.34 (m, 1H); 1.94 (m, 2H); 1.79 (m, 2H); 1.63 (m, 4H); 1.48 (t, 3H);
Beispiel-Nr. 2-155: ¹H-NMR (400 MHz, DMSO-d₆): δ = 11.93 (br s, 1H); 8.04 (d, 1H); 8.01 (d, 1H); 4.36 (q, 2H); 2.77 (m, 1H); 1.95 (m, 2H); 1.78 (m, 2H); 1.65 (m, 1H); 1.47 (t, 3H); 1.30 (m, 4H); 1.15 (m, 1H);
Beispiel-Nr. 2-162: ¹H-NMR (400 MHz, DMSO-d₆): δ = 12.03 (br s, 1H); 8.34 (d, 1H); 8.27 (d, 1H); 4.38 (q, 2H); 1.48 (t, 3H);
Beispiel-Nr. 2-163: ¹H-NMR (400 MHz, DMSO-d₆): δ = 12.00 (br s, 1H); 8.24 (d, 1H); 8.17 (d, 1H); 7.00 (t, 1H); 4.37 (q, 2H); 1.48 (t, 3H);
Beispiel-Nr. 2-164: ¹H-NMR (400 MHz, DMSO-d₆): δ = 11.85 (br s, 1H); 7.96 (d, 1H); 7.81 (d, 1H); 7.14 (t, 1H); 4.37 (q, 2H); 2.60 (3H); 1.47 (t, 3H);
Beispiel-Nr. 2-165: ¹H-NMR (400 MHz, DMSO-d₆): δ = 11.88 (br s, 1H); 7.96 (d, 1H); 7.81 (d, 1H); 7.11 (t, 1H); 4.37 (q, 2H); 2.90 (q, 2H); 1.47 (t, 3H); 1.13 (t, 1H);
Beispiel-Nr. 2-168: ¹H-NMR (400 MHz, DMSO-d₆): δ = 11.86 (br s, 1H); 7.97 (d, 1H); 7.82 (d, 1H); 7.05 (t, 1H); 4.37 (q, 2H); 2.38 (m, 1H); 1.47 (t, 3H); 1.24 (m, 4H);
Beispiel-Nr. 2-194: ¹H-NMR (400 MHz, DMSO-d₆): δ = 11.78 (br s, 1H); 8.06 (d, 1H); 7.53 (d, 1H); 4.35 (q, 2H); 2.29 (m, 1H); 1.46 (t, 3H); 1.25 (m, 4H);
Beispiel-Nr. 2-195: ¹H-NMR (400 MHz, DMSO-d₆): δ = 11.89 (br s, 1H); 8.05 (d, 1H); 7.99 (d, 1); 4.39 (q, 2H); 2.62 (s, 3H); 1.48 (t, 3H);
Beispiel-Nr. 2-197: ¹H-NMR (400 MHz, DMSO-d₆): δ = 11.91 (br s, 1H); 8.07 (d, 1H); 8.00 (d, 1H); 4.41 (q, 2H); 2.38 (m, 1H); 1.50 (t, 3H); 1.29 (m, 4H);
Beispiel-Nr. 2-202: ¹H-NMR (400 MHz, DMSO-d₆): δ = 11.83 (br s, 1H); 7.91 (d, 1H); 7.84 (d, 1H); 7.12 (t, 1H); 4.39 (q, 2H); 2.60 (s, 3H); 1.48 (t, 3H);
Beispiel-Nr. 2-204: ¹H-NMR (400 MHz, DMSO-d₆): δ = 11.86 (br s, 1H); 7.91 (d, 1H); 7.86 (d, 1H); 7.03 (t, 1H); 4.39 (q, 2H); 2.37 (m, 1H); 1.48 (t, 3H); 1.25 (m, 4H);
Beispiel-Nr. 2-231: ¹H-NMR (400 MHz, DMSO-d₆): δ = 11.60 (br s, 1H); 7.84 (m, 2H); 4.38 (q, 2H); 2.64 (s, 3H); 2.28 (m, 1H); 1.50 (t, 3H); 0.94 (m, 2H); 0.55 (m, 2H);
Beispiel-Nr. 2-233: ¹H-NMR (400 MHz, DMSO-d₆): δ = 11.60 (br s, 1H); 7.84 (m, 2H); 4.38 (q, 2H); 2.44 (m, 1H); 2.23 (m, 1H); 1.50 (t, 3H); 1.22 (m, 4H); 0.94 (m, 2H); 0.57 (m, 2H);
Beispiel-Nr. 2-280: ¹H-NMR (400 MHz, DMSO-d₆): δ = 11.81 (br s, 1H); 7.86 (d, 1H); 7.76 (d, 1H); 4.35 (q, 2H); 3.13 (m, 1H); 1.47 (t, 3H); 1.19 (d, 6H);
Beispiel-Nr. 2-281: ¹H-NMR (400 MHz, DMSO-d₆): δ = 11.80 (br s, 1H); 7.85 (d, 1H); 7.75 (d, 1H); 4.35 (q, 2H); 3.39 (m, 1H); 1.88 (m, 4H); 1.64 (m, 4H); 1.46 (t, 3H);
Beispiel-Nr. 2-282: ¹H-NMR (400 MHz, DMSO-d₆): δ = 11.85 (br s, 1H); 8.27 (d, 1H); 7.95 (d, 1H); 7.83 (d, 1H); 7.57 (m, 1H); 7.31 (m, 1H); 4.36 (q, 2H); 1.46 (t, 3H);
Beispiel-Nr. 2-283: ¹H-NMR (400 MHz, DMSO-d₆): δ = 11.84 (br s, 1H); 7.92 (d, 1H); 7.81 (d, 1H); 7.76 (d, 2H); 7.13 (d, 2H); 4.35 (q, 2H); 3.88 (s, 3H); 1.46 (t, 3H);
Beispiel-Nr. 2-284: ¹H-NMR (400 MHz, DMSO-d₆): δ = 11.73 ( br s, 1H); 8.02 (d, 1H); 7.93 (d, 1H); 4.42 (q, 2H); 3.73 (m, 1H); 2.33 (s, 3H); 2.32 (m, 2H); 2.18 (m, 2H); 1.97 (m, 1H); 1.82 (m, 1H); 1.48 (t, 3H);
Beispiel-Nr. 2-285: ¹H-NMR (400 MHz, DMSO-d₆): δ = 11.77 (br s, 1H); 8.04 (d, 1H); 7.95 (d, 1H); 4.44 (q, 2H); 2.78 (m, 1H); 2.32 (s, 3H); 1.95 (m, 2H); 1.79 (m, 2H); 1.65 (m, 1H); 1.50 (t, 3H); 1.28 (m, 4H); 1.16 (m, 1H);
Beispiel-Nr. 2-286: ¹H-NMR (400 MHz, DMSO-d₆): δ = 11.93 (br s, 1H); 8.15 (m, 2H); 7.76 (m, 1H); 7.54 (m, 2H); 4.36 (q, 2H); 1.46 (t, 3H);
Beispiel-Nr. 2-288: ¹H-NMR (400 MHz, DMSO-d₆): δ = 11.79 (br s, 1H); 7.89 (d, 1H); 7.76 (d, 1H); 4.35 (q, 2H); 2.59 (s, 3H); 1.46 (t, 3H);
Beispiel-Nr. 2-290: ¹H-NMR (400 MHz, DMSO-d₆): δ = 11.80 (br s, 1H); 7.89 (d, 1H); 7.75 (d, 1H); 4.35 (q, 2H); 2.32 (m, 1H); 1.46 (t, 3H); 1.24 (m, 4H);
Beispiel-Nr. 2-291: ¹H-NMR (400 MHz, DMSO-d₆): δ = 11.57 (br s, 1H); 7.71 (d, 1H); 7.57 (d, 1H); 4.33 (q, 2H); 2.57 (s, 3H); 2.29 (s, 3H); 1.46 (t, 3H);
Beispiel-Nr. 2-292: ¹H-NMR (400 MHz, DMSO-d₆): δ = 11.53 (br s, 1H); 7.71 (d, 1H); 7.56 (d, 1H); 4.33 (q, 2H); 2.86 (q, 2H); 2.26 (s, 3H); 1.46 (t, 3H); 1.12 (t, 3H);
Beispiel-Nr. 2-293: ¹H-NMR (400 MHz, DMSO-d₆): δ = 11.54 (br s, 1H); 7.71 (d, 1H); 7.57 (d, 1H); 4.33 (q, 2H); 2.31 (s, 3H); 2.31 (m, 1H); 1.46 (t, 3H); 1.19 (m, 4H);
Beispiel-Nr. 2-294: ¹H-NMR (400 MHz, DMSO-d₆): δ = 11.92 (br s, 1H); 8.07 (m, 2H); 6.74 (dd, 1H); 6.45 (d, 1H); 6.00 (d, 1H); 4.37 (q, 2H); 1.47 (t, 3H);
Beispiel-Nr. 3-1: ¹H-NMR (400 MHz, DMSO-d₆): δ = 11.36 (br s, 1H); 7.55 (d, 1H); 7.26 (d, 1H); 4.27 (t, 2H); 2.51 (s, 3H); 2.26 (s, 3H); 2.24 (s, 3H); 1.88 (m, 2H); 0.87 (t, 3H);
Beispiel-Nr. 3-3: ¹H-NMR (400 MHz, DMSO-d₆): δ = 11.37 (br s, 1H); 7.56 (d, 1H); 7.28 (d, 1H); 4.28 (t, 2H); 2.30 (s, 3H); 2.28 (m, 1H); 2.27 (s, 3H); 1.16 (m, 4H); 0.87 (t, 3H);
Beispiel-Nr. 3-7: ¹H-NMR (400 MHz, DMSO-d₆): δ = 11.68 (br s, 1H); 7.97 (d, 1H); 7.91 (d, 1H); 4.30 (t, 2H); 3.22 (s, 3H); 2.59 (s, 3H); 2.35 (s, 3H); 1.89 (m, 2H); 0.89 (t, 3H);
Beispiel-Nr. 3-9: ¹H-NMR (400 MHz, DMSO-d₆): δ = 11.70 (br s, 1H); 7.98 (d, 1H); 7.91 (d, 1H); 4.31 (t, 2H); 3.21 (s, 3H); 2.41 (s, 3H); 2.36 (m, 1H); 1.89 (m, 2H); 1.23 (m, 4H); 0.89 (t, 3H);
Beispiel-Nr. 3-14: ¹H-NMR (400 MHz, DMSO-d₆): δ = 11.64 (br s, 1H); 7.86 (d, 1H); 7.83 (d, 1H); 4.30 (t, 2H); 2.57 (s, 3H); 2.33 (s, 3H); 1.89 (m, 2H); 0.89 (t, 3H);
Beispiel-Nr. 3-15: ¹H-NMR (400 MHz, DMSO-d₆): δ = 11.64 (br s, 1H); 7.87 (d, 1H); 7.83 (d, 1H); 4.30 (t, 2H); 2.85 (q, 2H); 2.29 (s, 3H); 1.89 (m, 2H); 1.12 (t, 3H); 0.89 (s, 3H);
Beispiel-Nr. 3-16: ¹H-NMR (400 MHz, DMSO-d₆): δ = 11.65 (br s, 1H); 7.86 (d, 1H); 7.83 (d, 1H); 4.30 (t, 2H); 2.83 (t, 2H); 2.30 (s, 3H); 1.89 (m, 2H); 1.66 (m, 2H); 0.96 (t, 3H); 0.89 (t, 3H);
Beispiel-Nr. 3-18: ¹H-NMR (400 MHz, CDCl₃): δ = 11.00 (br s, 1H); 7.85 (d, 1H); 7.68 (d, 1H); 4.39 (q, 2H); 2.49 (s, 3H); 2.25 (m, 1H); 2.01 (m, 2H); 1.42 (m, 2H); 1.21(m, 2H); 0.98 (t, 3H);
Beispiel-Nr. 3-48: ¹H-NMR (400 MHz, DMSO-d₆): δ = 11.66 (br s, 1H); 7.92 (d, 1H); 7.72 (d, 1H); 4.30 (t, 2H); 3.84 (s, 3H); 2.55 (s, 3H); 1.89 (m, 2H); 0.89 (t, 3H);
Beispiel-Nr. 3-50: ¹H-NMR (400 MHz, DMSO-d₆): δ = 11.60 (br s, 1H); 7.93 (d, 1H); 7.74 (d, 1H); 4.30 (t, 2H); 3.84 (s, 3H); 2.37 (m, 1H); 1.89 (m, 2H); 1.18 (m, 4H); 0.89 (t, 3H);
Beispiel-Nr. 3-58: ¹H-NMR (400 MHz, CDCl₃): δ = 10.83 (br s, 1H); 7.86 (d, 1H); 7.82 (d, 1H); 4.45 (t, 2H); 2.67 (s, 3H); 2.40 (s, 3H); 2.05 (m, 2H); 1.00 (t, 3H);
Beispiel-Nr. 3-65: ¹H-NMR (400 MHz, DMSO-d₆): δ = 11.68 (br s, 1H); 7.84 (s, 2H); 7.09 (t, 1H); 4.37 (t, 2H); 2.62 (3H); 2.35 (s, 3H); 1.91 (2H); 0.90 (t, 3H);
Beispiel-Nr. 3-67: ¹H-NMR (400 MHz, DMSO-d₆): δ = 11.68 (br s, 1H); 7.85 (s, 2H); 6.97 (t, 1H); 4.37 (t, 2H); 2.38 (m, 1H); 2.36 (s, 3H); 1.91 (m, 2H); 1.23 (m, 4H); 0.90 (t, 3H);
Beispiel-Nr. 3-123: ¹H-NMR (400 MHz, DMSO-d₆): δ = 11.66 (br. s, 1H); 7.74 (d, 1H); 7.52 (d, 1H); 4.30 (t, 2H); 2.57 (s, 3H); 2.28 (m, 1H); 1.88 (m, 2H); 1.19 (m, 4H); 0.87 (t, 3H);
Beispiel-Nr. 3-129: ¹H-NMR (400 MHz, DMSO-d₆): δ = 11.93 (br s, 1H); 8.12 (d, 1H); 8.08 (d, 1H); 4.32 (t, 2H); 3.27 (s, 3H); 2.40 (m, 1H); 1.89 (m, 2H); 1.26 (m, 4H); 0.88 (t, 3H);
Beispiel-Nr. 3-134: ¹H-NMR (400 MHz, DMSO-d₆): δ = 11.65 (br s, 1H); 7.66 (d, 1H); 7.43 (d, 1H); 4.30 (t, 2H); 2.56 (s, 3H); 2.28 (s, 3H); 1.88 (m, 2H); 0.87 (t, 3H);
Beispiel-Nr. 3-135: ¹H-NMR (400 MHz, DMSO-d₆): δ = 11.65 (br s, 1H); 7.66 (d, 1H); 7.43 (d, 1H); 4.29 (t, 2H); 2.86 (q, 2H); 2.25 (s, 3H); 1.88 (m, 2H); 1.12 (t, 3H); 0.87 (t, 3H);
Beispiel-Nr. 3-136: ¹H-NMR (400 MHz, DMSO-d₆): δ = 11.65 (br s, 1H); 7.66 (d, 1H); 7.44 (d, 1H); 4.30 (t, 2H); 2.32 (m, 1H); 2.30 (s, 3H); 1.88 (m, 2H); 1.19 (m, 4H); 0.87 (t, 3H);
Beispiel-Nr. 3-137: ¹H-NMR (400 MHz, DMSO-d₆): δ = 11.89 (br s, 1H); 8.02 (m, 2H); 4.32 (t, 2H); 2.62 (s, 3H); 1.89 (m, 2H); 0.88 (t, 3H);
Beispiel-Nr. 3-140: ¹H-NMR (400 MHz, DMSO-d₆): δ = 11.91 (br s, 1H); 8.04 (s, 2H); 4.32 (t, 2H); 3.07 (m, 1H); 1.89 (m, 2H); 1.17 (d, 6H); 0.88 (t, 3H);
Beispiel-Nr. 3-141: ¹H-NMR (400 MHz, DMSO-d₆): δ = 11.89 (br s, 1H); 8.03 (m, 2H); 4.32 (t, 2H); 2.40 (m, 1H); 1.89 (m, 2H); 1.25 (m, 4H); 0.88 (t, 3H);
Beispiel-Nr. 3-144: ¹H-NMR (400 MHz, DMSO-d₆): δ = 11.94 (br s, 1H); 8.05 (m, 2H); 4.50 (s, 2H); 4.32 (t, 2H); 3.37 (s, 3H); 1.88 (m, 2H); 0.88 (t, 3H);
Beispiel-Nr. 3-150: ¹H-NMR (400 MHz, DMSO-d₆): δ = 11.93 (br s, 1H); 8.02 (br s, 2H); 4.33 (t, 2H); 1.91.89 (m, 2H); 1.63 (m, 2H); 1.17 (s, 3H); 1.15 (s, 3H); 0.88 (t, 3H); 0.87 (m, 1H);
Beispiel-Nr. 3-153: ¹H-NMR (400 MHz, DMSO-d₆): δ = 11.91 (br s, 1H); 8.03 (m, 2H); 4.33 (t, 2H); 3.77 (m, 1H); 2.34 (m, 2H); 2.21 (m, 2H); 2.01 (m, 1H); 1.88 (m, 3H); 0.90 (t, 3H);
Beispiel-Nr. 3-155: ¹H-NMR (400 MHz, DMSO-d₆): δ = 11.90 (br s, 1H); 8.03 (br s, 2H); 4.32 (t, 2H); 2.77 (m, 1H); 1.94 (m, 4H); 1.99 (m, 2H); 1.78 (m, 2H); 1.65 (m, 1H); 1.29 (m, 2H); 1.15 (m, 1H); 0.88 (t, 3H);
Beispiel-Nr. 3-163: ¹H-NMR (400 MHz, DMSO-d₆): δ = 11.98 (br s, 1H); 8.23 (d, 1H); 8.17 (d, 1H); 7.01 (t, 1H); 4.32 (t, 2H); 1.89 (m, 2H); 0.89 (t, 3H);
Beispiel-Nr. 3-164: ¹H-NMR (400 MHz, DMSO-d₆): δ = 11.83 (br s, 1H); 7.95 (d, 1H); 7.81 (d, 1H); 7.14 (t, 1H); 4.32 (t, 2H); 2.60 (s, 3H); 1.89 (m, 2H); 0.88 (t, 3H);
Beispiel-Nr. 3-165: ¹H-NMR (400 MHz, DMSO-d₆): δ = 11.86 (br s, 1H); 7.95 (d, 1H); 7.81 (d, 1H); 7.11 (t, 1H); 4.31 (t, 2H); 2.90 (q, 2H); 1.89 (m, 2H); 1.13 (t, 3H); 0.88 (t, 3H);
Beispiel-Nr. 3-168: ¹H-NMR (400 MHz, DMSO-d₆): δ = 11.84 (br s, 1H); 7.95 (d, 1H); 7.83 (d, 1H); 7.05 (t, 1H); 4.32 (t, 2H); 2.38 (m, 1H); 1.89 (m, 2H); 1.24 (m, 4H); 0.88 (t, 3H);
Beispiel-Nr. 3-192: ¹H-NMR (400 MHz, DMSO-d₆): δ = 11.75 (br s, 1H); 8.06 (d, 1H); 7.53 (d, 1H); 4.29 (t, 2H); 2.58 (s, 3H); 1.87 (m, 2H); 0.87 (t, 3H);
Beispiel-Nr. 3-195: ¹H-NMR (400 MHz, DMSO-d₆): δ = 11.87 (br s, 1H); 8.05 (d, 1H); 7.98 (d, 1H); 4.34 (t, 2H); 2.62 (s, 3H); 1.90 (m, 2H); 0.89 (t, 3H);
Beispiel-Nr. 3-197: ¹H-NMR (400 MHz, DMSO-d₆): δ = 11.89 (br s, 1H); 8.07 (d, 1H); 7.99 (d, 1H); 4.35 (t, 2H); 2.38 (m, 1H); 1.93 (m, 2H); 1.29 (m, 4H); 0.91 (t, 3H);
Beispiel-Nr. 3-202: ¹H-NMR (400 MHz, DMSO-d₆): δ = 11.82 (br s, 1H); 7.89 (d, 1H); 7.84 (d, 1H); 4.33 (t, 2H); 2.60 (s, 3H); 1.90 (m, 2H); 0.89 (t, 3H);
Beispiel-Nr. 3-204: ¹H-NMR (400 MHz, DMSO-d₆): δ = 11.81 (br s, 1H); 7.89 (d, 1H); 7.85 (d, 1H); 7.02 (t, 1H); 4.33 (t, 2H); 2.36 (m, 1H); 1.89 (m, 2H); 1.26 (m, 4H); 0.89 (t, 3H);
Beispiel-Nr. 3-231: ¹H-NMR (400 MHz, DMSO-d₆): δ = 11.58 (br s, 1H); 7.85 (d, 1H); 7.80 (d, 1H); 4.33 (t, 2H); 2.64 (s, 3H); 2.27 (m, 1H); 1.91 (m, 2H); 0.94 (m, 2H); 0.90 (t, 3H); 0.55 (m, 2H);
Beispiel-Nr. 3-280: ¹H-NMR (400 MHz, DMSO-d₆): δ = 11.73 (br s, 1H); 8.02 (d, 1H); 7.92 (d, 1H); 4.36 (t, 2H); 3.74 (m, 1H); 2.33 (s, 3H); 2.33 (m, 2H); 2.18 (m, 2H); 1.94 (m, 3H); 1.83 (m, 1H); 0.89 (t, 3H);
Beispiel-Nr. 3-281: ¹H-NMR (400 MHz, DMSO-d₆): δ = 11.74 (br s, 1H); 8.04 (d, 1H); 7.94 (d, 1H); 4.37 (t, 2H); 2.77 (m, 1H); 2.32 (s, 3H); 1.94 (m, 2H); 1.92 (m, 2H); 1.77 (m, 2H); 1.65 (m, 1H); 1.26 (m, 4H); 1.14 (m, 1H); 0.90 (s, 3H);
Beispiel-Nr. 3-282: ¹H-NMR (400 MHz, DMSO-d₆): δ = 11.91 (br s, 1H); 8.15 (m, 2H); 7.77 (m, 1H); 7.54 (m, 2H); 4.31 (t, 2H); 1.88 (m, 2H); 0.87 (t, 3H).
Beispiel-Nr. 3-284: ¹H-NMR (400 MHz, DMSO-d₆): δ = 11.78 (br s, 1H); 7.89 (d, 1H); 7.75 (d, 1H); 4.30 (t, 2H); 2.59 (s, 3H); 1.88 (m, 2H); 0.87 (t, 3H);
Beispiel-Nr. 3-286: ¹H-NMR (400 MHz, DMSO-d₆): δ = 11.78 (br s, 1H); 7.89 (d, 1H); 7.74 (d, 1H); 4.30 (t, 2H); 2.32 (m, 1H); 1.88 (m, 2H); 1.24 (m, 4H); 0.87 (t, 3H);
Beispiel-Nr. 3-287: ¹H-NMR (400 MHz, DMSO-d₆): δ = 11.54 (br s, 1H); 7.70 (d, 1H); 7.57 (d, 1H); 4.28 (t, 2H); 2.57 (s, 3H); 2.29 (s, 3H); 1.87 (m, 2H); 0.88 (t, 3H);
Beispiel-Nr. 3-288: ¹H-NMR (400 MHz, DMSO-d₆): δ = 11.51 (br s, 1H); 7.69 (d, 1H); 7.56 (d, 1H); 4.28 (t, 2H); 2.86 (q, 2H); 2.26 (s, 3H); 1.88 (m, 2H); 1.12 (t, 3H); 0.88 (t, 3H);
Beispiel-Nr. 3-289: ¹H-NMR (400 MHz, DMSO-d₆): δ = 11.52 (br s, 1H); 7.69 (d, 1H); 7.58 (d, 1H); 4.29 (t, 2H); 2.31 (s, 3H); 2.31 (m, 1H); 1.88 (m, 2H); 2.20 (m, 4H); 0.88 (t, 3H);

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I),
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I),
   5 Gew.-Teile 2,2'-dinaphthylmethan-6,6'-disulfonsaures Natrium,
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

Die hier verwendeten Abkürzungen bedeuten:

| | | | |
|---|---|---|---|
| ABUTH | Abutilon theophrasti | ALOMY | Alopecurus myosuroides |
| AMARE | Amaranthus retroflexus | AVEFA | Avena fatua |
| CYPES | Cyperus serotinus | DIGSA | Digitaria sanguinalis |
| ECHCG | Echinocloa crus galli | HORMU | Hordeum murinum |
| LOLMU | Lolium multiflorum | LOLRI | Lolium rigidum Gaudin |
| MATIN | Matricaria inodora | PHBPU | Pharbitis purpureum |
| POLCO | Polygonum convolvulus | SETVI | Setaria viridis |
| STEME | Stellaria media | VERPE | Veronica persica |
| VIOTR | Viola tricolor | | |
| D1 | WO 2012/028579 A1 | | |

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigten zahlreiche erfindungsgemäße Verbindungen bei einer Aufwandmenge von 320 g oder weniger pro Hektar eine mindestens 80%-ige Wirkung gegen eine Vielzahl bedeutender Schadpflanzen.
Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen. Die Daten nachfolgender Tabellen B1 bis B17 zeigen beispielhaft die herbizide Wirkung der erfindungsgemäßen Verbindungen im Vorauflauf, wobei die herbizide Wirkung in Prozent angegeben ist.

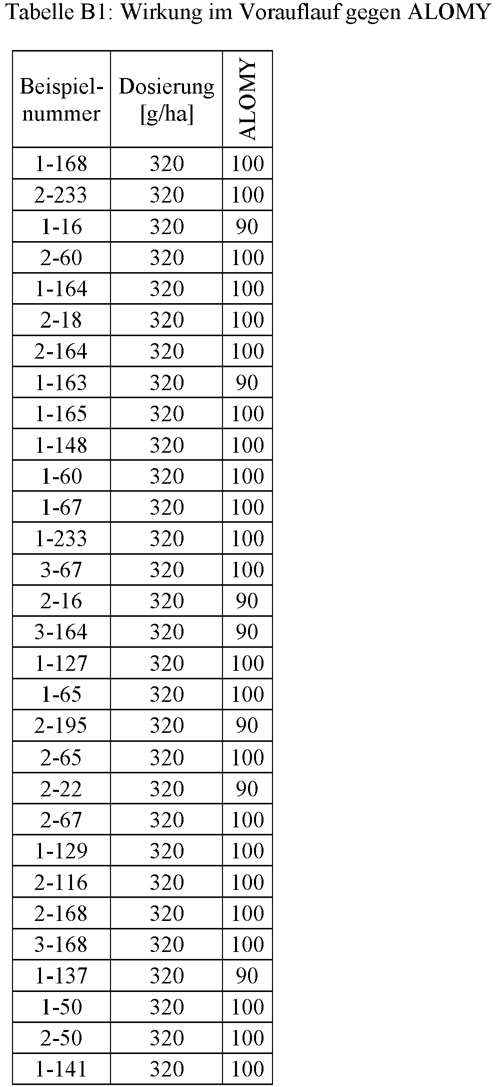

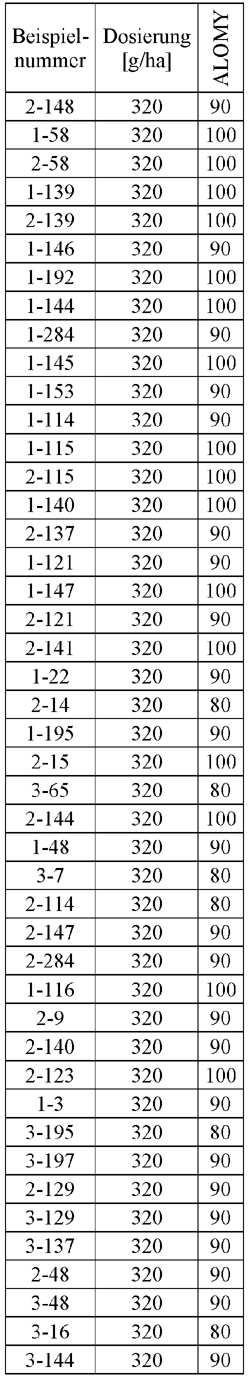

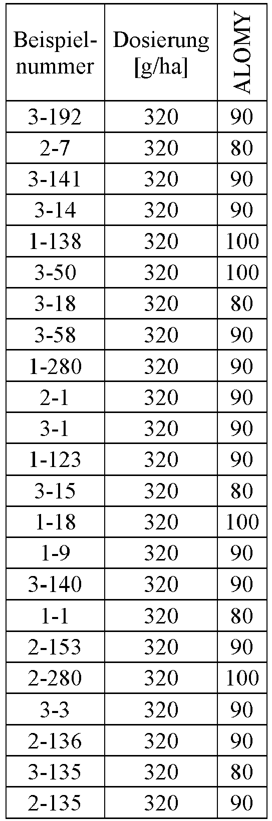

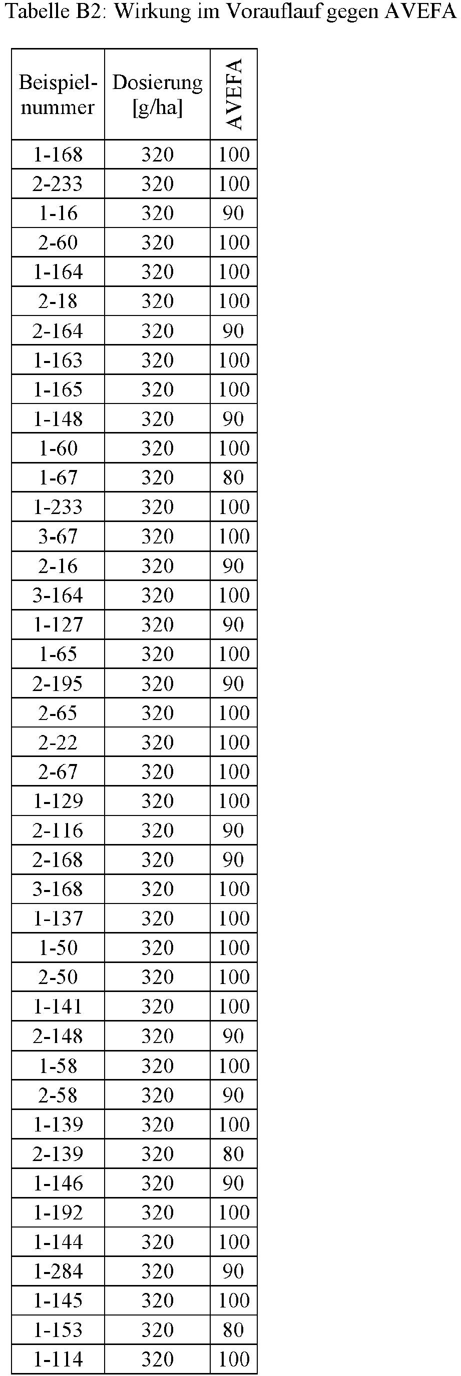

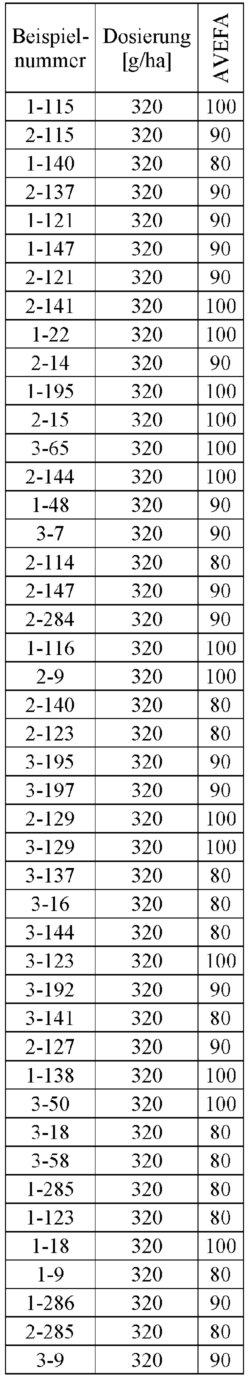

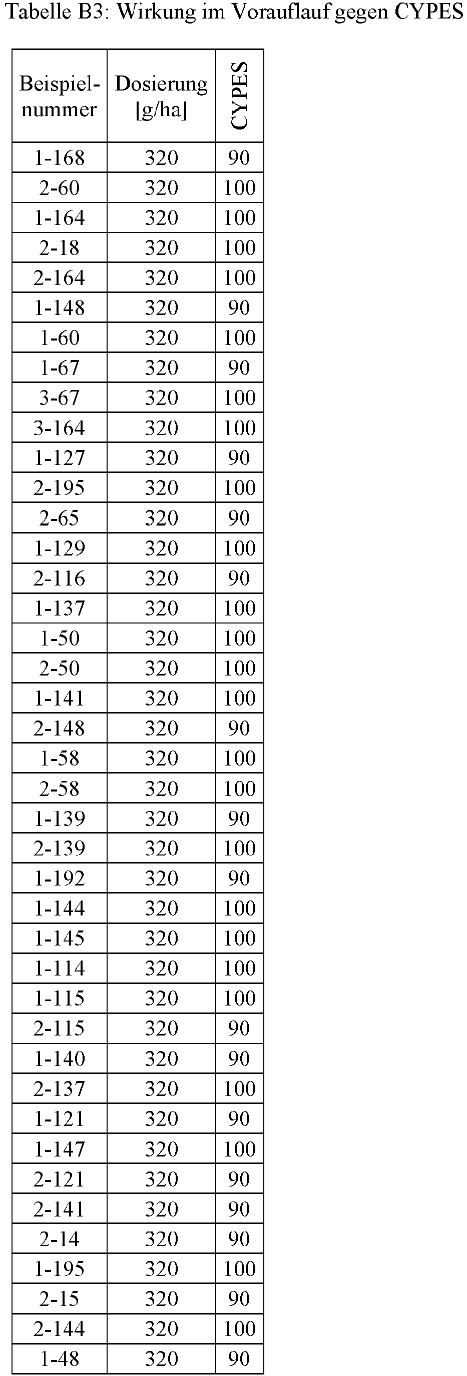

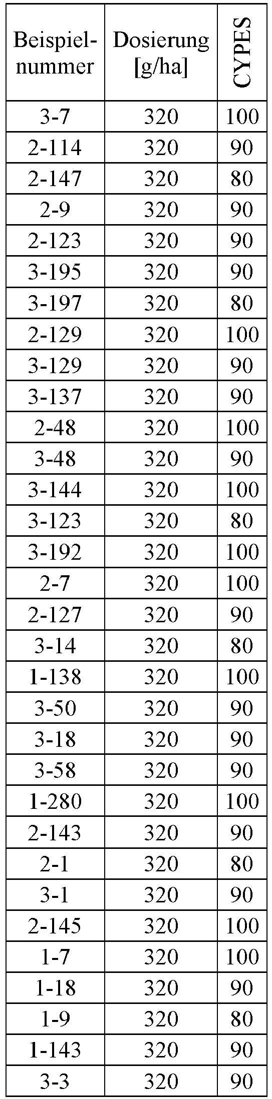

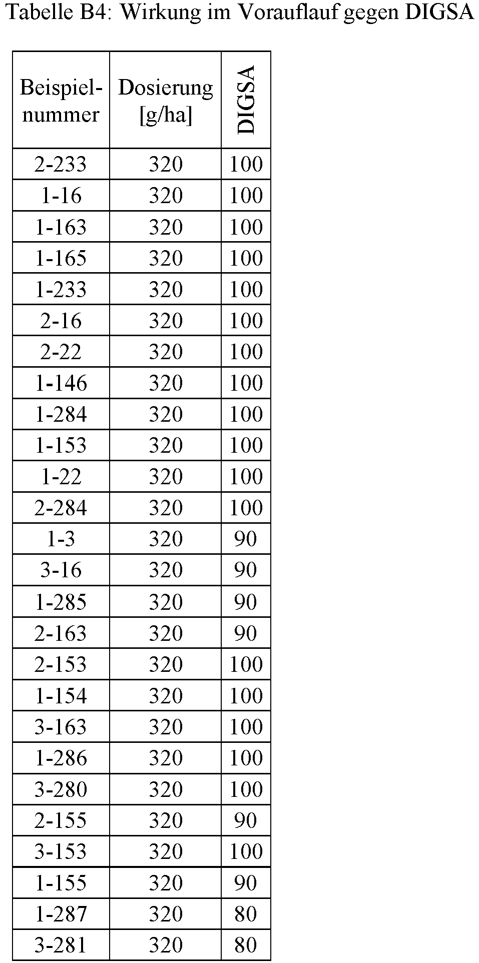

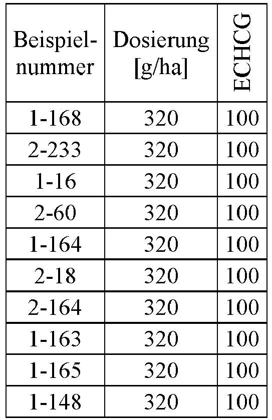

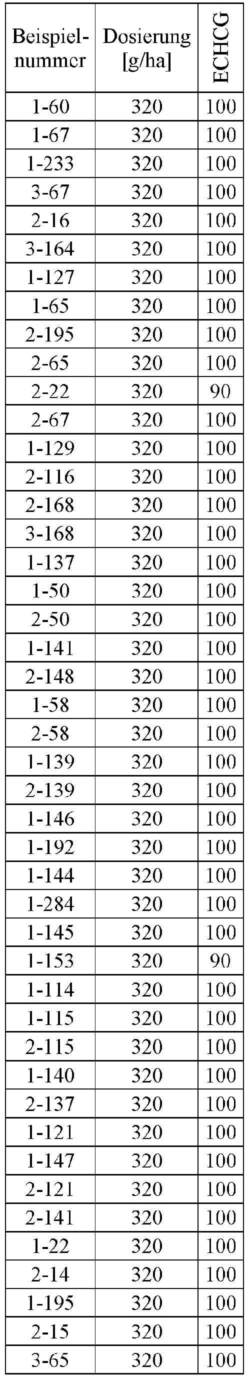

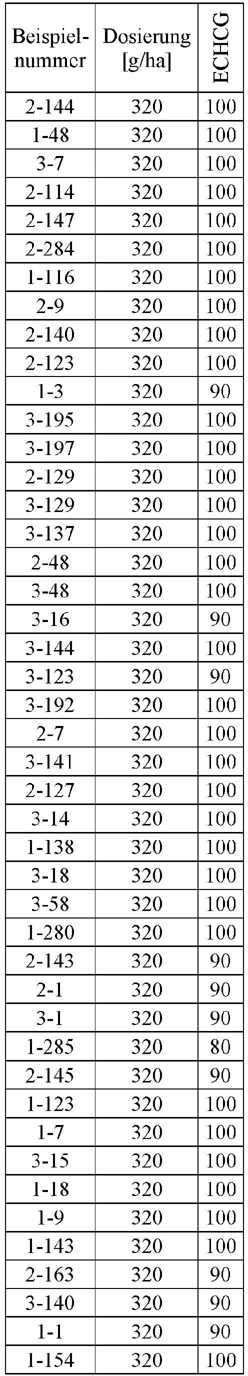

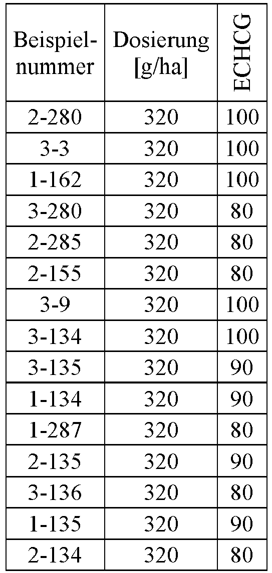

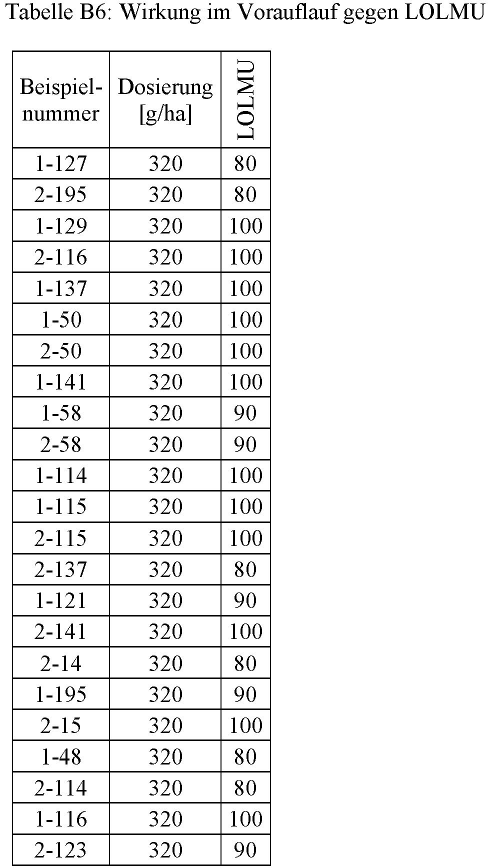

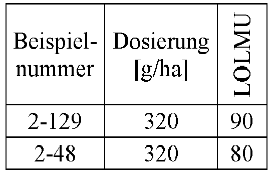

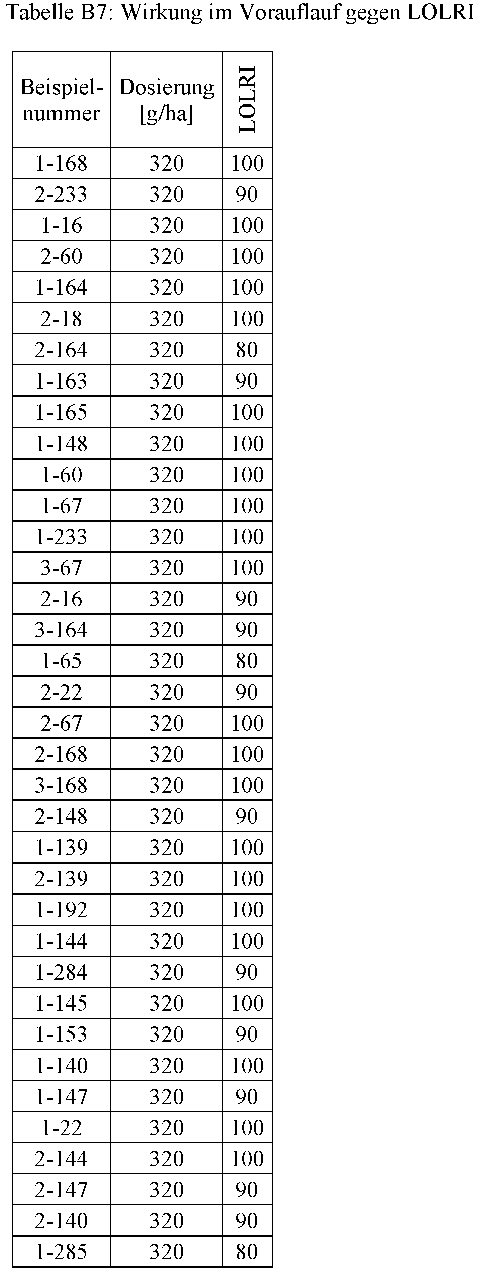

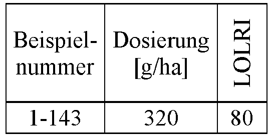

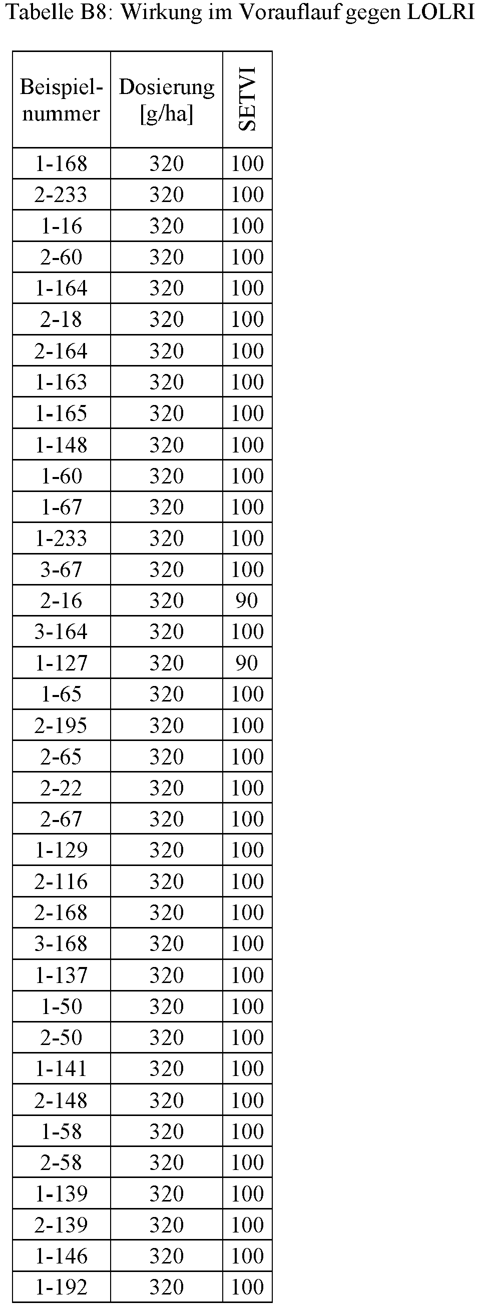

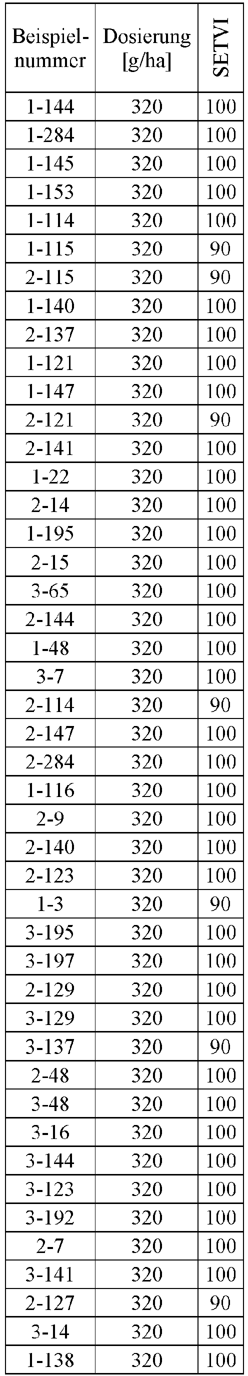

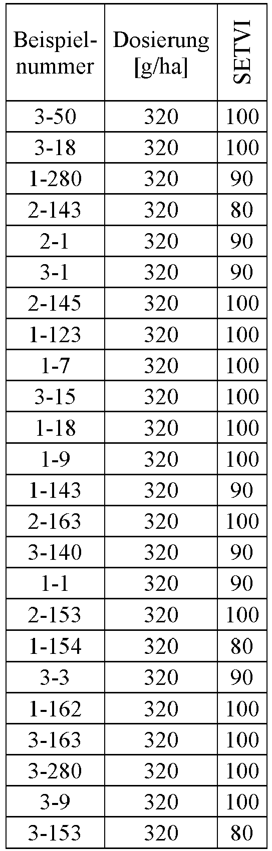

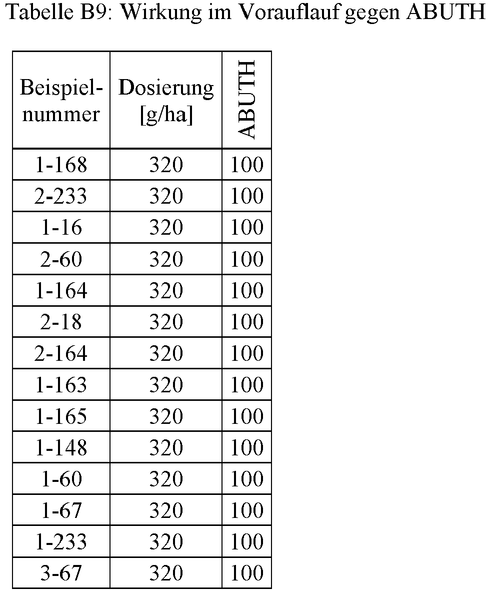

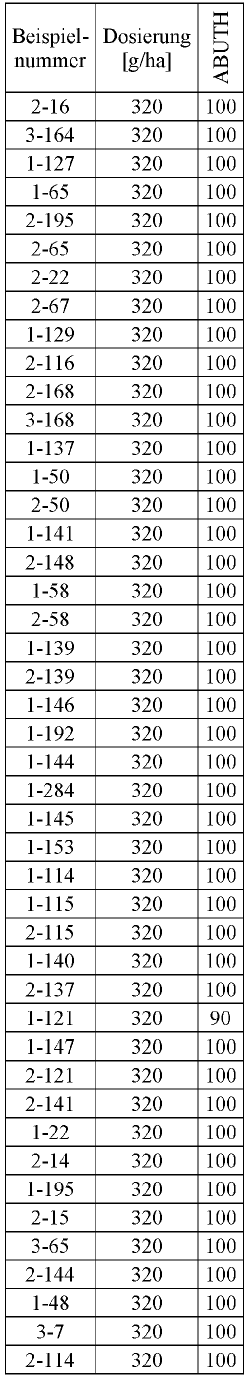

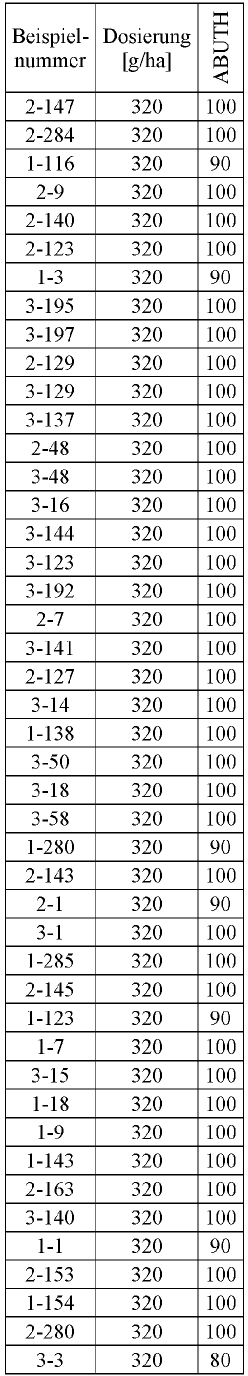

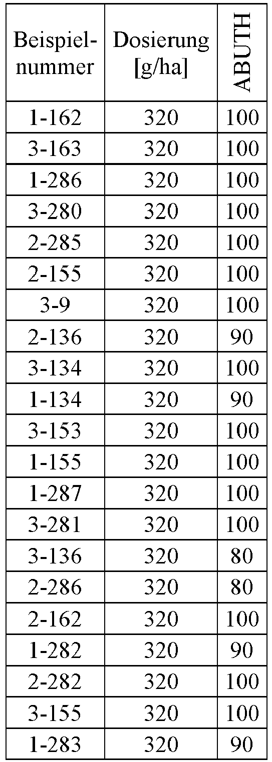

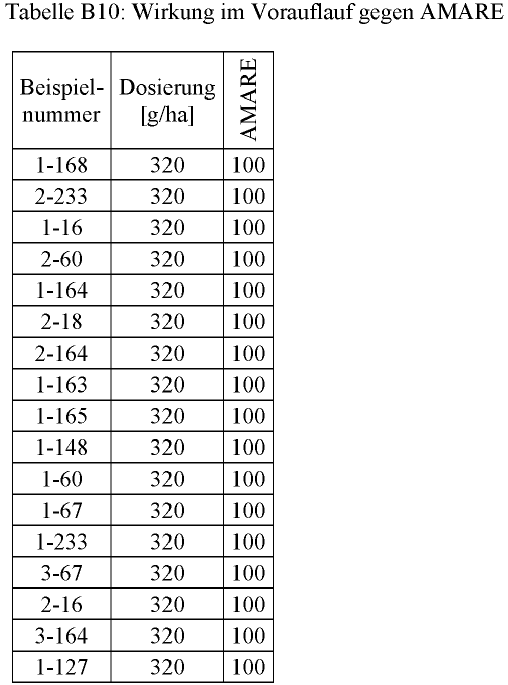

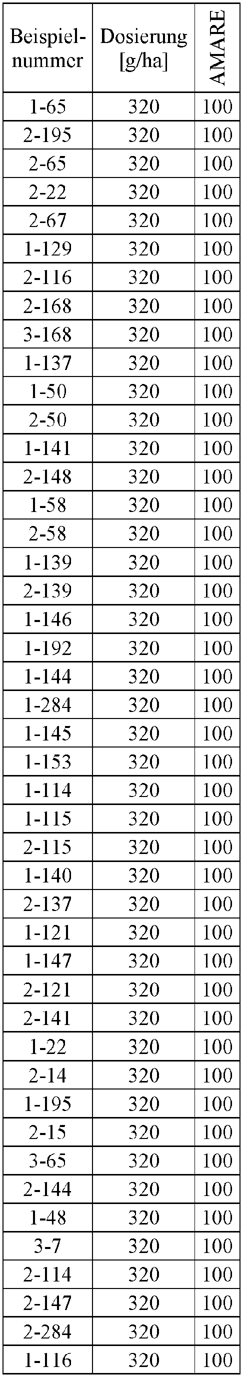

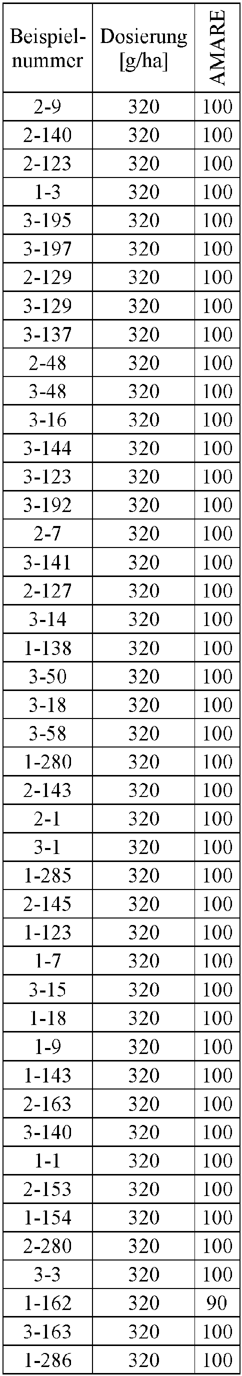

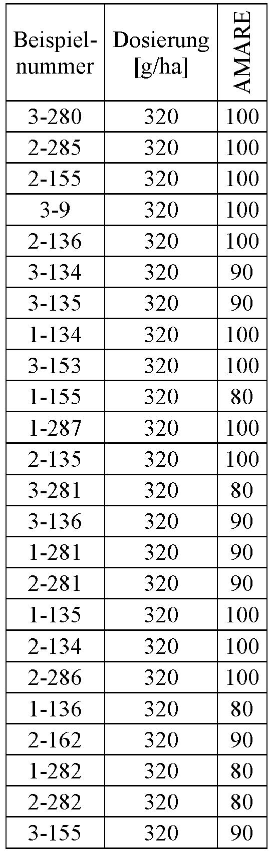

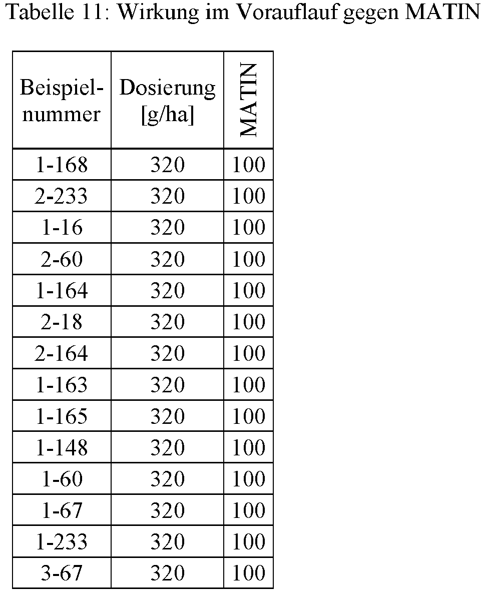

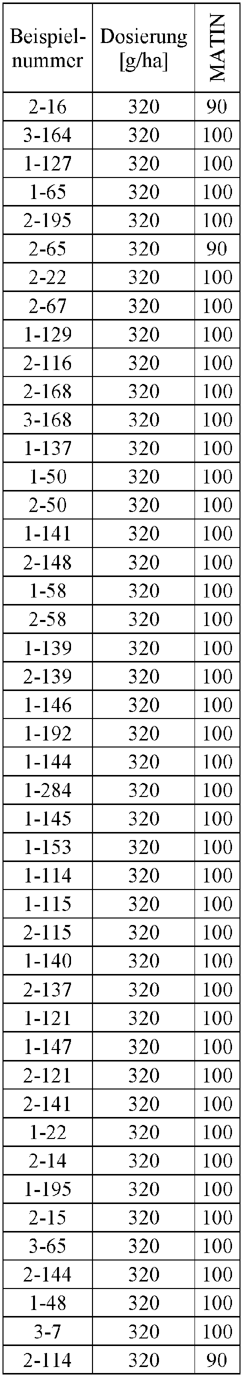

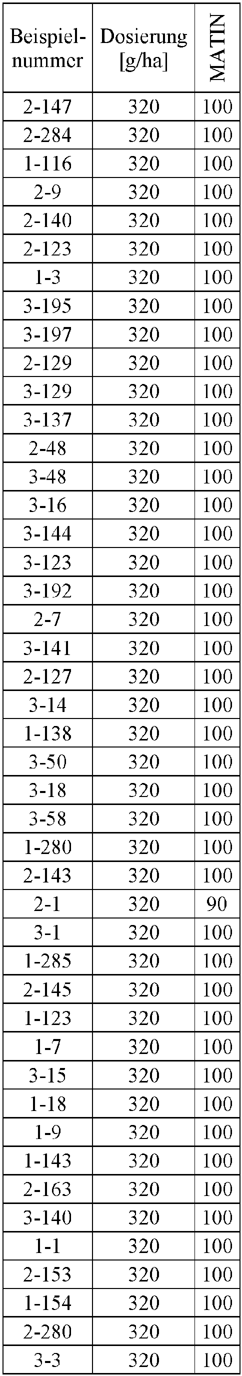

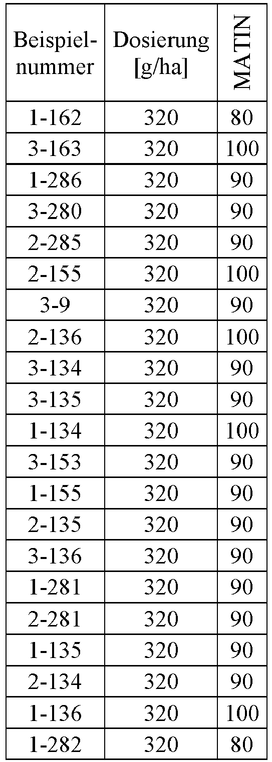

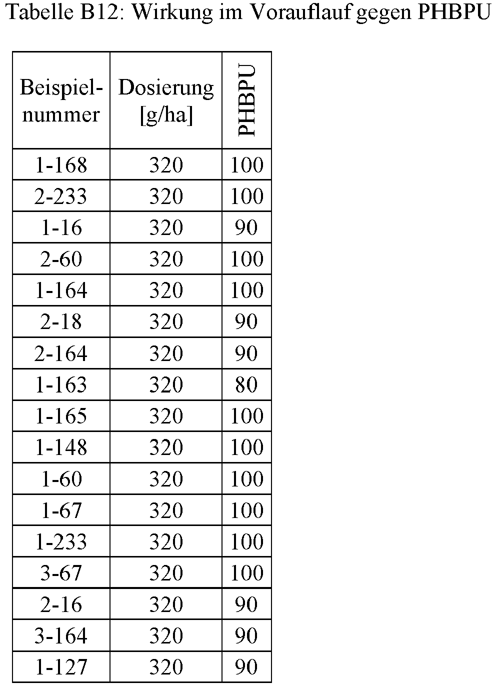

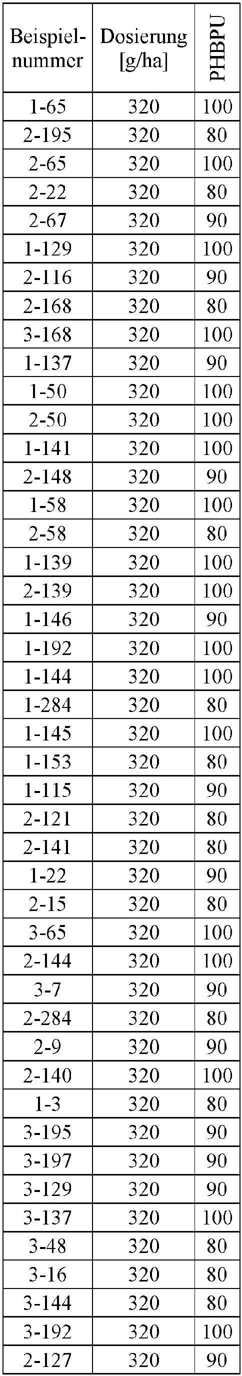

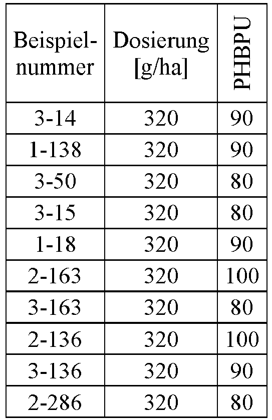

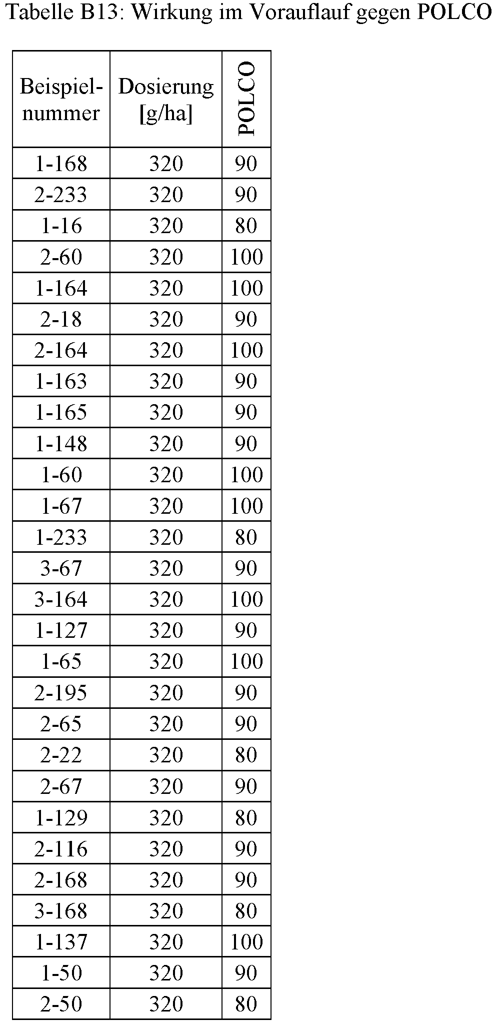

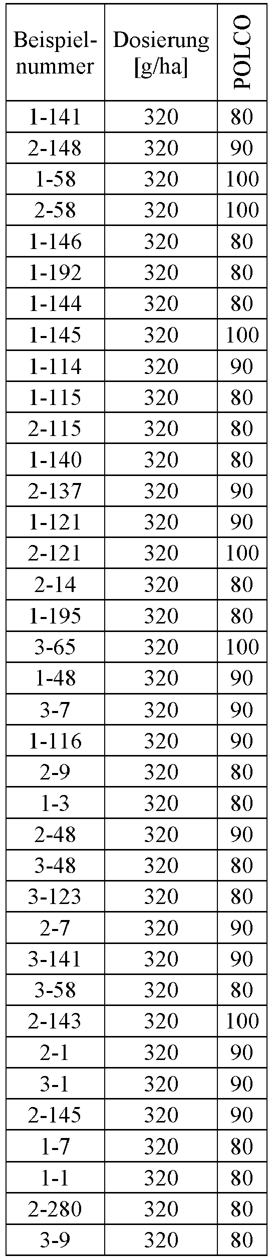

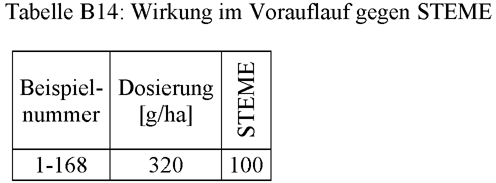

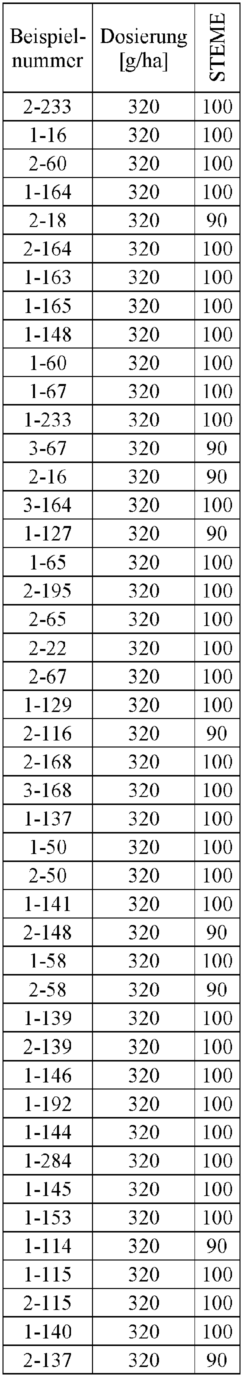

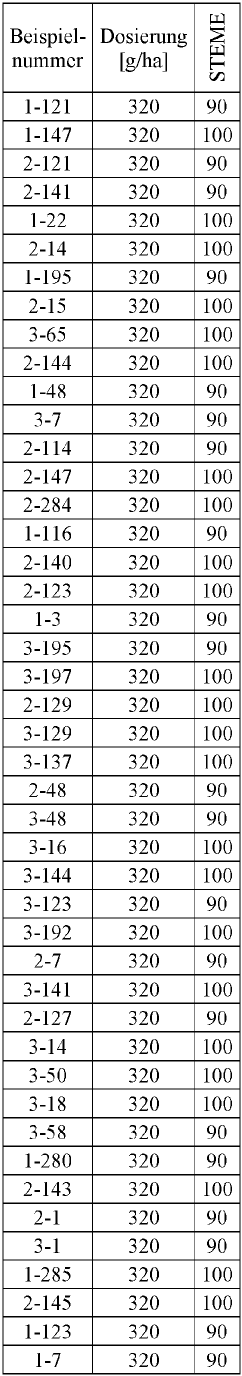

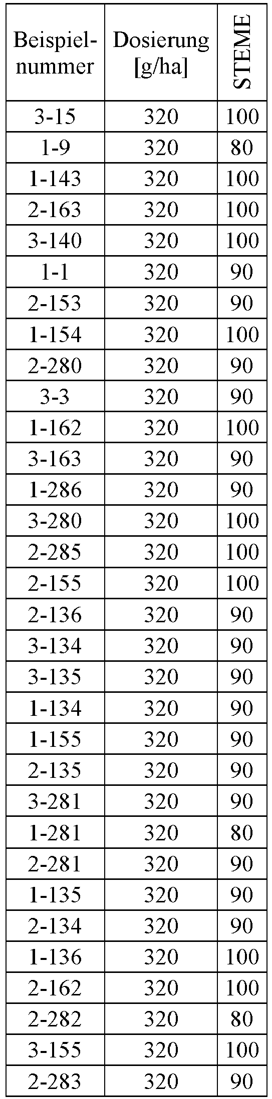

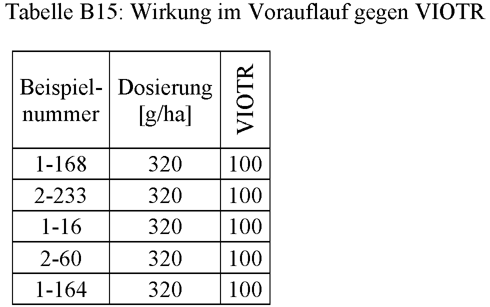

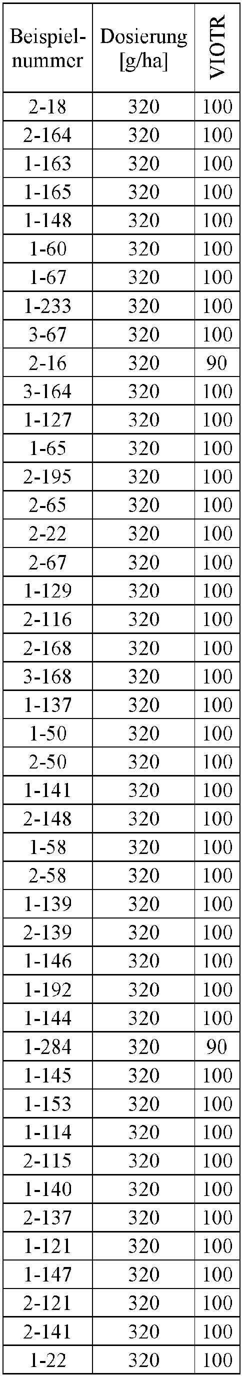

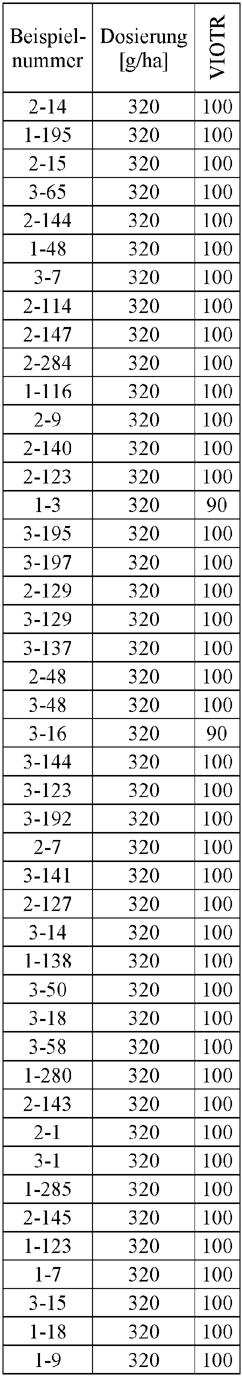

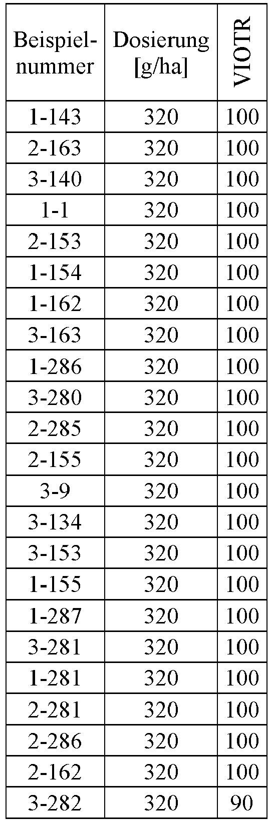

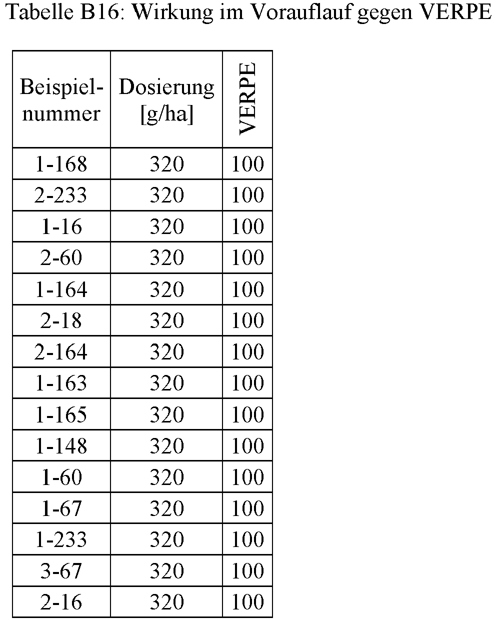

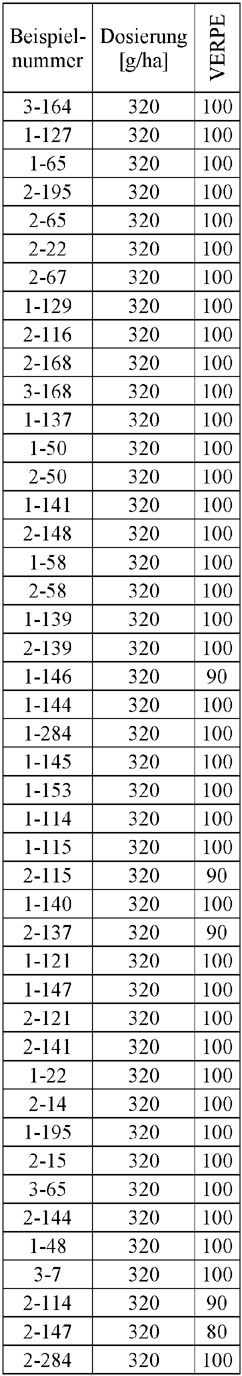

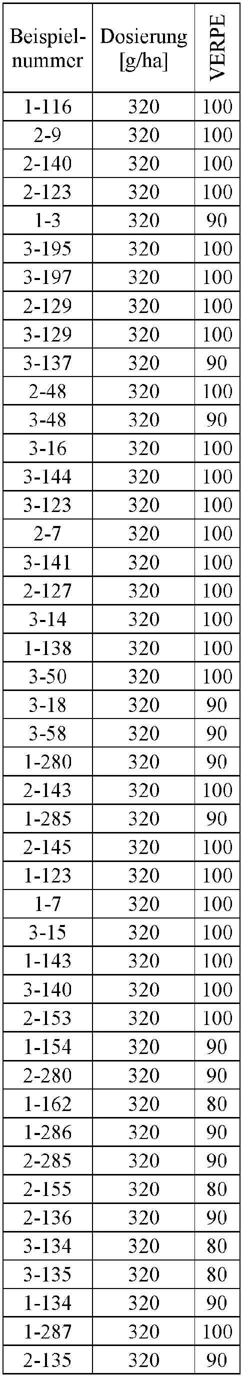

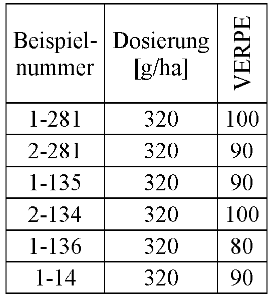

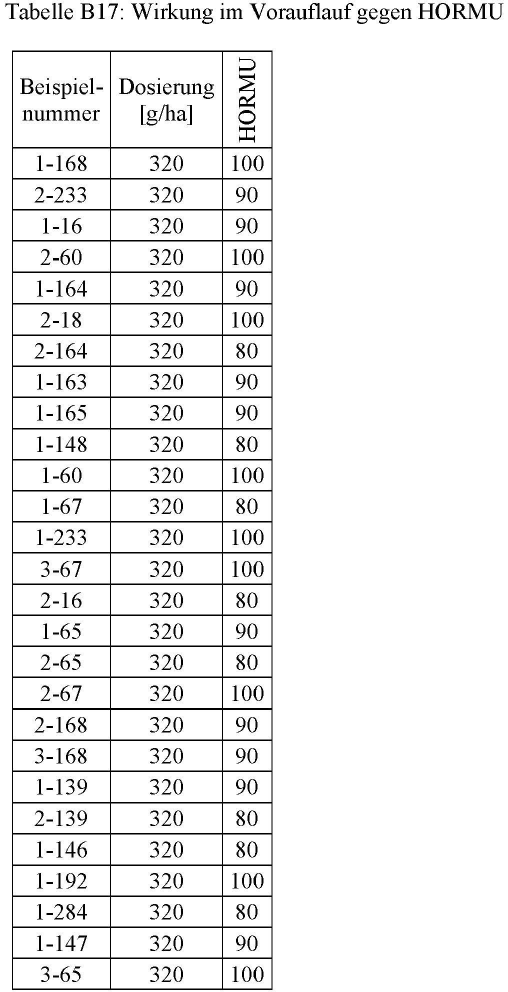

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigten zahlreiche erfindungsgemäße Verbindungen bei einer Aufwandmenge von 80 g oder weniger pro Hektar eine mindestens 80%-ige Wirkung gegen eine Vielzahl bedeutender Schadpflanzen. Gleichzeitig lassen erfindungsgemäße Verbindungen Gramineenkulturen wie Gerste, Weizen, Roggen, Hirse, Mais oder Reis im Nachauflaufverfahren selbst bei hohen Wirkstoffdosierungen praktisch ungeschädigt. Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen. Die Daten nachfolgender Tabellen B18 bis B34 zeigen beispielhaft die herbizide Wirkung der erfindungsgemäßen Verbindungen im Vorauflauf, wobei die herbizide Wirkung in Prozent angegeben ist.

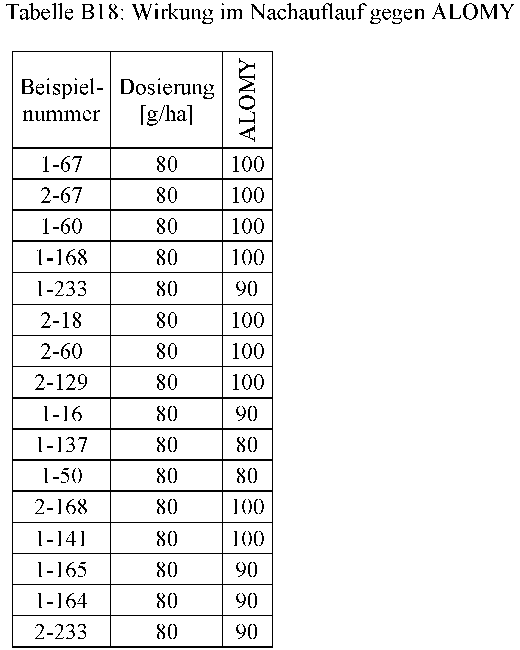

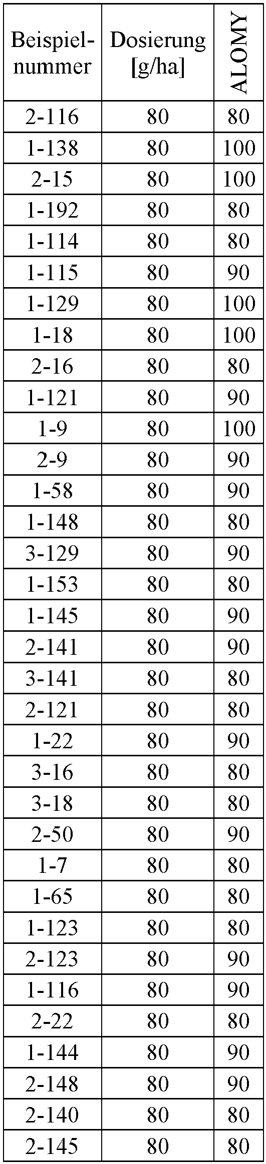

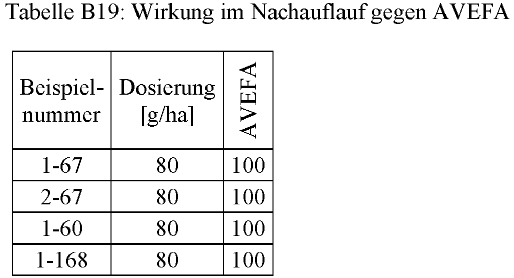

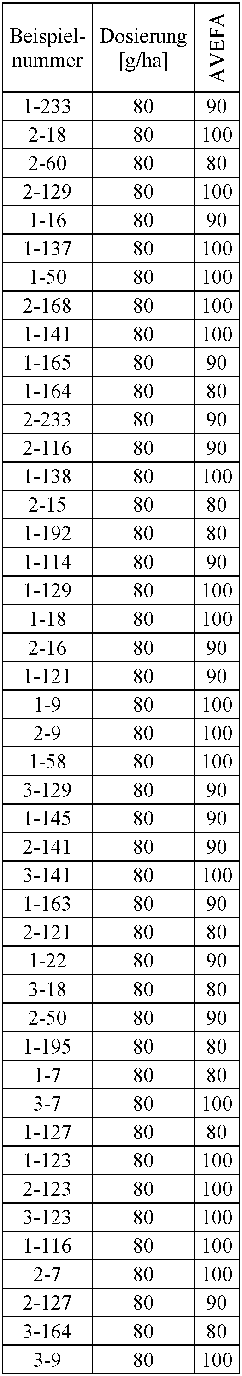

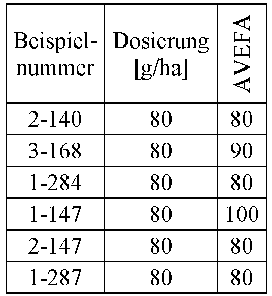

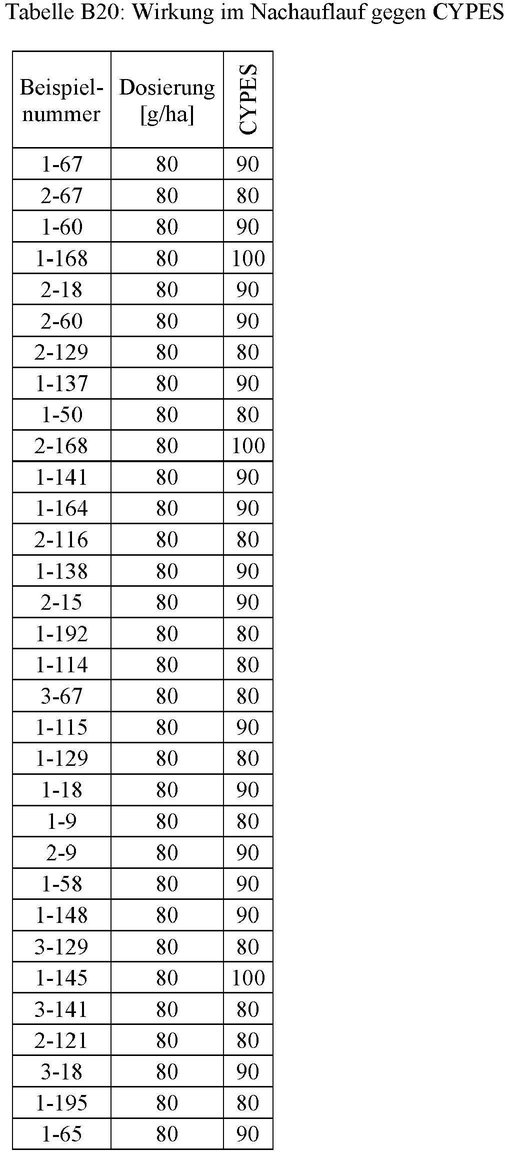

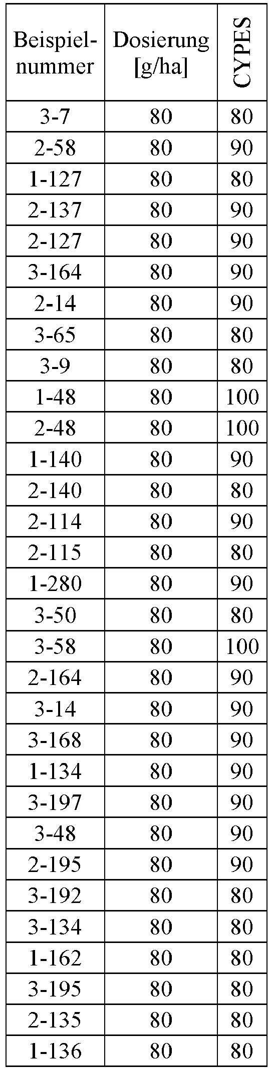

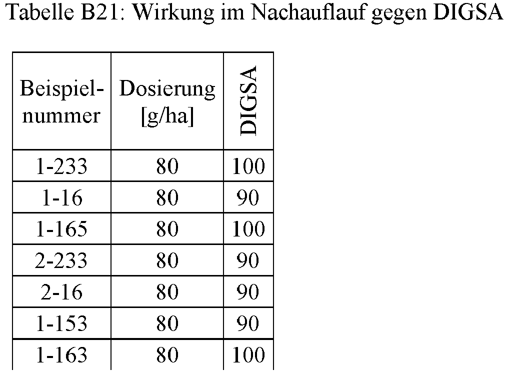

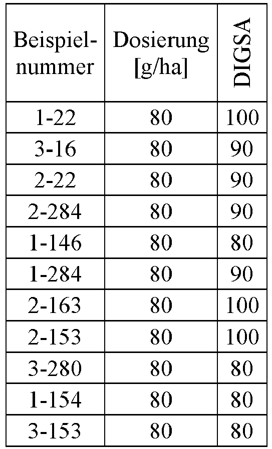

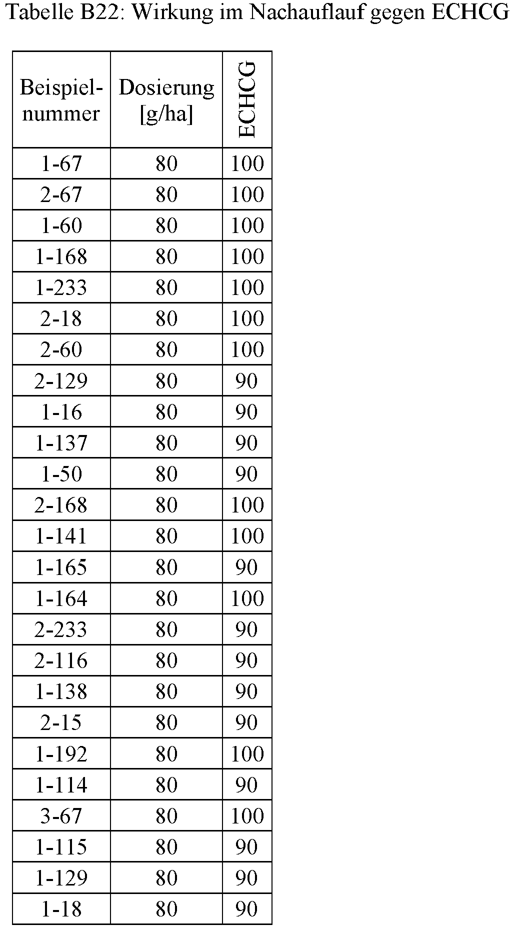

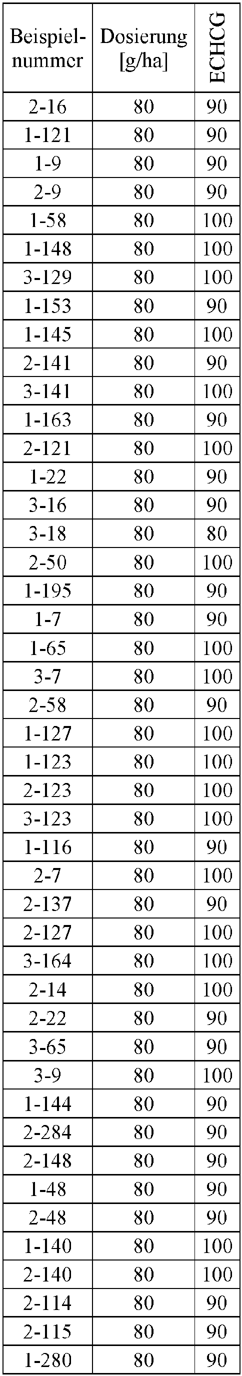

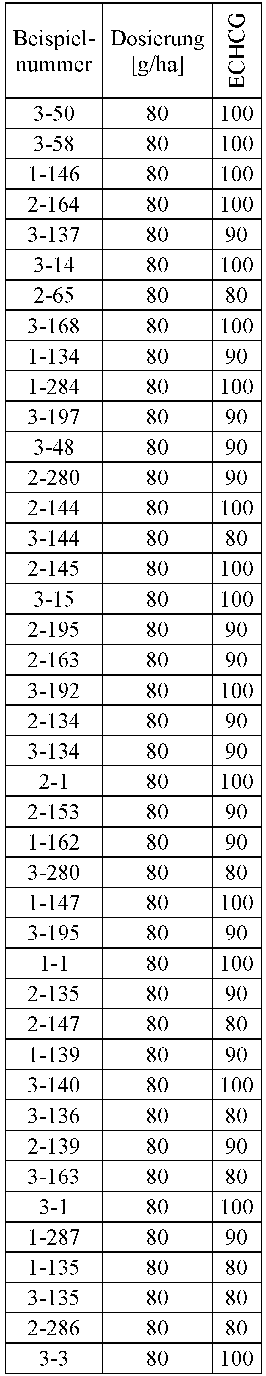

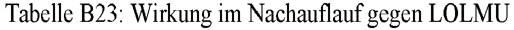

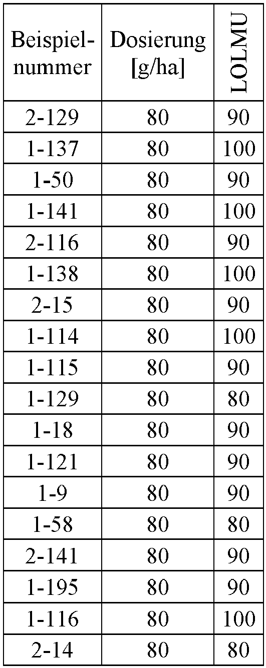

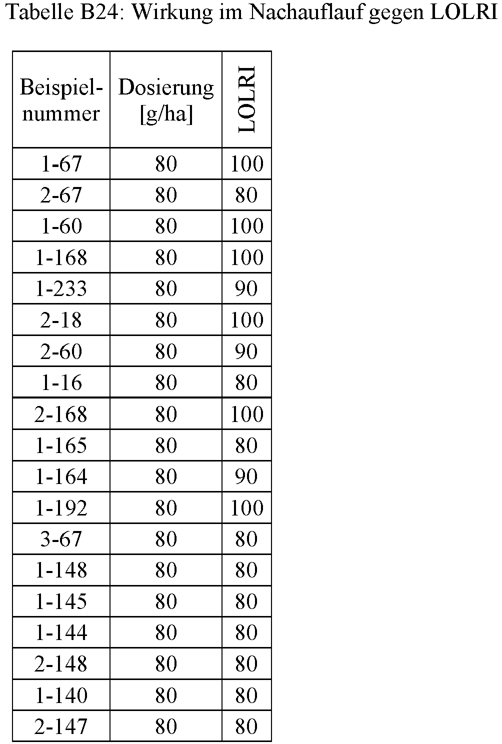

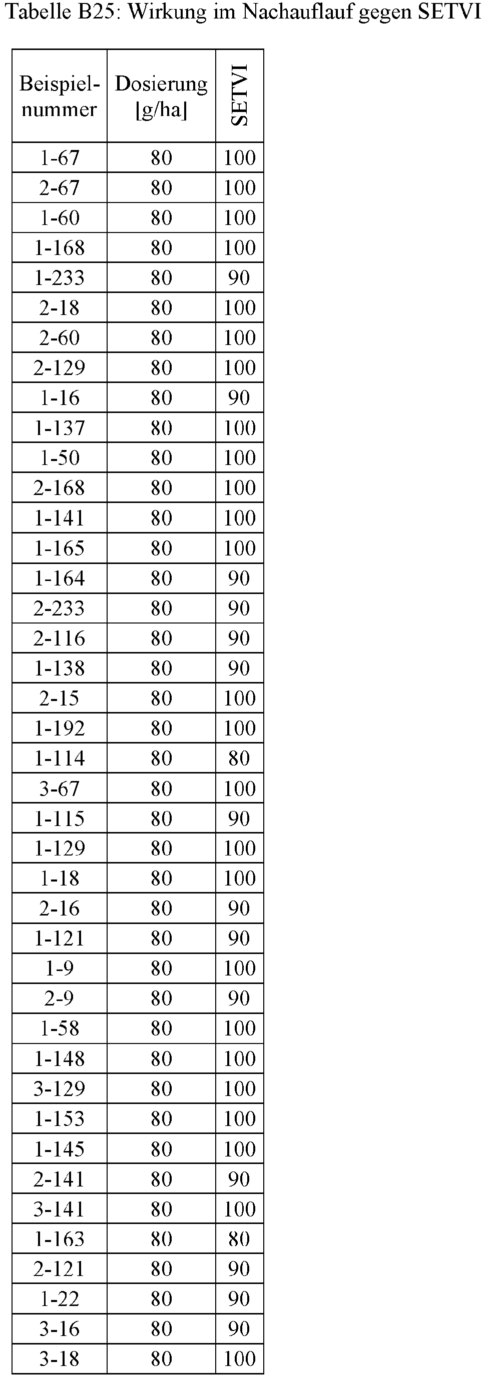

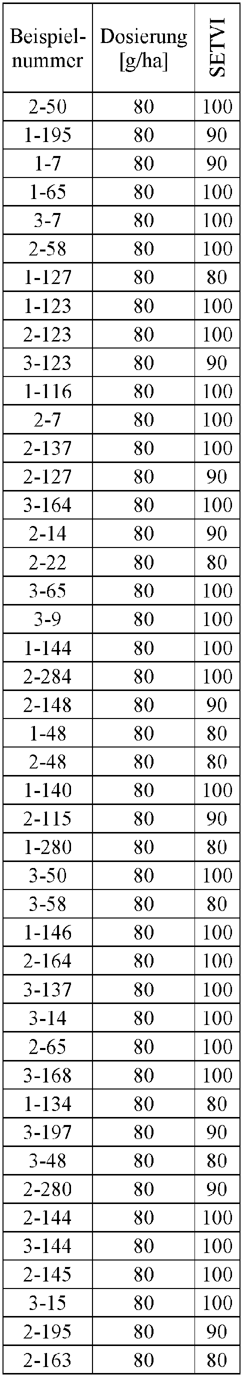

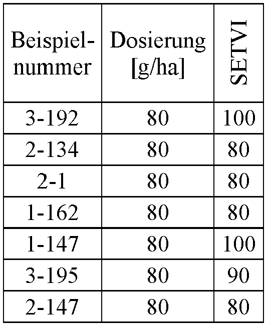

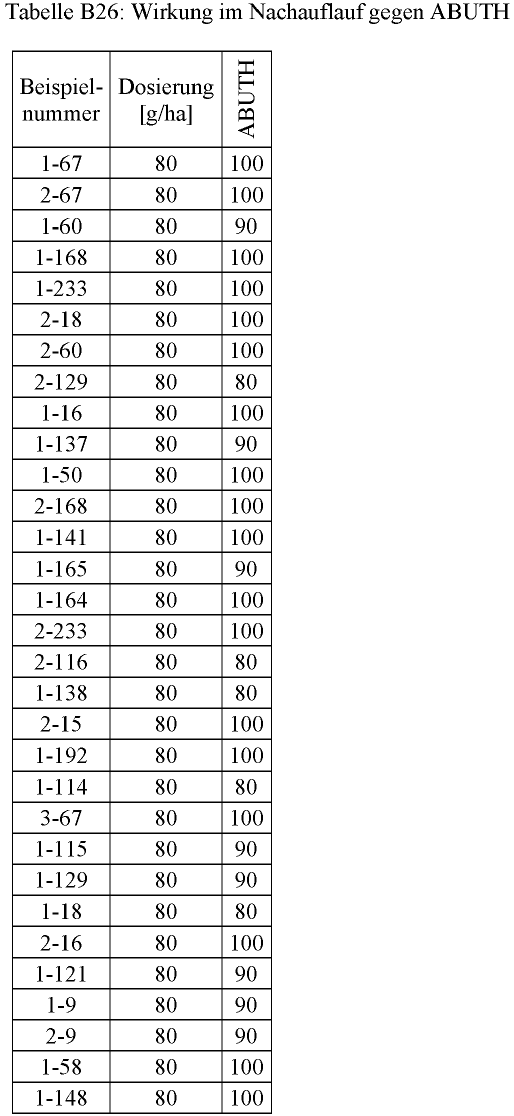

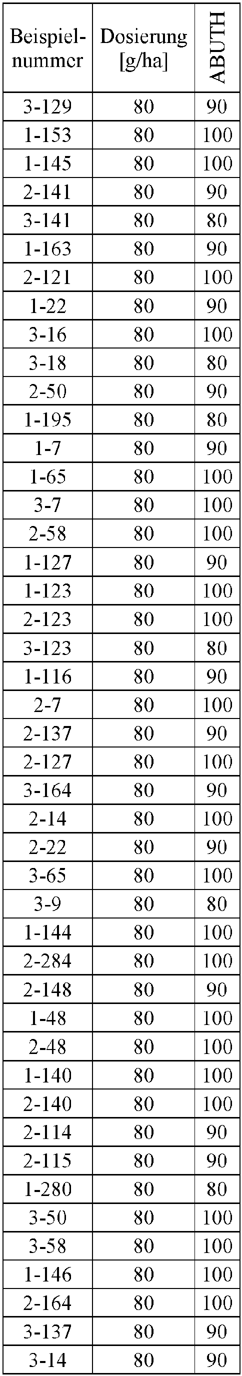

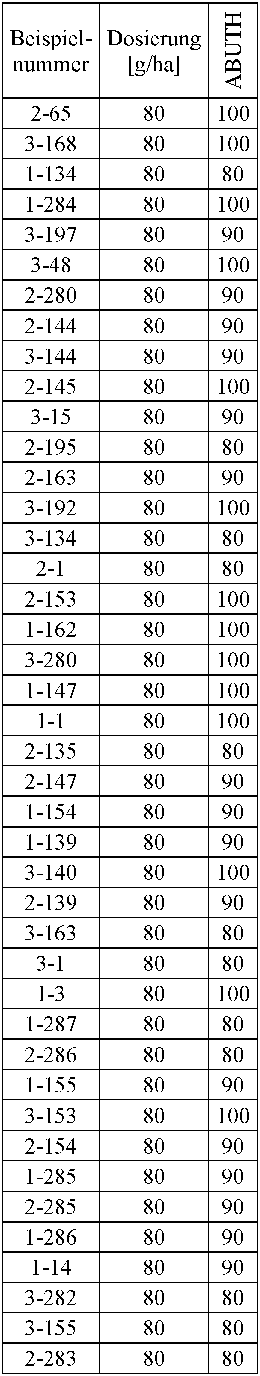

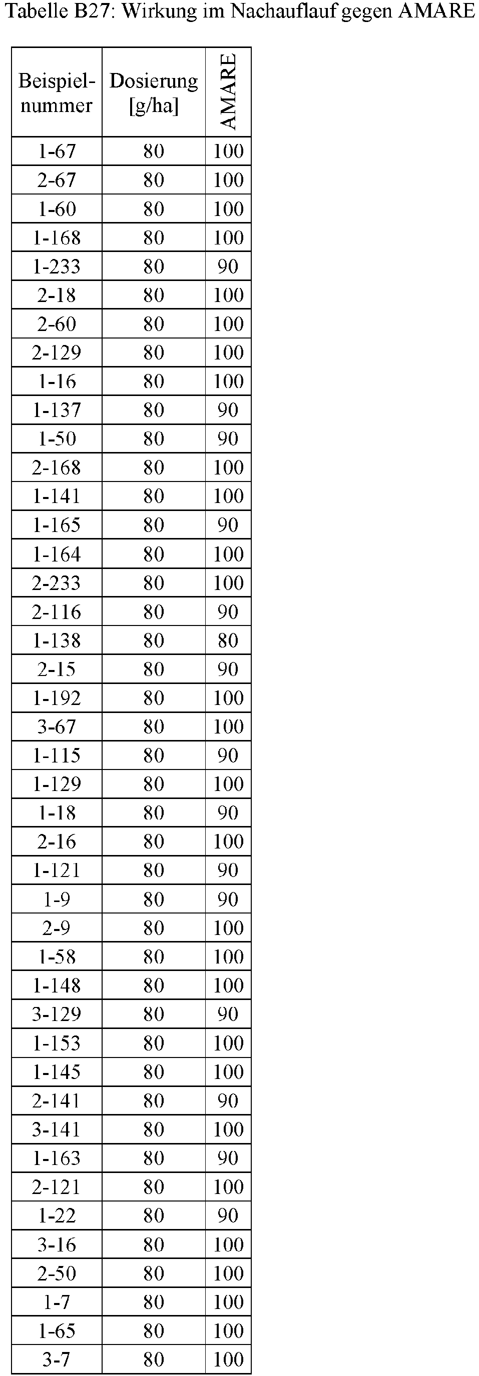

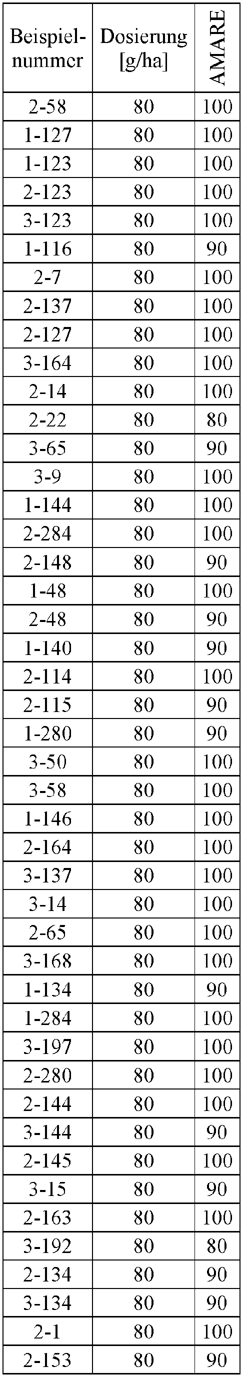

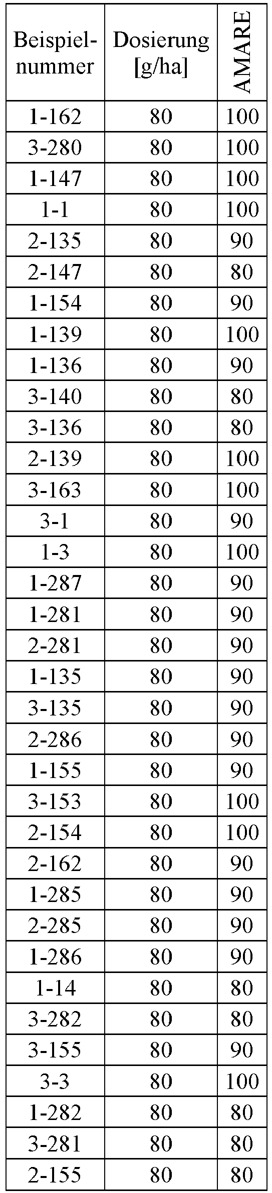

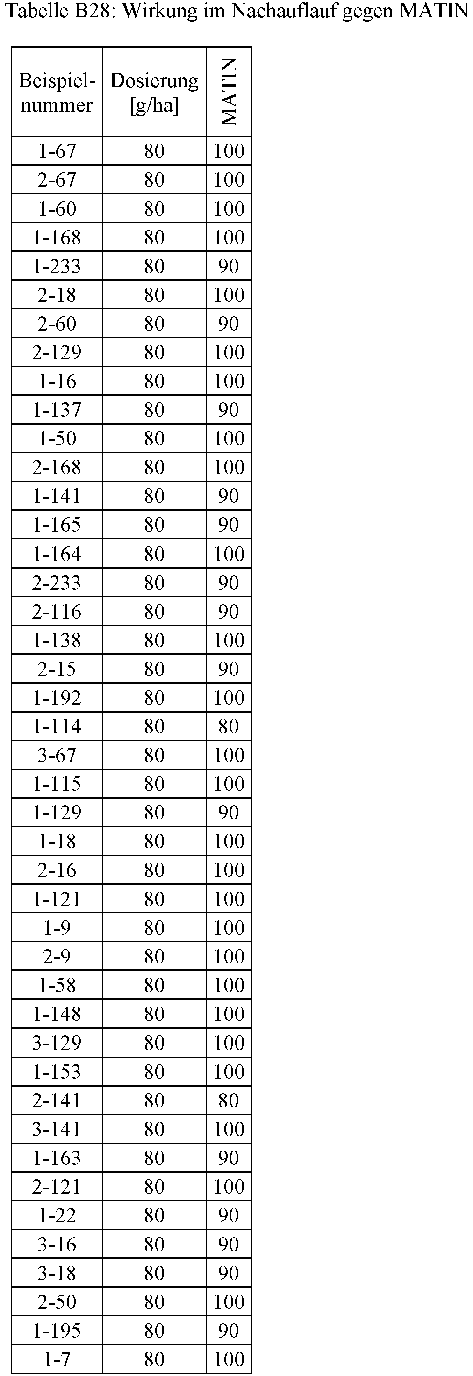

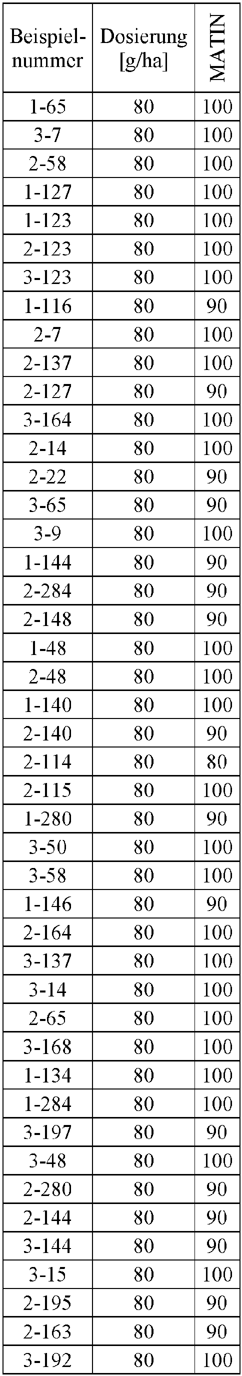

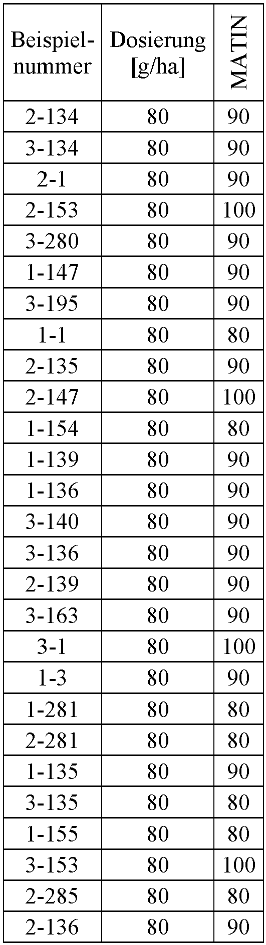

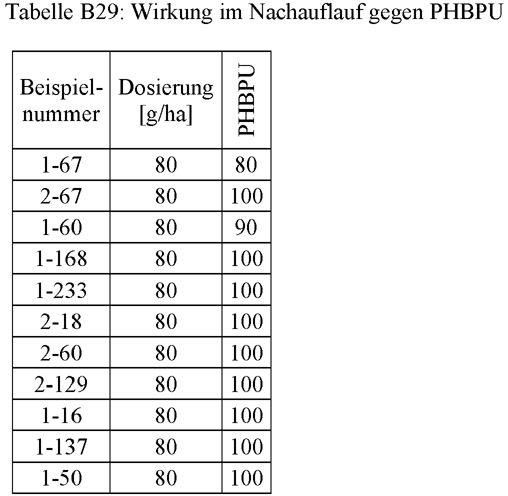

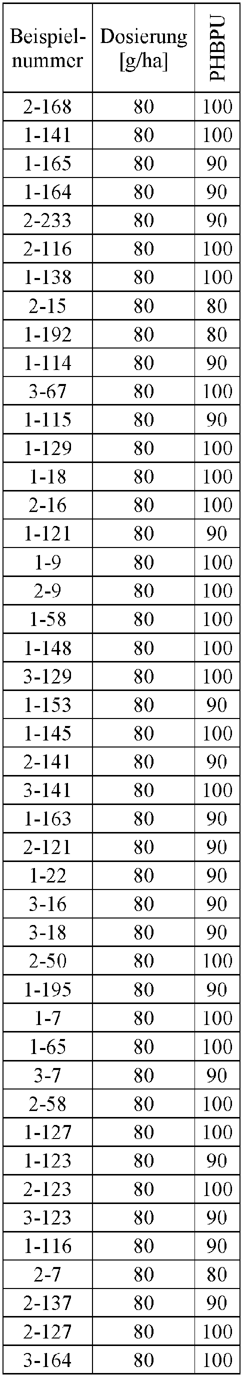

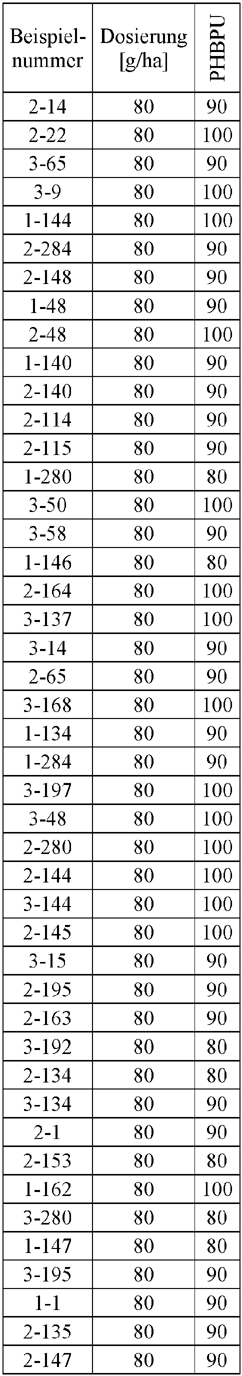

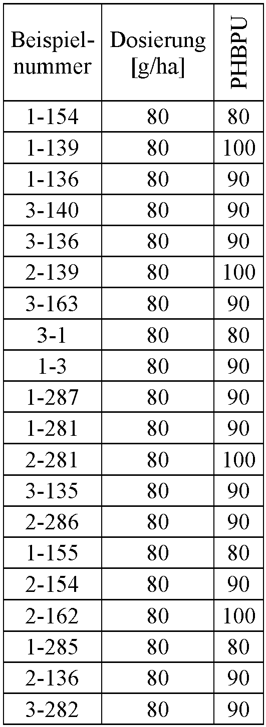

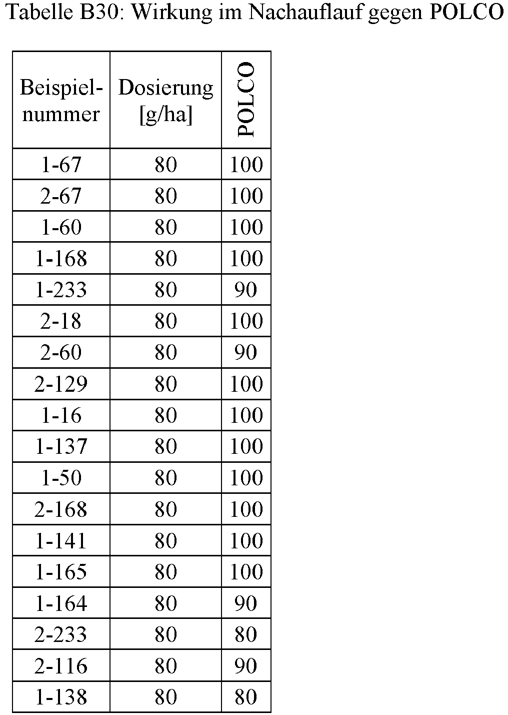

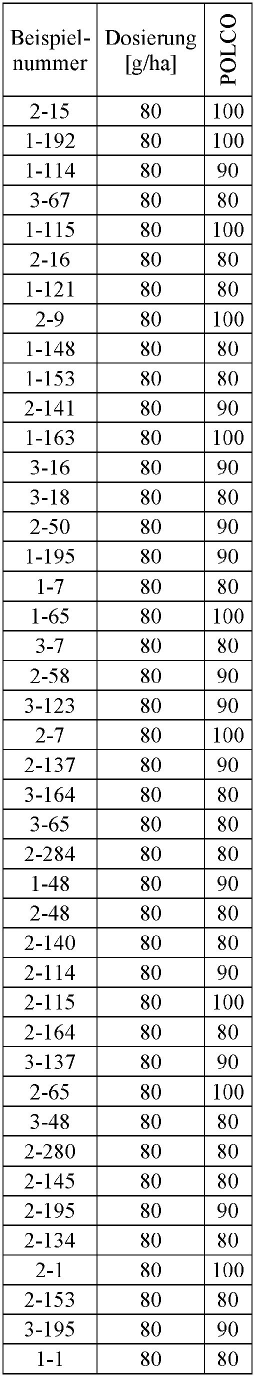

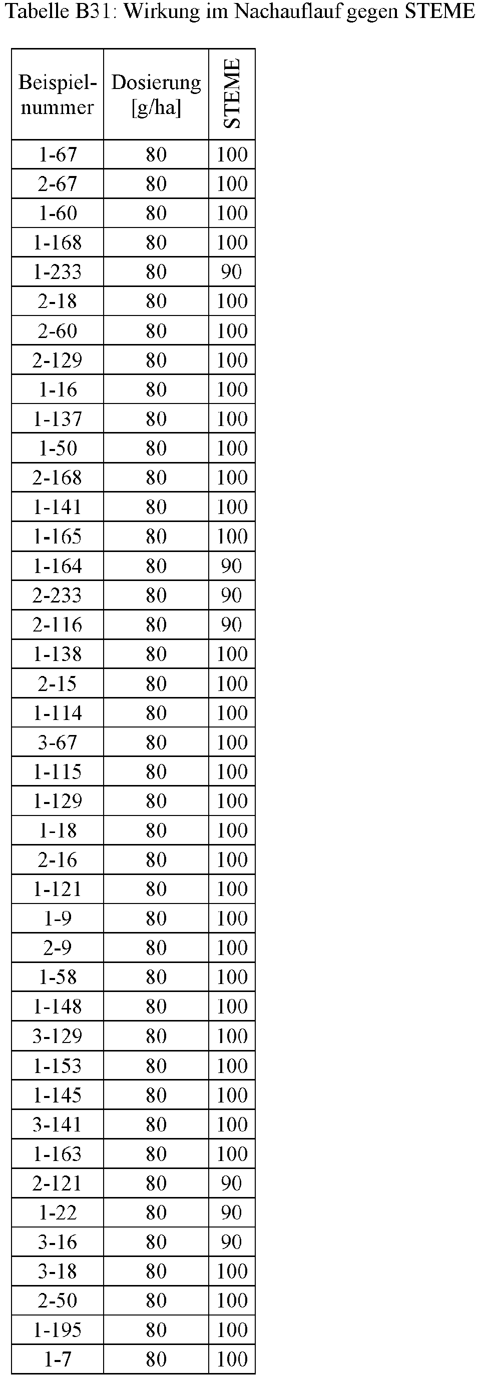

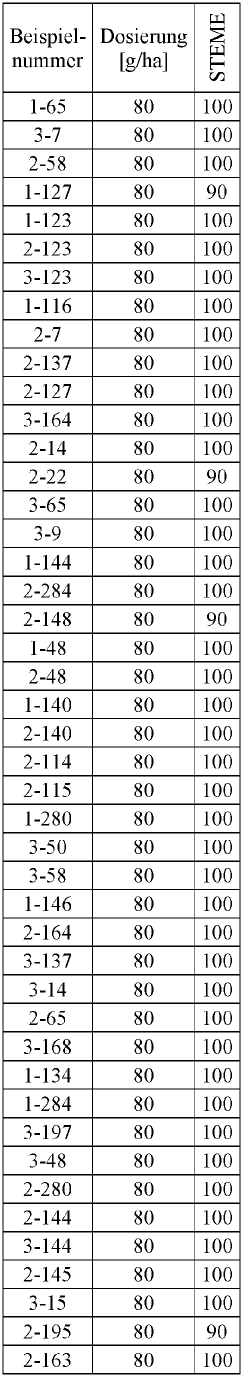

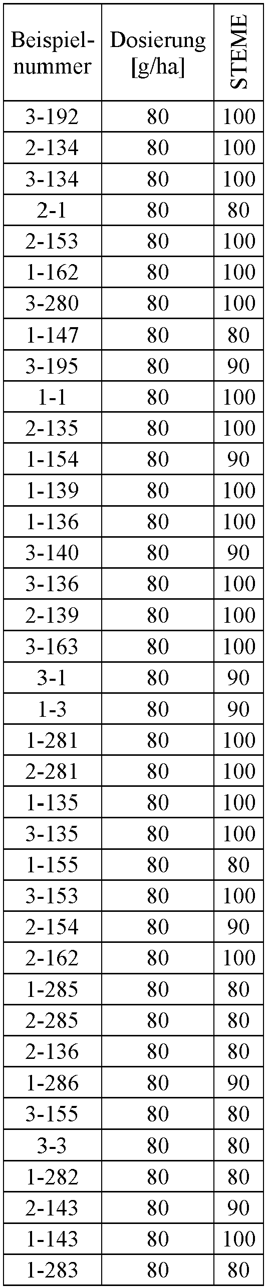

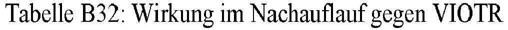

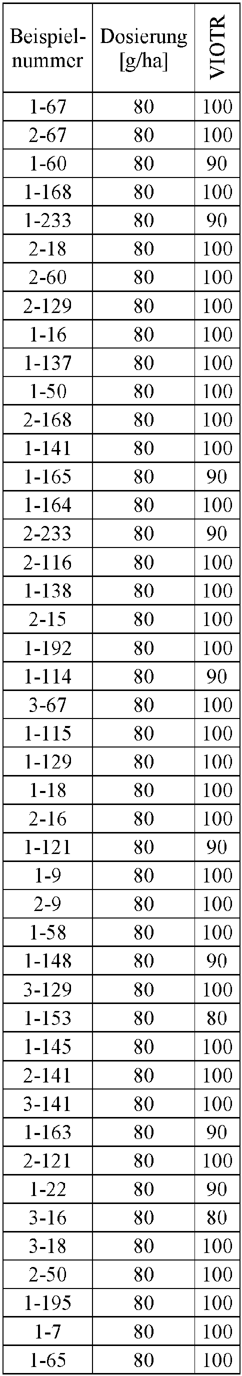

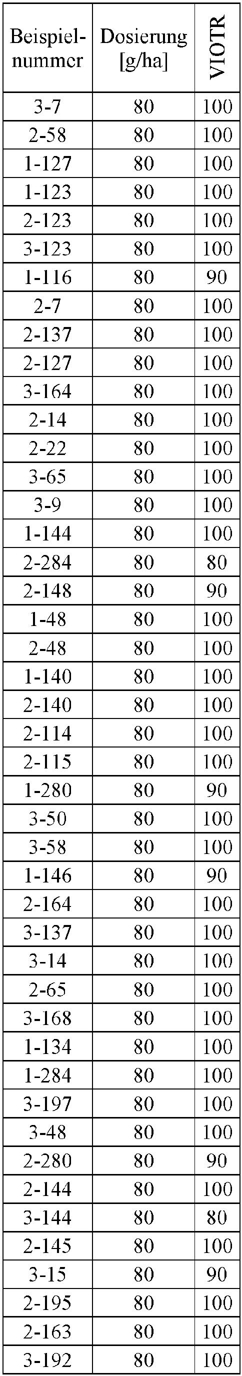

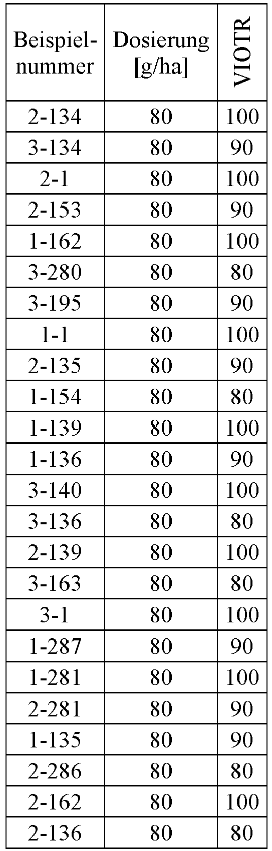

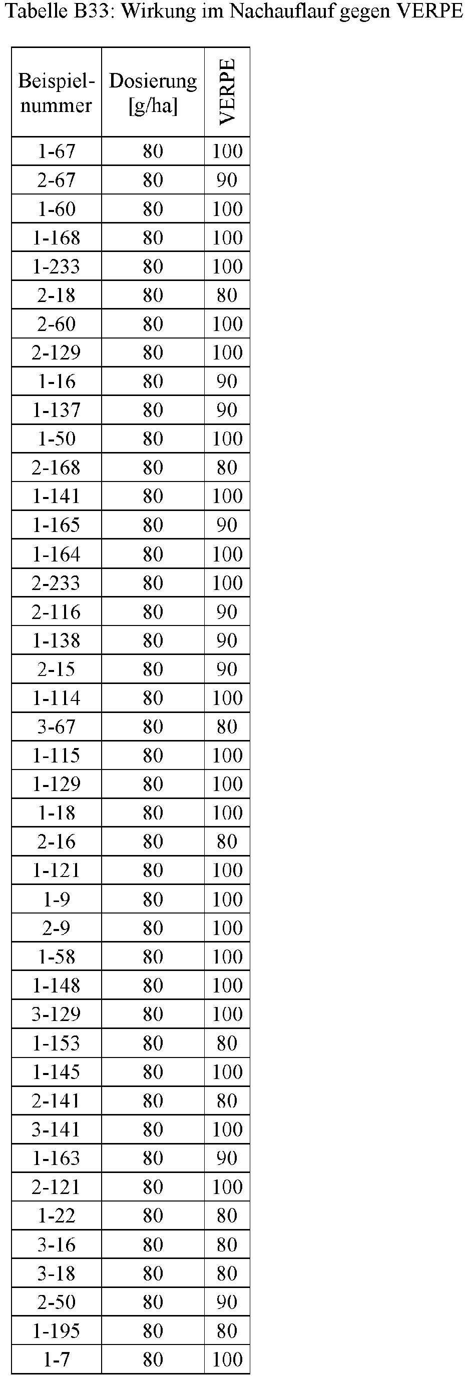

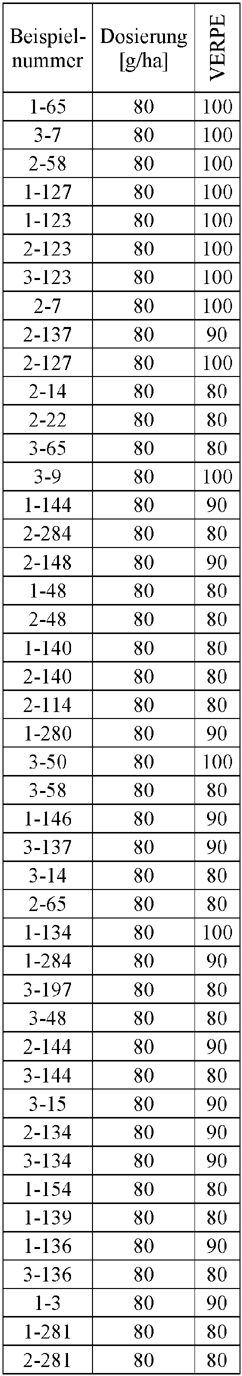

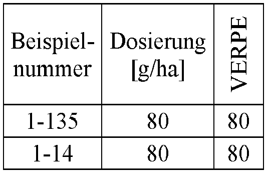

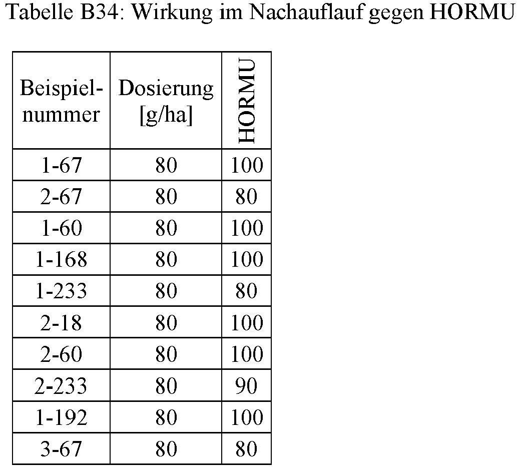

### 3. Vergleichsversuche

Die herbizide Wirkung gegen Schadpflanzen im Vor- und Nachauflauf einiger der in WO 2012/028579 A1 offenbarten Verbindungen wurde mit den von strukturell ähnlichsten erfindungsgemäßen Verbindungen verglichen. Die Daten dieser Vergleichsversuche zeigen die Überlegenheit der erfindungsgemäßen Verbindungen.

**Tabelle V1, Vorauflauf**

| Beispiel-Nr. | Dosierung (g a.i./ha) | Herbizide Wirkung gegen | | | |
|---|---|---|---|---|---|
| | | ALOMY | CYPES | POLCO | VIOTR |
| 1-127, erfindungsgemäß | 80 | 80 | 90 | 70 | 100 |
| 4-250, aus D1 | 80 | 20 | 60 | 0 | 50 |

**Tabelle V2 Vorauflauf**

| Beispiel-Nr. | Dosierung (g a.i./ha) | ECHCG | SETVI | AMARE | MATIN | VERPE |
|---|---|---|---|---|---|---|
| 1-129, erfindungsgemäß | 20 | 90 | 90 | 90 | 90 | 100 |
| 4-250, aus D1 | 20 | 40 | 10 | 60 | 30 | 50 |

**Tabelle V3, Vorauflauf**

| Beispiel-Nr. | Dosierung | Herbizide Wirkung gegen | | | | | | |
|---|---|---|---|---|---|---|---|---|
| | (g a.i./ ha) | ALOMY | CYPES | ECHCG | ABUTH | AMARE | MATIN | VIOTR |
| 1-127 | 80 | 80 | 90 | 100 | 100 | 100 | 90 | 100 |
| 4-251, aus D1 | 80 | 0 | 20 | 20 | 50 | 70 | 70 | 0 |
| | | | | | | | | |
| 1-129 | 80 | 100 | 100 | 100 | 100 | 100 | 100 | 100 |
| 4-251, aus D1 | 80 | 0 | 20 | 20 | 50 | 70 | 70 | 0 |

**Tabelle V4, Vorauflauf**

| Beispiel-Nr. | Dosierung (g a.i./ha) | Herbizide Wirkung gegen | | | |
|---|---|---|---|---|---|
| | | ABUTH | MATIN | STEME | VERPE |
| 1-127, erfindungsgemäß | 20 | 90 | 70 | 70 | 90 |
| 4-251, aus D1 | 20 | 40 | 0 | 30 | 40 |

**Tabelle V5, Vorauflauf**

| Beispiel-Nr. | Dosierung (g a.i./ha) | Herbizide Wirkung gegen | |
|---|---|---|---|
| | | MATIN | VIOTR |
| 1-7, erfindungsgemäß | 20 | 90 | 100 |
| 4-908, aus D1 | 20 | 20 | 20 |

**Tabelle V6, Vorauflauf**

| Beispiel-Nr. | Dosierung (g a.i./ha) | Herbizide Wirkung gegen | | |
|---|---|---|---|---|
| | | CYPES | PHBPU | POLCO |
| 1-9, erfindungsgemäß | 80 | 70 | 60 | 40 |
| 4-908, aus D1 | 80 | 10 | 40 | 10 |

**Tabelle V7, Vorauflauf**

| Beispiel-Nr. | Dosierung (g a.i./ha) | Herbizide Wirkung gegen | | |
|---|---|---|---|---|
| | | ABUTH | MATIN | VIOTR |
| 1-9, erfindungsgemäß | 20 | 100 | 90 | 70 |
| 4-908, aus D1 | 20 | 80 | 20 | 20 |
| | | | | |
| 2-9, erfindungsgemäß | 20 | 100 | 80 | 90 |
| 5-826, aus D1 | 20 | 60 | 0 | 0 |

**Tabelle V8, Vorauflauf**

| Beispiel-Nr. | Dosierung | Herbizide Wirkung gegen | | | | | |
|---|---|---|---|---|---|---|---|
| | (g a.i./ha) | ECHCG | AMARE | MATIN | STEME | VIOTR | VERPE |
| 1-7, erfindungsgemäß | 20 | 70 | 100 | 90 | 90 | 100 | 100 |
| 4-933, aus D1 | 20 | 0 | 80 | 40 | 60 | 0 | 0 |

**Tabelle V9, Vorauflauf**

| Beispiel-Nr. | Dosierung (g a.i./ha) | Herbizide Wirkung gegen | |
|---|---|---|---|
| | | SETVI | VIOTR |
| 1-9, erfindungsgemäß | 80 | 100 | 100 |
| 4-933, aus D1 | 80 | 0 | 0 |

**Tabelle V10, Vorauflauf**

| Beispiel-Nr. | Dosierung (g a.i./ha) | Herbizide Wirkung gegen | | | | |
|---|---|---|---|---|---|---|
| | | ALOMY | CYPES | SETVI | MATIN | STEME |
| 2-7, erfindungsgemäß | 80 | 70 | 90 | 80 | 100 | 90 |
| 5-826, aus D1 | 80 | 30 | 0 | 0 | 80 | 70 |

**Tabelle V11, Vorauflauf**

| Beispiel-Nr. | Dosierung (g a.i./ha) | Herbizide Wirkung gegen CYPES |
|---|---|---|
| 2-7, erfindungsgemäß | 80 | 90 |
| 5-827, aus D1 | 80 | 60 |

**Tabelle V12, Vorauflauf**

| Beispiel-Nr. | Dosierung (g a.i./ha) | Herbizide Wirkung gegen VIOTR |
|---|---|---|
| 2-7, erfindungsgemäß | 20 | 90 |
| 5-827, aus D1 | 20 | 70 |

**Tabelle V13, Vorauflauf**

| Beispiel-Nr. | Dosierung (g a.i./ha) | Herbizide Wirkung gegen AMARE VIOTR | |
|---|---|---|---|
| 2-9, erfindungsgemäß | 20 | 100 | 90 |
| 5-827, aus D1 | 20 | 80 | 70 |

**Tabelle V14, Nachauflauf**

| Beispiel- Nr. | Dosierung (g a.i./ha) | Herbizide Wirkung gegen AMARE MATIN | |
|---|---|---|---|
| 1-127, erfindungsgemäß | 5 | 100 | 90 |
| 4-250, aus D1 | 5 | 0 | 20 |

**Tabelle V15, Nachauflauf**

| Beispiel-Nr. | Dosierung (g a.i./ha) | Herbizide Wirkung gegen | | | | | |
|---|---|---|---|---|---|---|---|
| | | ALOMY | AVEFA | CYPES | SETVI | AMARE | MATIN |
| 1-129, erfindungsgemäß | 5 | 70 | 60 | 60 | 100 | 100 | 80 |
| 4-250, aus D1 | 5 | 20 | 20 | 40 | 80 | 0 | 20 |
| | | | | | | | |
| 1-129, erfindungsgemäß | 5 | 70 | 60 | 60 | 100 | 100 | 80 |
| 4-251, aus D1 | 5 | 0 | 0 | 30 | 0 | 80 | 0 |

**Tabelle V16, Nachauflauf**

| Beispiel-Nr. | Dosierung (g a.i./ha) | Herbizide Wirkung gegen | | | |
|---|---|---|---|---|---|
| | | ECHCG | MATIN | PHBPU | VIOTR |
| 1-127, erfindung sgemäß | 20 | 100 | 100 | 100 | 100 |
| 4-251 aus D1 | 20 | 80 | 30 | 60 | 50 |
| | | | | | |
| 1-127, erfindung sgemäß | 5 | 100 | 90 | 70 | 100 |
| 4-251 aus D1 | 5 | 50 | 0 | 40 | 20 |

**Tabelle V17, Nachauflauf**

| Beispiel-Nr. | Dosierung (g a.i./ha) | Herbizide Wirkung gegen | |
|---|---|---|---|
| | | MATIN | VIOTR |
| 1-7, erfindungsgemäß | 5 | 100 | 100 |
| 4-908 aus D1 | 5 | 80 | 80 |
| | | | |
| 1-9, erfindungsgemäß | 5 | 100 | 100 |
| 4-908 aus D1 | 5 | 80 | 80 |
| | | | |
| 1-7, erfindungsgemäß | 5 | 100 | 100 |
| 4-933 aus D1 | 5 | 60 | 60 |
| | | | |
| 2-7, erfindungsgemäß | 5 | 100 | 100 |
| 5-826 aus D1 | 5 | 70 | 80 |
| | | | |
| 2-9, erfindungsgemäß | 5 | 90 | 100 |
| 5-826 aus D1 | 5 | 70 | 80 |

**Tabelle V18, Nachauflauf**

| Beispiel-Nr. | Dosierung (g a.i./ha) | Herbizide Wirkung gegen | | | | | | |
|---|---|---|---|---|---|---|---|---|
| | | ALOMY | AVEFA | CYPES | SETVI | MATIN | PHBPU | STEME |
| 1-9, erfindungsgemäß | 20 | 80 | 80 | 70 | 100 | 100 | 100 | 100 |
| 4-933 aus D1 | 20 | 30 | 60 | 10 | 80 | 70 | 80 | 80 |

**Tabelle V19, Nachauflauf**

| Beispiel-Nr. | Dosierung (g a.i./ha) | SETVI | ABUTH | MATIN | STEME | VIOTR |
|---|---|---|---|---|---|---|
| 2-7, erfindungsgemäß | 5 | 90 | 100 | 100 | 100 | 100 |
| 5-826 aus D1 | 5 | 40 | 60 | 70 | 30 | 80 |

**Tabelle V20, Nachauflauf**

| Beispiel-Nr. | Dosierung (g a.i./ha) | Herbizide Wirkung gegen | | | |
|---|---|---|---|---|---|
| | | ALOMY | CYPES | ABUTH | POLCO |
| 2-9, erfindungsgemäß | 80 | 90 | 90 | 90 | 100 |
| 5-826 aus D1 | 80 | 70 | 0 | 60 | 60 |

**Tabelle V21, Nachauflauf**

| Beispiel-Nr. | Dosierung (g a.i./ha) | Herbizide Wirkung gegen | | |
|---|---|---|---|---|
| | | ABUTH | AMARE | STEME |
| 2-7, erfindungsgemäß | 5 | 100 | 100 | 100 |
| 5-827, aus D1 | 5 | 70 | 70 | 70 |

## Patentansprüche

1. 3-Acyl-benzamide der Formel (I), 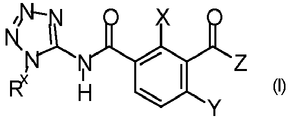 worin die Symbole und Indizes folgende Bedeutungen haben:
R^{x} bedeutet (C₁-C₆)-Alkyl,
X bedeutet Halogen, (C₁-C₆)-Alkyl, Halogen-(C₁-C₆)-alkyl, (C₃-C₆)-Cycloalkyl, R¹O, R²S(O)ₙ oder R¹O-(C₁-C₆)-Alkyl,
Y bedeutet Halogen, (C₁-C₆)-Alkyl, Halogen-(C₁-C₆)-alkyl oder R¹O, R²S(O)ₙ,
Z bedeutet (C₁-C₆)-Alkyl, (C₃-C₆)-Cycloalkyl, (C₂-C₆)-Alkenyl, (C₃-C₆)-Alkinyl, Halogen-(C₁-C₆)-alkyl, (C₁-C₆)-Alkyl-O-(C₁-C₆)-alkyl, (C₁-C₆)-Alkyl-C(O), (C₁-C₆)-Alkyl-C(O)-(C₁-C₆)-alkyl, Phenyl oder Heterocyclyl, wobei die Reste Phenyl, Heterocyclyl, (C₂-C₆)-Alkenyl, (C₃-C₆)-Alkinyl und (C₃-C₆)-Cycloalkyl jeweils m Substituenten R³ tragen,
R¹ bedeutet (C₁-C₆)-Alkyl oder Halogen-(C₁-C₆)-alkyl,
R² bedeutet (C₁-C₆)-Alkyl,
R³ bedeutet Halogen, (C₁-C₆)-Alkyl, (C₁-C₃)-Alkyl-O-C(O), Cyano oder Halogen-(C₁-C₆)-alkyl,
m bedeutet 0, 1, 2, 3 oder 4,
n bedeutet 0, 1 oder 2.

2. 3-Acyl-benzamide gemäß Anspruch 1, worin
R^{x} bedeutet (C₁-C₆)-Alkyl,
X bedeutet Halogen, (C₁-C₆)-Alkyl, Halogen-(C₁-C₆)-alkyl, (C₃-C₆)-Cycloalkyl, R¹O, R²S(O)ₙ oder R¹O-(C₁-C₆)-Alkyl,
Y bedeutet Halogen, (C₁-C₆)-Alkyl, Halogen-(C₁-C₆)-alkyl oder R¹O, R²S(O)ₙ,
Z bedeutet (C₁-C₆)-Alkyl, (C₃-C₆)-Cycloalkyl, (C₂-C₆)-Alkenyl, (C₃-C₆)-Alkinyl, Halogen-(C₁-C₆)-alkyl, (C₁-C₆)-Alkyl-O-(C₁-C₆)-alkyl, (C₁-C₆)-Alkyl-C(O), (C₁-C₆)-Alkyl-C(O)-(C₁-C₆)-alkyl oder Phenyl, wobei die Reste Phenyl, (C₂-C₆)-Alkenyl, (C₃-C₆)-Alkinyl und (C₃-C₆)-Cycloalkyl jeweils m Substituenten R³ tragen,
R¹ bedeutet (C₁-C₆)-Alkyl oder Halogen-(C₁-C₆)-alkyl,
R² bedeutet (C₁-C₆)-Alkyl,
R³ bedeutet Halogen, (C₁-C₆)-Alkyl, (C₁-C₃)-Alkyl-O-C(O), Cyano oder Halogen-(C₁-C₆)-alkyl, m bedeutet 0, 1, 2, 3 oder 4,
n bedeutet 0, 1 oder 2.

3. 3-Acyl-benzamide gemäß Anspruch 1 oder 2, worin
R^{x} bedeutet (C₁-C₆)-Alkyl,
X bedeutet Fluor, Chlor, Brom, Iod, Methyl, Ethyl, Cyclopropyl, Trifluormethyl, Difluormethyl, Methoxymethyl, Methoxy, Methylsulfanyl, Methylsulfinyl, Methylsulfonyl, Ethylsulfanyl oder Ethylsulfonyl,
Y bedeutet Chlor, Brom, Iod, Methyl, Ethyl, Trifluormethyl, Difluormethyl, Methylsulfanyl, Methylsulfinyl, Methylsulfonyl oder Ethylsulfonyl,
Z bedeutet Methyl, Ethyl, n-Propyl, iso-Propyl, Cyclopropyl, n-Butyl, tert-Butyl, Methoxymethyl, Chlormethyl, Acetyl, Vinyl, 1-Methylvinyl, 2-Methylvinyl, (1,2-Dimethyl)-vinyl, (2,2-Dimethyl)-vinyl, 1-Methylcyclopropyl, 2-Methylcyclopropyl, (2,2-Dimethyl)-cyclopropyl, (1,2-Dimethyl)-cyclopropyl, 2-Fluorcyclopropyl, (2,2-Difluor)-cyclopropyl, Cyclobutyl, Cyclopentyl, Cyclohexyl, 2-Thienyl, 2-Furyl, Phenyl, 4-Methoxyphenyl, 4-Chlorphenyl, (3-Trifluormethyl)-phenyl, 3,5-Difluorphenyl, Trifluormethyl oder Difluormethyl.

4. Herbizide Mittel enthaltend mindestens eine Verbindung gemäß einem der Ansprüche 1 bis 3 in Mischung mit Formulierungshilfsmitteln.

5. Herbizide Mittel gemäß Anspruch 4 enthaltend mindestens einen weiteren pestizid wirksamen Stoff aus der Gruppe Insektizide, Akarizide, Herbizide, Fungizide, Safener und Wachstumsregulatoren.

6. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder von herbiziden Mitteln nach Anspruch 4 oder 5 auf die Pflanzen oder auf den Ort des unerwünschten Pflanzenwachstums appliziert.

7. Verwendung von Verbindungen der Formel (I) gemäß einem der Ansprüche 1 bis 3 oder von herbiziden Mitteln nach Anspruch 4 oder 5 zur Bekämpfung unerwünschter Pflanzen.

8. Verwendung nach Anspruch 7, **dadurch gekennzeichnet, daß** die Verbindungen der Formel (I) zur Bekämpfung unerwünschter Pflanzen in Kulturen von Nutzpflanzen eingesetzt werden.

9. Verwendung nach Anspruch 8, **dadurch gekennzeichnet, daß** die Nutzpflanzen transgene Nutzpflanzen sind.

## Claims

1. 3-Acylbenzamides of the formula (I) 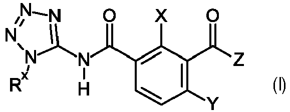 in which the symbols and indices are defined as follows:
R^{x} represents (C₁-C₆)-alkyl,
X represents halogen, (C₁-C₆)-alkyl, halo-(C₁-C₆)-alkyl, (C₃-C₆)-cycloalkyl, R¹O, R²S(O)ₙ or R¹O-(C₁-C₆)-alkyl,
Y represents halogen, (C₁-C₆)-alkyl, halo-(C₁-C₆)-alkyl or R¹O, R²S(O)ₙ,
Z represents (C₁-C₆) -alkyl, (C₃-C₆)-cycloalkyl, (C₂-C₆)-alkenyl, (C₃-C₆)-alkynyl, halo-(C₁-C₆) -alkyl, (C₁-C₆)-alkyl-O-(C₁-C₆)-alkyl, (C₁-C₆)-alkyl-C(O), (C₁-C₆)-alkyl-C(O)-(C₁-C₆)-alkyl, phenyl or heterocyclyl, where the radicals phenyl, heterocyclyl, (C₂-C₆)-alkenyl, (C₃-C₆)-alkynyl and (C₃-C₆)-cycloalkyl each carry m substituents R³,
R¹ represents (C₁-C₆)-alkyl or halo-(C₁-C₆)-alkyl,
R² represents (C₁-C₆)-alkyl,
R³ represents halogen, (C₁-C₆)-alkyl, (C₁-C₃)-alkyl-O-C(O), cyano or halo-(C₁-C₆)-alkyl,
m represents 0, 1, 2, 3 or 4,
n represents 0, 1 or 2.

2. 3-Acylbenzamides according to Claim 1 in which
R^{x} represents (C₁-C₆)-alkyl,
X represents halogen, (C₁-C₆)-alkyl, halo-(C₁-C₆)-alkyl, (C₃-C₆)-cycloalkyl, R¹O, R²S(O)ₙ or R¹O-(C₁-C₆)-alkyl,
Y represents halogen, (C₁-C₆)-alkyl, halo-(C₁-C₆)-alkyl or R¹O, R²S(O)ₙ,
Z represents (C₁-C₆)-alkyl, (C₃-C₆)-cycloalkyl, (C₂-C₆)-alkenyl, (C₃-C₆)-alkynyl, halo-(C₁-C₆)-alkyl, (C₁-C₆)-alkyl-O-(C₁-C₆)-alkyl, (C₁-C₆)-alkyl-C(O), (C₁-C₆)-alkyl-C(O)-(C₁-C₆)-alkyl or phenyl, where the radicals phenyl, (C₂-C₆)-alkenyl, (C₃-C₆)-alkynyl and (C₃-C₆)-cycloalkyl each carry m substituents R³,
R¹ represents (C₁-C₆)-alkyl or halo-(C₁-C₆)-alkyl,
R² represents (C₁-C₆)-alkyl,
R³ represents halogen, (C₁-C₆)-alkyl, (C₁-C₃)-alkyl-O-C(O), cyano or halo-(C₁-C₆)-alkyl,
m represents 0, 1, 2, 3 or 4,
n represents 0, 1 or 2.

3. 3-Acylbenzamides according to Claim 1 or 2 in which R^{x} represents (C₁-C₆) -alkyl,
X represents fluorine, chlorine, bromine, iodine, methyl, ethyl, cyclopropyl, trifluoromethyl, difluoromethyl, methoxymethyl, methoxy, methylsulfanyl, methylsulfinyl, methylsulfonyl, ethylsulfanyl or ethylsulfonyl,
Y represents chlorine, bromine, iodine, methyl, ethyl, trifluoromethyl, difluoromethyl, methylsulfanyl, methylsulfinyl, methylsulfonyl or ethylsulfonyl,
Z represents methyl, ethyl, n-propyl, isopropyl, cyclopropyl, n-butyl, tert-butyl, methoxymethyl, chloromethyl, acetyl, vinyl, 1-methylvinyl, 2-methylvinyl, (1,2-dimethyl)vinyl, (2,2-dimethyl)vinyl, 1-methylcyclopropyl, 2-methylcyclopropyl, (2,2-dimethyl)cyclopropyl, (1,2-dimethyl)cyclopropyl, 2-fluorocyclopropyl, (2,2-difluoro) cyclopropyl, cyclobutyl, cyclopentyl, cyclohexyl, 2-thienyl, 2-furyl, phenyl, 4-methoxyphenyl, 4-chlorophenyl, (3-trifluoromethyl)phenyl, 3,5-difluorophenyl, trifluoromethyl or difluoromethyl.

4. Herbicidal compositions comprising at least one compound according to any of Claims 1 to 3 mixed with formulation auxiliaries.

5. Herbicidal compositions according to Claim 4, comprising at least one further pesticidally active substance from the group of insecticides, acaricides, herbicides, fungicides, safeners and growth regulators.

6. Method for controlling unwanted plants, **characterized in that** an effective amount of at least one compound of the formula (I) according to any of Claims 1 to 3 or of herbicidal compositions according to Claim 4 or 5 is applied to the plants or the site of the unwanted vegetation.

7. Use of compounds of the formula (I) according to any of Claims 1 to 3 or of herbicidal compositions according to Claim 4 or 5 for controlling unwanted plants.

8. Use according to Claim 7, **characterized in that** the compounds of the formula (I) are used for controlling unwanted plants in crops of useful plants.

9. Use according to Claim 8, **characterized in that** the useful plants are transgenic useful plants.

## Revendications

1. 3-Acyl-benzamides de formule (I), 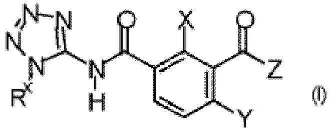 dans laquelle les symboles et les indices possèdent les significations suivantes :
R^{x} signifie (C₁-C₆)-alkyle,
X signifie halogène, (C₁-C₆) -alkyle, halogéno- (C₁-C₆) - alkyle, (C₃-C₆) -cycloalkyle, R¹O, R²S(O)ₙ ou R¹O- (C₁-C₆) - alkyle,
Y signifie halogène, (C₁-C₆) -alkyle, halogéno- (C₁-C₆) - alkyle ou R¹O, R²S(O)ₙ,
Z signifie (C₁-C₆)-alkyle, (C₃-C₆) -cycloalkyle, (C₂-C₆)-alcényle, (C₃-C₆) -alcynyle, halogéno-(C₁-C₆)-alkyle, (C₁-C₆)-alkyl-O-(C₁-C₆)-alkyle, (C₁-C₆)-alkyl-C(O), (C₁-C₆)-alkyl-C(O)-(C₁-C₆)-alkyle, phényle ou hétérocyclyle, les radicaux phényle, hétérocyclyle, (C₂-C₆)-alcényle, (C₃-C₆)-alcynyle et (C₃-C₆) -cycloalkyle portant à chaque fois m substituants R³,
R¹ signifie (C₁-C₆)-alkyle ou halogéno-(C₁-C₆) -alkyle,
R² signifie (C₁-C₆)-alkyle,
R³ signifie halogène, (C₁-C₆) -alkyle, (C₁-C₃)-alkyl-OC(O), cyano ou halogéne-(C₁-C₆)-alkyle,
m signifie 0, 1 , 2, 3 ou 4,
n signifie 0, 1 ou 2.

2. 3-Acyl-benzamides selon la revendication 1, dans lesquels
R^{x} signifie (C₁-C₆)-alkyle,
X signifie halogène, (C₁-C₆) -alkyle, halogéno-(C₁-C₆) - alkyle, (C₃-C₆)-cycloalkyle, R¹O, R²S(O)ₙ ou R¹O-(C₁-C₆)-alkyle,
Y signifie halogène, (C₁-C₆) -alkyle, halogéno-(C₁-C₆) - alkyle ou R¹O, R²S(O)ₙ,
Z signifie (C₁-C₆)-alkyle, (C₃-C₆)-cycloalkyle, (C₂-C₆)-alcényle, (C₃-C₆)-alcynyle, halogéno-(C₁-C₆)-alkyle, (C₁-C₆)-alkyl-O-(C₁-C₆)-alkyle, (C₁-C₆)-alkyl-C(O), (C₁-C₆)-alkyl-C(O)-(C₁-C₆)-alkyle ou phényle, les radicaux phényle, (C₂-C₆)-alcényle, (C₃-C₆)-alcynyle et (C₃-C₆)-cycloalkyle portant à chaque fois m substituants R³,
R¹ signifie (C₁-C₆)-alkyle ou halogéno-(C₁-C₆)-alkyle,
R² signifie (C₁-C₆)-alkyle,
R³ signifie halogène, (C₁-C₆)-alkyle, (C₁-C₃)-alkyl-O-C(O), cyano ou halogéno-(C₁-C₆)-alkyle,
m signifie 0, 1, 2, 3 ou 4,
n signifie 0, 1 ou 2.

3. 3-Acyl-benzamides selon la revendication 1 ou 2, dans lesquels
R^{x} signifie (C₁-C₆)-alkyle,
X signifie fluor, chlore, brome, iode, méthyle, éthyle, cyclopropyle, trifluorométhyle, difluorométhyle, méthoxyméthyle, méthoxy, méthylsulfanyle, méthylsulfinyle, méthylsulfonyle, éthylsulfanyle ou éthylsulfonyle,
Y signifie chlore, brome, iode, méthyle, éthyle, trifluorométhyle, difluorométhyle, méthylsulfanyle, méthylsulfinyle, méthylsulfonyle ou éthylsulfonyle,
Z signifie méthyle, éthyle, n-propyle, iso-propyle, cyclopropyle, n-butyle, tert-butyle, méthoxyméthyle, chlorométhyle, acétyle, vinyle, 1-méthylvinyle, 2-méthylvinyle, (1,2-diméthyl)-vinyle, (2,2-diméthyl)-vinyle, 1-méthylcyclopropyle, 2-méthylcyclopropyle, (2,2-diméthyl)-cyclopropyle, (1,2-diméthyl)-cyclopropyle, 2-fluorocyclopropyle, (2,2-difluor)-cyclopropyle, cyclobutyle, cyclopentyle, cyclohexyle, 2-thiényle, 2-furyle, phényle, 4-méthoxyphényle, 4-chlorophényle, (3-trifluorométhyl)-phényle, 3,5-difluorophényle, trifluorométhyle ou difluorométhyle.

4. Agents herbicides contenant au moins un composé selon l'une quelconque des revendications 1 à 3 en mélange avec des auxiliaires de formulations.

5. Agents herbicides selon la revendication 4 contenant au moins une matière supplémentaire efficace de manière pesticide du groupe des insecticides, des acaricides, des herbicides, des fongicides, des phytoprotecteurs et des régulateurs de croissance.

6. Procédé pour la lutte contre des végétaux indésirables, **caractérisé en ce qu'**on applique une quantité efficace d'au moins un composé de formule (I) selon l'une quelconque des revendications 1 à 3 ou d'agents herbicides selon la revendication 4 ou 5 sur les végétaux ou sur le lieu de la croissance végétale indésirable.

7. Utilisation de composés de formule (I) selon l'une quelconque des revendications 1 à 3 ou d'agents herbicides selon la revendication 4 ou 5 pour la lutte contre des végétaux indésirables.

8. Utilisation selon la revendication 7, **caractérisée en ce que** les composés de formule (I) sont utilisés pour la lutte contre des végétaux indésirables dans des cultures de végétaux utiles.

9. Utilisation selon la revendication 8, **caractérisée en ce que** les végétaux utiles sont des végétaux utiles transgéniques.
